# EUROPEAN PATENT SPECIFICATION

(11) **EP 3 797 818 B1**
(45) Date of publication and mention of the grant of the patent: **18.01.2023**
(21) Application number: 19808333.9
(22) Date of filing: 23.05.2019
(51) Int. Cl.: A61M 31/00, A61K 9/22, A61K 31/198, A61P 25/16, A61K 9/00, A61J 1/00, A61J 7/00, A61K 9/20, A61K 9/28, B33Y 80/00

(54) **CONTROLLED-RELEASE SYSTEM OF ACTIVE PHARMACEUTICAL INGREDIENT AND PREPARATION METHOD THEREFOR**
SYSTEM ZUR VERZÖGERTEN FREISETZUNG EINES WIRKSTOFFES UND VERFAHREN ZU SEINER HERSTELLUNG
SYSTÈME À LIBÉRATION CONTRÔLÉE D'UN PRINCIPE ACTIF PHARMACEUTIQUE ET SON PROCÉDÉ DE PRÉPARATION

(30) Priority: 23.05.2018 CN 201810503654
(43) Date of publication of application: 31.03.2021
(73) Proprietor: Shanghai WD Pharmaceutical Co., Ltd, Shanghai 201203 (CN)
(72) Inventor: DONG, Liang Chang, Shanghai 201203 (CN); CHEN, Xishan, Shanghai 201203 (CN); SHI, Jingmin, Shanghai 201203 (CN); ZHANG, Danyong, Shanghai 201203 (CN)
(74) Representative: Hoffmann Eitle
(86) International application number: PCT/CN2019/088084
(87) International publication number: WO 2019/223753

(56) References cited:
- EP-A1- 0 389 224
- WO-A1-91/02518
- WO-A1-2015/054302
- WO-A2-2006/072940
- CN-A- 1 997 421
- CN-A- 1 997 421
- CN-A- 101 926 785

## Description

### Cross reference to related application

The present application claims priority of the Chinese Patent Application CN201810503654.4 filed on May 23, 2018.

### Field of invention

The present invention relates to the fields of biopharmaceuticals and medical devices. Specifically, it relates to a controlled-release system of active pharmaceutical ingredients and a preparation method thereof.

### Prior arts

A great number of active pharmaceutical ingredients (APIs), including levodopa (LD), carbidopa (CD), baclofen, acyclovir, valacyclovir, ganciclovir, metformin, gabapentin, etc., have their absorption window limited at the upper gastrointestinal tract. Incorporation of these APIs into conventional extended-release dosages will not only result in compromised bioavailability, but also lead to failure of achieving prolonged therapeutic coverage. Therefore, a number of technologies have been disclosed in the prior art to extend the retention time in the stomach. Following are these technologies: expansion (USP 4735804, 5002772 and 6685962), swelling (USP 4434153, 5750585, 5972389, 6120803, 6660300 B1, US 2007/0196396A1 and USP 9439851), floating (USP 4167558, 5232704 and 6261601), raft-forming (USP 4140760 and 5068109), sinking (USP 4193985 and 4900557) and muco-adhesion (USP 6207197 and US2005/030552), etc. The technologies described above have very limited success, especially when oral dosage forms using these technologies are administered at fasting state. Patent document WO 2006/072940 discloses a controlled long acting release pharmaceutical preparation for use in the oral cavity either for the treatment of diseases of the oral cavity or; for releasing said pharmaceutical preparation into said oral cavity, comprising at least a therapeutic agent, a binder and a lubricant.

Therefore, there is a need for a novel controlled release system that could provide prolonged exposure to these active pharmaceutical ingredients with their absorption window limited at the upper gastrointestinal tract.

One of these active pharmaceutical ingredients is levodopa, which is used for the treatment of Parkinson's disease. Parkinson's disease is a progressive disease which results from the loss of dopamine-producing cells in the brain. Dopamine is a substance that is naturally present in the brain and spinal cord. Dopamine helps the nerve cells in the brain properly control the movement function. As the level of dopamine in the brain gets lower, the symptoms of Parkinson's disease appear, for example, muscle stiffness, slow movements and difficulty of keeping one's balance. Dopamine cannot penetrate the blood-brain barrier (BBB), which is the reason why oral dosage form of dopamine does not work. Levodopa is the precursor of dopamine, which can penetrate the BBB and convert into dopamine in brain tissue. Levodopa therapy is still a "gold standard" for treatment of Parkinson's disease, and almost all PD patients receive LD treatment at certain stage of the disease. However, most levodopa is decarboxylated to dopamine before reaching the brain. Therefore, LD is usually administered with decarboxylase inhibitors such as carbidopa or benserazide to prevent the formation of peripheral dopamine. LD/CD treatment can increase the content of dopamine in the brain and alleviate the symptoms of Parkinson's disease.

Despite oral administration of LD drugs, patients often develop into motor complications in the form of fluctuating - so-called "on-off" phenomena - and involuntary movements (dyskinesia) (Fahn S. The spectrum of levodopa-induced dyskinesias. Ann Neurol 2000; 47 [suppl 1]:2-11and Golbe LI. Young-onset Parkinson's disease: a clinical review. Neurology 1991; 41:168-73.). The reason of this fluctuation can be attributed to the short half-life of LD, resulting in non-physiological, pulsatile stimulation of Postsynaptic dopamine receptors in the striatum. In addition, the therapeutic window is becoming narrower as the PD is progressing, implying that patients with advanced PD are more likely to develop akinasia and dyskinesia.

Theoretically, an extended release oral dosage form could provide a steady plasma concentration of LD for prolonging therapeutic coverage, thus alleviating the "on-off" phenomenon. In fact, due to the limited absorption of LD at the proximal gastrointestinal tract, it is a great challenge to develop an extended release LD dosage form. The residence time of the dosage form at the proximal gastrointestinal tract is approximately 3-4 hours. Therefore, any amount of LD released longer than 3-4 hours will not be absorbed and ends up in fecal material. U.S. Patents 9086079, 908608, 8557283, 8454998 and 8377474 disclose the use of organic acids to prolong the LD absorption time, thus leading to an extended release dosage form with an absorption duration of approximately 4-5 hours. Products using these patented formulations, such as Rytary, are suitable for the treatment of early and moderate Parkinson's disease.

Treatment of patients with advanced Parkinson's disease remains a medical challenge. Deep brain stimulation (DBS) (Marconi R, Landi A, Valzania F. Subthalamic nucleus stimulation in Parkinson's disease. Neurol Sci 2008; 29 Suppl 5: S389-91) and continuous intestinal LD infusion to the duodenum (DUOPA) are currently the method for the treatment of advanced PD patients who have experienced unsatisfactory effect with oral dosage forms. Since DBS involves a brain surgery, this treatment is very invasive and usually intimidating most patients. In addition, DBS also carries a risk of neuropsychiatric side effects (Voon V, Kubu C, Krack P et al; Deep brain stimulation: neuropsychological and neuropsychiatric issues. Mov Disord 2006; 21 [Suppl. 14]: S305-27). DUOPA includes a gel formulation that is administered with a pump via a tube, directly into the upper small intestine throughout the day. Continuous intestinal LD infusion into the duodenum can maintain consistent LD plasma levels for 16 hours, providing a more sustained stimulation of dopamine receptors, thus reducing motor and non-motor complications associated with pulsatile dopaminergic stimulation produced by current oral medications (Nyholm, D., Odin, P., Johansson, A., Chatamra, K., Locke, C., Freeman, S., et al; Stable levodopa plasma levels with jejunal infusion of levodopa-carbidopa intestinal gel in advanced Parkinson's disease patients [abstract]. Movement Disorders 2012; 27 Suppl 1:410).

However, the DUOPA therapy is extremely invasive. Surgery or percutaneous endoscopic gastrostomy is necessitated for the placement of a small tube to the duodenum (Health Canada Fact Sheet for DUODOPA). For some patients, a portable pump can be cumbersome. Other issues may also occur, including sporadic blockage of tubes, displacement of the internal tube, leakage at the tube connection and local infections. In addition, the DUOPA gel formulation is unstable; it has to be stored in a refrigerator (2 °C-8 °C) to minimize the degradation products, especially hydrazine, which is known to be genotoxic and possibly carcinogenic. Even at the refrigerated condition, the shelf life of the product is still very short, only 15 weeks (DUODOPA Prescription Insert). Finally, high costs may be a limiting factor. Treatment with DUOPA is expensive and requires an expert team including neurologists, gastroenterologists, nurses as well as an outbound contact to cooperate in the care of patients.

### Content of the present invention

The technical problem to be solved in the present invention is to provide a controlled-release system of active pharmaceutical ingredients and a preparation method thereof so as to overcome the defect that the current extended release drugs cannot provide a prolonged and a steady plasma profiles of active pharmaceutical ingredients; and to avoid the potential precipitation of cellulose acetate during the coating process of the release-controlling membrane so as to solve the defects of uneven membrane coating and drug release.

To solve the above technical problems, one of the technical solutions of the present invention is: an extended release dosage form with an absorption window at the upper gastrointestinal tract, wherein the extended release dosage form comprises an extended release platform and a retention enabling platform; the claims disclose an extended release dosage form with an absorption window at the upper gastrointestinal tract comprising an extended release platform and a retention enabling platform; wherein the extended release platform is a pharmaceutical composition comprising a tablet core and a coating membrane, the tablet core comprises a drug-containing layer, and the coating membrane comprises cellulose acetate and copovidone, wherein the weight of the cellulose acetate is 50-70% of the weight of the coating membrane, the weight of the copovidone is 30-50% of the weight of the coating membrane; wherein the retention enabling platform is used for maintaining the extended release platform in the oral cavity, and at least one end of the retention enabling platform is connected with a cover, so that the extended release platform can be retained in the retention enabling platform; the drug-containing layer comprises active pharmaceutical ingredients and excipients, and the active pharmaceutical ingredient is one or more of levodopa and carbidopa.

The invention is defined by the scope of the claims.

The extended release platform (ERP) is a pharmaceutical composition comprising a tablet core and a coating membrane, wherein the tablet core comprises a drug-containing layer, and the coating membrane comprises cellulose acetate and copovidone, wherein the weight of the cellulose acetate is 50-70% of the weight of the coating membrane, and the weight of the copovidone is 30-50% of the weight of the coating membrane.

The function of the retention enabling platform is to maintain the extended release platform in the oral cavity. At least one end of the retention enabling platform is connected with a cover, so that the extended release platform can maintain in the retention enabling platform.

In some preferred embodiments, the copovidone is prepared by the following method, which comprises the following steps: polymerizing vinylpyrrolidone and vinyl acetate, and the weight of the vinylpyrrolidone and the vinyl acetate is in the ratio of 40: 60-80: 20. Preferably, the weight of the vinylpyrrolidone and the vinyl acetate is in the ratio of 50: 50-70: 30. More preferably, the weight ratio of the vinylpyrrolidone and the vinyl acetate is in the ratio of 60:40.

Preferably, the retention enabling platform comprises a personalized retention enabling module and a drug fastened module, the drug fastened module can fasten the extended release platform, and the personalized retention enabling module can maintain the drug fastened module in the oral cavity.

More preferably, the drug fastened module is one or more reservoirs. In one embodiment, the reservoir is in a basket structure. In another embodiment, the shape of the cross section of the reservoir is polygon, circular closed-loop or circular open-loop, or a combination thereof.

Preferably, at least one end of the reservoir is connected with a cover, so that the extended release platform can be maintained in the reservoir; more preferably, the cover is a strip.

Preferably, the retention enabling module can be fitted to any one or more teeth in the oral cavity. Preferably the mandibular permanent teeth. More preferably the mandibular molar. Most preferably the mandibular second molar and its anterior and posterior molar.

The retention enabling module can be customized to fit and wrap, clamp or insert the entire maxillary tooth or the entire mandibular permanent tooth; preferably wrap, clamp or insert the mandibular permanent tooth; more preferably wrap the mandibular molar; most preferably wrap, clamp or insert the second mandibular molar and its adjacent parts of the first molar and the second bicuspid.

The extended release platform of the present invention is an osmotic pump delivery system including LD and CD. The osmotic pump delivery system can be a single-layer elementary osmotic pump or a two-layer push-pull system. Osmotic pump delivery system could provide constant release of LD/CD in the oral cavity, which is in sharp contrast with matrix extended release system that is susceptible to the conditions in oral cavity such as pH, saliva availability and voluntary or involuntary rubbing of the hydrated matrix tablets by tongue.

The retention enabling platform (REP) of the present invention is a personalized ERP retainer that enables to fasten the ERP in oral cavity. Therefore, LD/CD can be released near the throat and readily swallowed to the stomach. The REP is a kind of REP with security features, which can prevent the accidental blockage of the extended release system. In the present invention, the " personalized" refers to the preparation of the retention enabling platform or retention enabling module that enables to fasten ERP in the oral cavity and fit to the shape of one or more teeth or the whole maxillary teeth or the whole mandibular teeth of the patient according to the shape of the teeth of the patient.

To solve the above technical problems, one of the technical solutions of the present invention is: a preparation method of the extended release dosage form, and the preparation method of the retention enabling platform comprises 3D printing, injection molding or impression molding. By combining oral scanning, CAD/CAM design and preparation method, REP can be accurately and rapidly prepared according to the image of individual oral scanning.

Wherein, the working principle of 3D printing is basically the same as that of ordinary printer, except that the printing materials are different. The printing materials of ordinary printers are ink and paper, while 3D printers are equipped with different "printing materials" such as metal, ceramics, plastic, sand, etc. After the printer is connected with the computer, the "printing materials" can be stacked layer upon layer through computer control, thereby making the blueprint on the computer into a real object in the end. 3D printing refers to the technical principles of ordinary printers, in which the process of layering is very similar to inkjet printing. This printing technology is called 3D stereo printing technology. There are many different technologies for 3D printing, which differ in that modules are created by using available materials and different layers. Common materials for 3D printing are nylon glass fiber, polylactic acid, ABS resin, durable nylon material, gypsum material, aluminum material, metal titanium, titanium alloy, stainless steel, silver plating, gold plating, cobalt chromium alloy, cobalt chromium molybdenum alloy or rubber material.

Injection molding is a method of injecting and molding, that is, at a certain temperature, the polymer material that is completely molten is stirred by a screw, injected into a mold cavity with high pressure, and cooled and solidified to get the molding products. This method is suitable for mass production of complex shape parts and is one of the important processing methods. The injection molding method has the advantages of fast production speed, high efficiency, automation of operation, a large variety of designs, a large variety of shapes including simple and complex shapes, and a large variety of sizes including large and small sizes. Moreover, dimension of product is precise, product is easy to be updated and module can be made into complex shapes. Injection molding is suitable for mass production and is applicable to the molding processing field of products with complex shape and the like.

For impression molding, firstly, a drug fastened module suitable for the extended release platform is prepared. Secondly, elliptical thermoplastic sheet is processed by conventional technology. Finally, a personalized REP was prepared. A thermoplastic sheet is heated in hot water at about 70 °C to soften it so as to have good plasticity. When the thermoplastic sheet becomes translucent (about 1 minute), take it out and put it on the teeth, and press the softened thermoplastic sheet to completely wrap the teeth to form a retention enabling module that completely fitted to the teeth. The drug fastened module is then quickly embedded into the uncured retention enabling module and cooled to solidify into a personalized retention enabling platform (REP). Some water can be sprayed briefly to further accelerate the cooling, wait for the personalized retention enabling platform to cool and restore the original opaque plate state, and take out the cooled personalized retention enabling platform from the oral cavity.

Preferably, the retention enabling platform is prepared from one or more oral stable materials including oral stable metals and thermoplastic elastomers.

More preferably, the oral stable metal comprises dental titanium, stainless steel, cobalt chromium alloy, cobalt chromium molybdenum alloy, nickel chromium alloy or precious metal, and the thermoplastic elastomer comprises polycaprolactone (PCL), ethylene vinyl acetate copolymer (EVA), high density polyethylene (HDPE), polypropylene (PP), polyacrylate, polyurethane, silicone polymer, polyester, poly (styrene-ethylene-butene-styrene)("SEBS"), poly (styrene-butadiene-styrene) ("SBS"), poly (styrene-isoprene-styrene) ("SIS"), or a copolymer of any two or more of the above , or a physical combination thereof.

Osmotic pump extended release drug delivery system is an advanced oral osmotic pump extended release drug delivery system, which can be in the form of tablets with an outer semi-permeable membrane and one or more small laser drilled orifices. When the tablet is taken orally through the gastrointestinal tract, water is absorbed by osmosis through the semi-permeable membrane, and the resulting osmotic pressure will push the active drug through the orifice of the tablet. The elementary osmotic pump (EOP) developed by Alza in 1974 is the first embodiment of the drug delivery system of the oral osmotic pump. It is composed of a drug-containing tablet core coated with a semi-permeable coating membrane, and an orifice drilled for drug release on the coating membrane, so that simplifies the osmotic pump preparation into the form of an ordinary coated tablet. Push-pull osmotic pump (PPOP) is suitable for soluble or poorly soluble drugs, which can be a bi-layer tablet with a semi-permeable membrane. The drug-containing layer comprises a drug chamber containing drugs and osmotic active substances, and an osmotic push layer comprising osmotic polymer. When the system contacts with the water, the osmotic push layer swells and pushes the drug in the drug chamber to release through the drug delivery orifice. The pharmaceutical composition or the upper gastrointestinal tract (UGI) extended release drug delivery system of the present invention comprises the forms such as a single-layer elementary osmotic pump, a bi-layer push-pull osmotic pump, and a bi-layer push-pull osmotic pump comprising an immediate-release drug overcoat, but differs from the osmotic pump in the prior art.

In the extended release dosage form of the present invention as described above, preferably, in the extended release platform, the drug-containing layer comprises active pharmaceutical ingredients and excipients, and the active pharmaceutical ingredient is one or more of levodopa and carbidopa.

The active pharmaceutical ingredient comprises levodopa and/or carbidopa. The excipient is one or more of filler, osmotic agent, hydrophilic polymer, binding agent, lubricant, preservative, flavoring agent, acidifying agent and antioxidant. More preferably, the excipient is one or more of filler, osmotic agent, hydrophilic polymer, binding agent, lubricant and preservative. Even more preferably, the excipients are filler, osmotic agent, hydrophilic polymer, binding agent, lubricant and preservative.

In one embodiment, when the pharmaceutically active ingredient comprises levodopa, the weight percentage of the levodopa is 20-70%. In another embodiment, when the active ingredient comprises carbidopa, the weight percentage of carbidopa is 0-20% but not 0%, Wherein the weight percentage is the weight percentage of each component of the drug-containing layer.

In one embodiment, the pharmaceutically active ingredient comprises levodopa, and the weight percentage of the levodopa is 30-50%. In another embodiment, when the active ingredient comprises carbidopa, the weight percentage of the carbidopa is 1-10%, wherein the weight percentage is the weight percentage of each component of the drug-containing layer.

Preferably, in the pharmaceutical composition described above, when the excipient comprises a filler, the filler is one or more of microcrystalline cellulose, hydroxypropyl cellulose and mannitol; wherein the weight percentage of the filler is 0-50% but not 0%.

In another embodiment, when the excipient comprises an osmotic agent, the osmotic agent is one or more of magnesium sulfate, magnesium chloride, sodium chloride, lithium chloride, potassium sulfate, sodium sulfate, mannitol, urea, sorbitol, inositol, sucrose and glucose, wherein the weight percentage of the osmotic agent is 0-50% but not 0%.

In another embodiment, when the excipient comprises a hydrophilic polymer, the hydrophilic polymer is one or more of hydroxypropyl cellulose, hydroxypropyl methyl cellulose, carboxymethyl cellulose, polyvinylpyrrolidone and hydroxyethyl cellulose, wherein the weight percentage of the hydrophilic polymer is 0-50% but not 0%.

In another embodiment, when the excipient comprises an acidifying agent, the acidifying agent is one or more of citric acid, sodium citrate, potassium citrate, malic acid, fumaric acid, lactic acid, phosphoric acid and tartaric acid, wherein the weight percentage of the acidifying agent is 0-10% but not 0%.

The weight percentage is the weight percentage of each component of the drug-containing layer.

Preferably, the tablet core of the pharmaceutical composition further comprises an osmotic push layer, wherein the osmotic push layer comprises a hydrophilic polymer, an osmotic agent and a binding agent. Preferably, the osmotic push layer comprises a hydrophilic polymer, an osmotic agent, a binding agent, and a lubricant. More preferably, the osmotic push layer comprises a hydrophilic polymer, an osmotic agent, a binding agent, a lubricant and a colorant. The osmotic push layer and the drug-containing layer are components of a bi-layer tablet core, and the coating membrane is wrapped outside the tablet core. The pharmaceutical composition can be a kind of osmotic pump extended release drug delivery system, that is, a bi-layer push-pull osmotic pump.

As a pharmaceutical composition of the bi-layer push-pull osmotic pump, preferably, the hydrophilic polymer of the osmotic push layer is κ-carrageenan, sodium carboxymethyl cellulose or polyethylene oxide, wherein the molecular weight of the hydrophilic polymer is 75,000-7,500,000, and the weight percentage of the hydrophilic polymer is 25-85%.

In one embodiment, the osmotic agent in the osmotic pump push layer is one or more of magnesium sulfate, magnesium chloride, sodium chloride, lithium chloride, potassium sulfate, sodium sulfate, mannitol, urea, sorbitol, inositol, sucrose and glucose, wherein the weight percentage of the osmotic agent is 5-65%.

In another embodiment, the osmotic push layer comprises a binding agent, the binding agent is one or more of methyl cellulose, hydroxypropyl methyl cellulose, hydroxypropyl cellulose, sodium carboxymethyl cellulose, povidone and gelatin, wherein the weight percentage of the binding agent is 3-20%.

In another embodiment, the osmotic push layer comprises a lubricant, the lubricant is one or more of magnesium stearate, magnesium stearate fumarate, talc, and colloidal silica, wherein the weight percentage of the lubricant is 0-2% but not 0%.

In another embodiment, the osmotic push layer comprises a colorant, the colorant is one or more of iron oxide red, iron oxide yellow, and iron oxide black, wherein the weight percentage of the colorant is 0-5% but not 0%.

The weight percentage is the weight percentage of each component of the osmotic push layer.

Preferably, the osmotic push layer comprises sodium carboxymethyl cellulose, sorbitol, povidone, iron oxide red and magnesium stearate; or comprises sodium carboxymethyl cellulose, hydroxypropyl cellulose, sorbitol, iron oxide red, and magnesium stearate; preferably, is composed of sodium carboxymethyl cellulose, povidone K30, sorbitol, iron oxide red and magnesium stearate; or is composed of sodium carboxymethyl cellulose, hydroxypropyl cellulose, sorbitol, iron oxide red and magnesium stearate. More preferably, the sodium carboxymethyl cellulose is sodium carboxymethyl cellulose 7H4XF or 9H4XF.

More preferably, in one embodiment, the osmotic push layer comprises, in weight percentage, 25-85 wt% of sodium carboxymethyl cellulose, 5-65 wt% of sorbitol, 3-20 wt% of povidone, 0-5 wt% of iron oxide red and 0.5-2 wt% of magnesium stearate; or 25-85wt% of sodium carboxymethyl cellulose, 5-65 wt% of sorbitol, 3-20 wt% of hydroxypropyl cellulose, 0-5 wt% of iron oxide red, and 0.5-2 wt% of magnesium stearate; wherein the weight percentage is the weight percentage of each component of the osmotic push layer.

Even more preferably, the osmotic push layer comprises 55 wt% of sodium carboxymethyl cellulose, 34.0-39.0 wt% of sorbitol, 3-20 wt% of povidone or 10 wt% of hydroxylpropyl cellulose, 0.5-5 wt% of iron oxide red and 0.5-2 wt% of magnesium stearate;

In another embodiment, the osmotic push layer comprises 55 wt% of sodium carboxymethyl cellulose, 39.0 wt% of sorbitol, 5.0 wt% of povidone and 0.5 wt% of iron oxide red and 0.5 wt% of magnesium stearate.

In another embodiment, the osmotic push layer comprises 55 wt% of sodium carboxymethyl cellulose, 34.0 wt% of sorbitol, 10.0 wt% of povidone K30, 0.5 wt% of iron oxide red and 0.5 wt% of magnesium stearate. The weight percentage is the weight percentage of each component of the osmotic push layer.

In another embodiment, the osmotic push layer comprises 55 wt% of sodium carboxymethyl cellulose, 34.0 wt% of sorbitol, 10.0 wt% of hydroxypropyl cellulose, 0.5 wt% of iron oxide red and 0.5 wt% of magnesium stearate. Wherein the weight percentage is the weight percentage of each component of the osmotic push layer.

In some preferred embodiments, the coating membrane of the pharmaceutical composition is further provided with a drug-containing immediate release overcoat. This constitutes a three-layer structure in which the inner layer is a tablet core, the middle layer is a coating membrane, and the outer layer is an overcoat.

More preferably, the drug-containing immediate release overcoat comprises an active pharmaceutical ingredient and an excipient, the active pharmaceutical ingredient comprises levodopa and/or carbidopa, and the excipient is one or more of hydroxypropyl cellulose, aspartame and the Mint Flavors.

In one embodiment, in the extended release platform, when the active pharmaceutical ingredient is levodopa, the weight percentage of the levodopa is 0-75% but not 0%, preferably 23.78-75%; and/or, when the active pharmaceutical ingredient is carbidopa, the weight percentage of carbidopa is 0-93% but not 0%, preferably 26.85-93%; and/or, when the excipient of the overcoat comprises hydroxypropyl cellulose, the weight percentage of the hydroxypropyl cellulose is 2-20%, preferably 10%; and/or, when the excipient of the overcoat comprises aspartame, the weight percentage of the aspartame is 0-5%, preferably 0.9-5%; and/or, when the excipient of the overcoat comprises Mint Flavor, the weight percentage of the Mint Flavor is 0-5 %, preferably 0.1%; wherein the weight percentage is the weight percentage of each component of the overcoat.

More preferably, the weight of the coating membrane is not less than 2.0% of the weight of the tablet core; the coating membrane has one or more orifices, and the diameter of orifice is preferably 0.5 mm-1.0 mm, more preferably 0.5 mm, 0.75 mm and 1.0 mm. Preferably, the weight of the coating membrane is 2.0-15.0% of the weight of the tablet core. More preferably, the weight of the coating membrane is 4.0-8.0% of the weight of the tablet core.

Preferably, in one embodiment, the pharmaceutical composition is composed of a drug-containing layer and a coating membrane. In another embodiment, the pharmaceutical composition is composed of a drug-containing layer, an osmotic push layer and a coating membrane. In another embodiment, the pharmaceutical composition is composed of a drug-containing layer, an osmotic push layer, a coating membrane, and an overcoat.

Preferably, the drug-containing layer is composed of levodopa, carbidopa, microcrystalline cellulose, mannitol, citric acid, sodium hydroxypropyl methyl cellulose and magnesium stearate. In another embodiment, the drug-containing layer is composed of levodopa, microcrystalline cellulose, hydroxypropyl methyl cellulose and magnesium stearate. In another embodiment, the drug-containing layer is composed of levodopa, carbidopa, mannitol, citric acid and magnesium stearate. In another embodiment, the drug-containing layer is composed of levodopa, carbidopa, hydroxypropyl cellulose, mannitol, citric acid and magnesium stearate. In another embodiment, the drug-containing layer is composed of levodopa, carbidopa, hydroxypropyl cellulose, mannitol, citric acid, and povidone K30. In another embodiment, the drug-containing layer is composed of levodopa, hydroxypropyl cellulose, mannitol, povidone K30, magnesium stearate, Mint Flavor and aspartame. In another embodiment, the drug-containing layer is composed of levodopa, mannitol, povidone K30 and magnesium stearate. In another embodiment, the drug-containing layer is composed of levodopa, carbidopa, hydroxypropyl cellulose, mannitol, aspartame and magnesium stearate. In another embodiment, the drug-containing layer is composed of levodopa, hydroxypropyl cellulose, mannitol, povidone K30, magnesium stearate, Mint Flavor and aspartame. In another embodiment, the drug-containing layer is composed of levodopa, hydroxypropyl cellulose, mannitol, povidone K30, magnesium stearate, and aspartame. In another embodiment, the drug-containing layer is composed of levodopa, hydroxypropyl cellulose, mannitol, magnesium stearate, Mint Flavor and aspartame.

The osmotic push layer is composed of sodium carboxymethyl cellulose, povidone K30, sorbitol, iron oxide red and magnesium stearate, or the osmotic push layer is composed of sodium carboxymethyl cellulose, hydroxypropyl cellulose, sorbitol, iron oxide red and magnesium stearate. Preferably, the sodium carboxymethyl cellulose is sodium carboxymethyl cellulose 7H4XF or 9H4XF.

In one embodiment, the overcoat is composed of levodopa, carbidopa, hydroxypropyl cellulose, aspartame, and Mint Flavor; or the overcoat is composed of levodopa, carbidopa, hydroxypropyl cellulose and aspartame. In another embodiment, the overcoat is composed of carbidopa, hydroxypropyl cellulose and aspartame. In another embodiment, the overcoat is composed of levodopa, hydroxypropyl cellulose, and Mint Flavor. It is well known to those skilled in the art that the above "include" can be replaced by "consist of'.

Preferably, the drug-containing layer is composed of levodopa, carbidopa, microcrystalline cellulose, mannitol, citric acid, sodium hydroxypropyl methyl cellulose and magnesium stearate, the weight percentage of levodopa is 40%, the weight percentage of carbidopa is 10.8%, the weight percentage of microcrystalline cellulose is 20%, the weight percentage of mannitol is 18.7%, the weight percentage of citric acid is 5%, the weight percentage of hydroxypropyl methyl cellulose sodium is 5%, and the weight percentage of magnesium stearate is 0.5%. The weight percentage is the weight percentage of each component of the drug-containing layer.

In another embodiment, the drug-containing layer is composed of levodopa, microcrystalline cellulose, hydroxypropyl methyl cellulose and magnesium stearate, and the weight percentage of the levodopa is 38%, the weight percentage of microcrystalline cellulose is 50%, the weight percentage of hydroxypropyl methyl cellulose is 10%, and the weight percentage of magnesium stearate is 2%, wherein the weight percentage is the weight percentage of each component of the drug-containing layer.

In another embodiment, the drug-containing layer is composed of levodopa, carbidopa, mannitol, citric acid and magnesium stearate. The weight percentage of the levodopa is 19.5%, and the weight percentage of carbidopa is 20%, the weight percentage of mannitol is 50%, the weight percentage of citric acid is 10%, and the weight percentage of magnesium stearate is 0.5%, wherein the weight percentage is the weight percentage of each component of the drug-containing layer.

In another embodiment, the drug-containing layer is composed of levodopa, carbidopa, hydroxypropyl cellulose, mannitol, citric acid and magnesium stearate, the weight percentage of the levodopa is 40 %, the weight percentage of carbidopa is 10.8%, the weight percentage of hydroxypropyl cellulose is 31%, the weight percentage of mannitol is 12.7%, and the weight percentage of citric acid is 5%, and the weight percentage of the magnesium stearate is 0.5%, wherein the weight percentage is the weight percentage of each component of the drug-containing layer.

In another embodiment, the drug-containing layer is composed of levodopa, carbidopa, hydroxypropyl cellulose, mannitol, citric acid and povidone K30, the weight percentage of levodopa is 40 %, the weight percentage of carbidopa is 10.8%, the weight percentage of hydroxypropyl cellulose is 31%, the weight percentage of mannitol is 12.7%, and the weight percentage of citric acid is 5%, and the weight percentage of povidone K30 is 0.5%, wherein the weight percentage is the weight percentage of each component of the drug-containing layer.

In another embodiment, the drug-containing layer is composed of levodopa, hydroxypropyl cellulose, mannitol, povidone K30, magnesium stearate, Mint Flavor and aspartame, the weight percentage of levodopa is 45%, the weight percentage of hydroxypropyl cellulose is 31%, the weight percentage of mannitol is 16%, the weight percentage of povidone K30 is 5%, the weight percentage of the magnesium stearate is 1%, the weight percentage of the Mint Flavor is 1%, and the weight percentage of the aspartame is 1%, wherein the weight percentage is the weight percentage of each component of the drug-containing layer.

In another embodiment, the drug-containing layer is composed of levodopa, mannitol, povidone K30, and magnesium stearate, the weight percentage of levodopa is 70%, the weight percentage of mannitol is 9%, the weight percentage of povidone K30 is 20%, and the weight percentage of magnesium stearate is 1%, wherein the weight percentage is the weight percentage of each component of the drug-containing layer.

In another embodiment, the drug-containing layer is composed of levodopa, carbidopa, hydroxypropyl cellulose, mannitol, aspartame and magnesium stearate, the weight percentage of levodopa is 20%, the weight percentage of carbidopa is 20%, the weight percentage of hydroxypropyl cellulose is 50%, the weight percentage of mannitol is 4%, and the weight percentage of aspartame is 5%, and the weight percentage of magnesium stearate is 1%, wherein the weight percentage is the weight percentage of each component of the drug-containing layer.

In another embodiment, the drug-containing layer is composed of levodopa, hydroxypropyl cellulose, mannitol, povidone K30, magnesium stearate, Mint Flavor and aspartame, the weight percentage of levodopa is 45%, the weight percentage of the hydroxypropyl cellulose is 31%, the weight percentage of the mannitol is 17%, the weight percentage of the povidone K30 is 5%, the weight percentage of magnesium stearate is 1% and the weight percentage of aspartame is 1%, wherein the weight percentage is the weight percentage of each component of the drug-containing layer.

In another embodiment, the drug-containing layer is composed of levodopa, hydroxypropyl cellulose, mannitol, magnesium stearate, Mint Flavor and aspartame. The weight percentage of the levodopa is 62.5%, the weight percentage of the hydroxypropyl cellulose is 31%, the weight percentage of the mannitol is 4.5%, the weight percentage of the Mint Flavor is 0.1%, the weight percentage of aspartame is 0.9% and the weight percentage of magnesium stearate is 1%, wherein the weight percentage is the weight percentage of each component of the drug-containing layer.

In another embodiment, the drug-containing layer is composed of levodopa, hydroxypropyl cellulose, mannitol, magnesium stearate, Mint Flavor and aspartame. The weight percentage of the levodopa is 46.9%, the weight percentage of the hydroxypropyl cellulose is 31%, the weight percentage of the mannitol is 20.1%, the weight percentage of the Mint Flavor is 0.1%, the weight percentage of aspartame is 0.9%, and the weight percentage of magnesium stearate is 1%, wherein the weight percentage is the weight percentage of each component of the drug-containing layer.

In another embodiment, the drug-containing layer is composed of levodopa, hydroxypropyl cellulose, mannitol, povidone K30, magnesium stearate and aspartame. The weight percentage of the levodopa is 45%, the weight percentage of the hydroxypropyl cellulose is 31%, the weight percentage of the mannitol is 17%, the weight percentage of the povidone K30 is 5%, the weight of the magnesium stearate is 1%, and the weight percentage of the aspartame is 1%, wherein the weight percentage is the weight percentage of each component of the drug-containing layer.

In another embodiment, the drug-containing layer is composed of levodopa, hydroxypropyl cellulose, mannitol, povidone K30, magnesium stearate, Mint Flavor and aspartame. The weight percentage of the levodopa is 45%, the weight percentage of the hydroxypropyl cellulose is 31%, the weight percentage of the mannitol is 12%, and the weight percentage of the povidone K30 is 5%, the weight percentage of the Mint Flavor is 5%, the weight percentage of aspartame is 1%, and the weight percentage of magnesium stearate is 1%, wherein the weight percentage is the weight percentage of each component of the drug-containing layer.

Or when the drug-containing layer is composed of levodopa, hydroxypropyl cellulose, mannitol, magnesium stearate, Mint Flavor and aspartame, the drug-containing layer is composed of 45 wt% of levodopa, 31 wt% of hydroxypropyl cellulose, 22 wt% of mannitol, 0.9 wt% of aspartame, 1.0 wt% of magnesium stearate and 0.1 wt% of Mint Flavor; wherein the weight percentage is the weight percentage of each component of the drug-containing layer.

More preferably, the coating membrane is composed of 50 wt% of cellulose acetate membrane and 50 wt% of copovidone VA64; the coating membrane is composed of 70 wt% of cellulose acetate membrane and 30 wt% of copovidone VA64; the coating membrane is composed of 60 wt% of cellulose acetate membrane and 40 wt% of copovidone VA64; wherein the weight percentage is the weight percentage of each component of the coating membrane.

More preferably, the weight of the coating membrane is 2.0%, 4.2%, 4.5%, 4.6%, 4.8%, 5.0%, 5.9%, 6.5%, 6.7%, 7.0%, 7.7%, 7.9% or 9.7% of the weight of the tablet core.

Preferably, when the osmotic push layer is composed of sodium carboxymethyl cellulose 7H4XF, povidone K30, sorbitol, iron oxide red and magnesium stearate,

In one embodiment, the weight percentage of the carboxymethyl cellulose sodium 7H4XF is 55%, the weight percentage of the povidone K30 is 5%, the weight percentage of the sorbitol is 39%, the weight percentage of the iron oxide red is 0.5%, and the weight percentage of the magnesium stearate is 0.5%, wherein the weight percentage is the weight percentage of each component of the osmotic push layer.

In another embodiment, the weight percentage of the carboxymethyl cellulose sodium 7H4XF is 55%, the weight percentage of the povidone K30 is 10%, the weight percentage of the sorbitol is 34%, the weight percentage of the iron oxide red is 0.5%, and the weight percentage of the magnesium stearate is 0.5%, and the weight percentage is the weight percentage of each component of the osmotic push layer.

In another embodiment, the weight percentage of the sodium carboxymethyl cellulose is 85%, the weight percentage of the povidone K30 is 3%, the weight percentage of the sorbitol is 5%, the weight percentage of the iron oxide red is 5%, and the weight percentage of the magnesium stearate is 2%, wherein the weight percentage is the weight percentage of each component of the osmotic push layer.

In another embodiment, the weight percentage of the sodium carboxymethyl cellulose is 25%, the weight percentage of the povidone K30 is 9.5%, the weight percentage of the sorbitol is 65% and the weight percentage of the magnesium stearate is 0.5%, wherein the weight percentage is the weight percentage of each component of the osmotic push layer.

In another embodiment, the weight percentage of the carboxymethyl cellulose sodium 7H4XF is 60%, the weight percentage of the povidone K30 is 10%, the weight percentage of the sorbitol is 26%, the weight percentage of the iron oxide red is 2%, and the weight percentage of the magnesium stearate is 2%, wherein the weight percentage is the weight percentage of each component of the osmotic push layer.

In another embodiment, the weight percentage of the carboxymethyl cellulose sodium 7H4XF is 40%, the weight percentage of the povidone K30 is 20%, the weight percentage of the sorbitol is 36%, the weight percentage of the iron oxide red is 3.5%, and the weight percentage of the magnesium stearate is 0.5%, wherein the weight percentage is the weight percentage of each component of the osmotic push layer.

In another embodiment, the osmotic push layer is composed of sodium carboxymethyl cellulose 9H4XF, povidone K30, sorbitol, iron oxide red and magnesium stearate, the weight percentage of sodium carboxymethyl cellulose 9H4XF is 55%, the weight percentage of povidone K30 is 5%, the weight percentage of sorbitol is 39%, and the weight percentage of iron oxide red is 0.5 % and the weight percentage of magnesium stearate is 0.5%, wherein the weight percentage is the weight percentage of each component of the osmotic push layer.

Or, in another embodiment, the osmotic push layer is composed of sodium carboxymethyl cellulose 7H4XF, hydroxypropyl cellulose, sorbitol, iron oxide red and magnesium stearate, the weight percentage of sodium carboxymethyl cellulose 7H4XF is 55%, the weight percentage of hydroxypropyl cellulose is 10%, the weight percentage of sorbitol is 34 %, the weight percentage of the iron oxide red is 0.5% and the weight percentage of the magnesium stearate is 0.5%, wherein the weight percentage is the weight percentage of each component of the osmotic push layer.

More preferably, when the overcoat is composed of levodopa, carbidopa, hydroxypropyl cellulose, aspartame and Mint Flavor, the weight percentage of the levodopa is 23.78%, the weight percentage of the carbidopa is 64.22%, the weight percentage of the hydroxypropyl cellulose is 10%, and the weight percentage of the aspartame is 1%, and the weight percentage of the Mint Flavor is 1%, wherein the weight percentage is the weight percentage of each component of the overcoat.

In another embodiment, the overcoat is composed of carbidopa, hydroxypropyl cellulose and aspartame, the weight percentage of carbidopa is 93%, the weight percentage of hydroxypropyl cellulose is 2% and the weight percentage of aspartame is 5%, wherein the weight percentage is the weight percentage of each component of the overcoat.

In another embodiment, the overcoat is composed of levodopa, hydroxypropyl cellulose and Mint Flavor, the weight percentage of the levodopa is 75%, the weight percentage of the hydroxypropyl cellulose is 20%, and the weight percentage of the Mint Flavor is 5%, wherein the weight percentage is the weight percentage of each component of the overcoat.

In another embodiment, when the overcoat is composed of levodopa, carbidopa, hydroxypropyl cellulose, aspartame and Mint Flavor, the weight percentage of the levodopa is 62.15%, the weight percentage of the carbidopa is 26.85%, the weight percentage of the hydroxypropyl cellulose is 10%, and the weight percentage of the aspartame is 0.9%, and the weight percentage of the Mint Flavor is 0.1%, wherein the weight percentage is the weight percentage of each component of the overcoat.

When the overcoat is composed of levodopa, carbidopa, hydroxypropyl cellulose and aspartame, the weight percentage of levodopa is 24%, the weight percentage of carbidopa is 65%, the weight percentage of hydroxypropyl cellulose is 10% and the weight percentage of aspartame is 1 %, wherein the weight percentage is the weight percentage of each component of the overcoat.

When the overcoat is composed of levodopa, carbidopa, hydroxypropyl cellulose, aspartame and Mint Flavor, the weight percentage of the levodopa is 54%, the weight percentage of the carbidopa is 35%, the weight percentage of the hydroxypropyl cellulose is 10%, the weight percentage of the aspartame is 0.9%, and the weight percentage of the Mint Flavor is 0.1%; or, the weight percentage of the levodopa is 42.8%, the weight percentage of the carbidopa is 46.2%, the weight percentage of the hydroxypropyl cellulose is 10%, the weight percentage of the aspartame is 0.9%, the weight percentage of the Mint Flavor is 0.1%, or the weight percentage of the levodopa is 28.2%, and the weight percentage of the carbidopa is 60.8%, the weight percentage of the hydroxypropyl cellulose is 10%, the weight percentage of the aspartame is 0.9%, and the weight percentage of the Mint Flavor is 0.1%, wherein the weight percentage is the weight percentage of each component of the overcoat.

More preferably, the weight gain of the overcoat relative to the tablet core is 12.9% or 13.2% or 15.7% or 21.0% of weight percentage.

Preferably, the pharmaceutical composition is composed of a drug-containing layer and a coating membrane, the drug containing layer is composed of 40 wt% of levodopa, 10.8 wt% of carbidopa, 20 wt% of microcrystalline cellulose, 18.7 wt% of mannitol, 5 wt% of citric acid, 5 wt% of hydroxypropyl methyl cellulose sodium and 0.5 wt% of magnesium stearate, and the weight percentage is the weight percentage of each component of the drug-containing layer. The coating membrane is composed of 50% wt% of cellulose acetate membrane and 50 wt% of copovidone VA64, and the weight percentage is the weight percentage of each component of the coating membrane. The weight of the coating membrane is 2.0% of the weight of the tablet core. A dosage form containing the pharmaceutical composition has a 0.5 mm exit orifice mechanically drilled on the drug-containing layer side of the coated tablet, and levodopa and carbidopa are delivered at an average rate of 14.17 mg/hr and 4.59 mg/hr, with 85% of the drug delivered in 12 and 10 hours, respectively. The dosage form can be maintained in the oral cavity until the osmotic layer reaches the delivery orifice, or maintained in the oral cavity for 8-9 hours and then swallowed.

The pharmaceutical composition is composed of a drug-containing layer and a coating membrane, the drug-containing layer is composed of 38 wt% of levodopa, 50 wt% of microcrystalline cellulose, 10 wt% of hydroxypropyl methyl cellulose and 2 wt% of magnesium stearate, wherein the weight percentage is the weight percentage of each component of the drug-containing layer. The coating membrane is composed of 50 wt% of cellulose acetate membrane and 50 wt% of copovidone VA64, wherein the weight percentage is the weight percentage of each component of the coating membrane, and the weight of the coating membrane is 4.5% of the weight of tablet core. The dosage form with a membrane weight gain of 4.5% delivered levodopa at an average rate of 9.4 mg/hr, with 85% of levodopa delivered in 9.0 hours.

Or, the pharmaceutical composition is composed of a drug-containing layer and a coating membrane, the drug-containing layer comprises 19.5 wt% of levodopa, 20 wt% of carbidopa, 50 wt% of mannitol, 10 wt% of citric acid and 0.5 wt% of magnesium stearate, wherein the weight percentage is the weight percentage of each component of the drug-containing layer. The coating membrane is composed of 50 wt% of cellulose acetate membrane and 50 wt% of copovidone VA64, wherein the weight percentage is the weight percentage of each component of the coating membrane, and the weight of the coating membrane is 4.5% of the weight of tablet core. The dosage form with a membrane weight gain of 4.5% delivered levodopa at an average rate of 22.9 mg/hr, with 85% of levodopa delivered in 13.0 hours.

Preferably, in one embodiment, the pharmaceutical composition is composed of a drug-containing layer, an osmotic push layer, and a coating membrane, and the drug-containing layer is composed of 40 wt% of levodopa, 10.8 wt% of carbidopa, 31 wt% of hydroxypropyl cellulose, 12.7 wt% of mannitol, 5 wt% of citric acid and 0.5 wt% of magnesium stearate, wherein the weight percentage is the weight percentage of each component of the drug-containing layer. The osmotic push layer is composed of 55 wt% of sodium carboxymethyl cellulose 7H4XF or 9H4XF, 5 wt% of povidone K30, 39 wt% of sorbitol, 0.5 wt% of iron oxide red and 0.5 wt% of magnesium stearate, wherein the weight percentage is the weight percentage of each component of the osmotic push layer. The coating membrane is composed of 70 wt% of cellulose acetate membrane and 30 wt% of copovidone VA64, wherein the weight percentage is the weight percentage of each component of the coating membrane. The weight of the coating membrane is 2.0%, 4.0% or 5.0% of the weight of the tablet core. When the sodium carboxymethyl cellulose is 7H4XF, one side of the drug-containing layer contains an exit orifice of 0.5 mm, the dosage form with a tablet core weight gain of 5.0% delivered levodopa and carbidopa at an average rate of 17.0 mg/hr and 4.6 mg/hr, with 85% of levodopa and carbidopa delivered in 10.0 hours. The dosage form can be maintained in the oral cavity until the osmotic layer reaches the delivery orifice, or maintained in the oral cavity for 6-7 hours and then swallow. The sizes of delivery orifice vary from 0.5 mm, 0.75 mm and 1.0 mm. The dosage form with a tablet core weight gain of 4.0% delivered levodopa and carbidopa at an average rate of 21.3 mg/hr and 5.7 mg/hr, with 85% of levodopa and carbidopa delivered in 10.0 hours. The dosage form can be maintained in the oral cavity until the osmotic layer reaches the delivery orifice, or maintained in the oral cavity for 4-5 hours and then swallow. When the sodium carboxymethyl cellulose is 9H4XF, the dosage form with a coating membrane weight gain of 2.0% delivered levodopa and carbidopa at an average rate of 24.3 mg/hr and 6.6 mg/hr, with 85% levodopa and carbidopa delivered in 7.0 hours. The dosage form can be maintained in the oral cavity until the osmotic layer reaches the delivery port, or maintained in the oral cavity for 3-4 hours and then swallowed.

In another embodiment, the pharmaceutical composition is composed of a drug-containing layer, an osmotic push layer, and a coating membrane, the drug-containing layer is composed of 40 wt% of levodopa, 10.8 wt% of carbidopa, 31 wt% of hydroxypropyl cellulose, 12.7 wt% of mannitol, 5 wt% of citric acid and 0.5 wt% of povidone K30, wherein the weight percentage is the weight percentage of each component of the drug-containing layer. The osmotic push layer is composed of 55 wt% of sodium carboxymethyl cellulose 7H4XF, 5 wt% of povidone K30, 39 wt% of sorbitol, 0.5 wt% of iron oxide red, and 0.5 wt% of magnesium stearate, wherein the weight percentage is the weight percentage of each component of the osmotic push layer; the coating membrane is composed of 60 wt% of cellulose acetate membrane and 40% copovidone VA64, wherein the weight percentage is the weight percentage of each component of the coating membrane; and the weight of the coating membrane is 5.0% of the weight of the tablet core. The dosage form containing the pharmaceutical composition delivered 85% of the drug in 6 hours. The dosage form can be maintained in the oral cavity until the osmotic layer reaches the delivery port, or maintained in the oral cavity for 2-3 hours, and then swallowed.

In another embodiment, the pharmaceutical composition is composed of a drug-containing layer, an osmotic push layer, and a coating membrane, the drug-containing layer is composed of 45 wt% of levodopa, 31 wt% of hydroxypropyl cellulose, 16 wt% of mannitol, 5 wt% of povidone K30, and 1 wt% of magnesium stearate, 1 wt% of Mint Flavor and 1 wt% of aspartame, wherein the weight percentage is the weight percentage of each component of the drug-containing layer; the osmotic push layer is composed of 55 wt% of sodium carboxymethyl cellulose 7H4XF, 10 wt% of povidone K30, 34 wt% of sorbitol, 0.5 wt% of iron oxide red and 0.5 wt% of magnesium stearate, wherein the weight percentage is the weight percentage of each component of the osmotic push layer; the coating membrane is composed of 60 wt% of cellulose acetate membrane and 40 wt% of copovidone VA64, wherein the weight percentage is the weight percentage of each component of the coating membrane; and the weight of the coating membrane is 4.2%, 6.7% or 9.7% of the weight of the tablet core. The dosage form containing the pharmaceutical composition with a membrane weight gain of 4.2%, 6.7% and 9.7% delivered levodopa at an average rate of 38.3 mg/hr, 27.3 mg/hr, and 21.3 mg/hr, with 85% of levodopa delivered in 5.0 hours, 7.0 hours and 9.0 hours, respectively.

In another embodiment, the pharmaceutical composition is composed of a drug-containing layer, an osmotic push layer, and a coating membrane, the drug-containing layer is composed of 45 wt% of levodopa, 31 wt% of hydroxypropyl cellulose, 16 wt% of mannitol, 5 wt% of povidone K30, 1 wt% of magnesium stearate, 1 wt% of Mint Flavor and 1 wt% of aspartame, wherein the weight percentage is the weight percentage of each component of the drug-containing layer; the osmotic push layer is composed of 55 wt% of sodium carboxymethyl cellulose 7H4XF, 10 wt% of povidone K30, 34 wt% of sorbitol, 0.5 wt% of iron oxide red and 0.5 wt% of magnesium stearate, wherein the weight percentage is the weight percentage of each component of the osmotic push layer; the coating membrane is composed of 70 wt% of cellulose acetate membrane and 30 wt% of copovidone VA64, wherein the weight percentage is the weight percentage of each component of the coating membrane; and the weight of the coating membrane is 4.6% or 7.9% of the weight of the tablet core. The dosage form containing the pharmaceutical composition with a membrane weight gain of 4.6% and 7.9% delivered levodopa at an average rate of 25.5 mg/hr and 16.9 mg/hr, with 85% of levodopa delivered in 7.5 hours and 11.5 hours, respectively.

In another embodiment, the pharmaceutical composition is composed of a drug-containing layer, an osmotic push layer, and a coating membrane, the drug-containing layer is composed of 70 wt% of levodopa, 9 wt% of mannitol, 20 wt% of povidone K30, and 1 wt% of magnesium stearate, wherein the weight percentage is the weight percentage of each component of the drug-containing layer; the osmotic push layer is composed of 85 wt% of sodium carboxymethyl cellulose, 3 wt% of povidone K30, 5 wt% of sorbitol, 5 wt% of iron oxide red and 2 wt% of magnesium stearate, wherein the weight percentage is the weight percentage of each component of the osmotic push layer; the coating membrane is composed of 70 wt% of cellulose acetate membrane and 30 wt% of copovidone VA64, wherein the weight percentage is the weight percentage of each component of the coating membrane; and the weight of the coating membrane is 4.5% of the weight of the tablet core. The dosage form containing the pharmaceutical composition delivered levodopa at an average rate of 35.0 mg/hr, with 85% of levodopa delivered in 8.5 hours.

In another embodiment, the pharmaceutical composition is composed of a drug-containing layer, an osmotic push layer, and a coating membrane, the drug-containing layer is composed of 20 wt% of levodopa, 20 wt% of carbidopa, 50 wt% of hydroxypropyl cellulose, 4 wt% of mannitol, 5 wt% of aspartame and 1 wt% of magnesium stearate, wherein the weight percentage is the weight percentage of each component of the drug-containing layer; the osmotic push layer is composed of 25 wt% of sodium carboxymethyl cellulose, 9.5 wt% of povidone K30, 65 wt% of sorbitol and 0.5 wt% of magnesium stearate, wherein the weight percentage is the weight percentage of each component of the osmotic push layer; the coating membrane is composed of 90 wt% of cellulose acetate membrane and 10 wt% of copovidone VA64, wherein the weight percentage is the weight percentage of each component of the coating membrane; and the weight of the coating membrane is 4.5% of the weight of the tablet core. The dosage form containing the pharmaceutical composition delivered levodopa and carbidopa at an average rate of 7.1 mg/hr, with 85% of levodopa and carbidopa delivered in 12 hours.

Preferably, in one embodiment, the pharmaceutical composition is composed of a drug-containing layer, an osmotic push layer, a coating membrane and an overcoat, the drug-containing layer is composed of 45 wt% of levodopa, 31 wt% of hydroxypropyl cellulose, 16 wt% of mannitol, 5 wt% of povidone K30, 1 wt% of magnesium stearate, 1 wt% of Mint Flavor and 1 wt% of aspartame, wherein the weight percentage is the weight percentage of each component of the drug-containing layer; the osmotic push layer is composed of 55 wt% of sodium carboxymethyl cellulose 7H4XF, 10 wt% of povidone K30, 34 wt% of sorbitol, 0.5 wt% of iron oxide red and 0.5 wt% of magnesium stearate, wherein the weight percentage is the weight percentage of each component of the osmotic push layer; the coating membrane is composed of 70 wt% of cellulose acetate membrane and 30 wt% of copovidone VA64, wherein the weight percentage is the weight percentage of each component of the coating membrane; and the weight of the coating membrane is 4.8% or 7.7% of the weight of the tablet core, the overcoat is composed of 23.78 wt% of levodopa, 64.22 wt% of carbidopa, 10 wt% of hydroxypropyl cellulose, 1 wt% of aspartame and 1 wt% of Mint Flavor immediate release composition, wherein the weight percentage is the weight percentage of each component of the overcoat, and the weight gain of the overcoat relative to the tablet core is 13.2 % and 12.9 % by weight, respectively. The release profile of the dosage form containing the pharmaceutical composition showed a rapid release of levodopa/carbidopa, the dosage form with 4.8% and 7.7% weight gain in coating membrane followed by sustained release with a release duration of approximately 8.5 hours and 12.0 hours, respectively. The dosage form with a 4.8% membrane weight gain can be maintained in the oral cavity for 4-5 hours and then maintained in the oral cavity for the duration of the meal or throughout the release period. Dosage forms with a 7.7 % membrane weight gain can be maintained in the oral cavity for 8-9 hours before swallowing, or be maintained in the oral cavity for the entire release period.

In another embodiment, the pharmaceutical composition is composed of a drug-containing layer, an osmotic push layer, a coating membrane and an overcoat, the drug-containing layer is composed of 45 wt% of levodopa, 31 wt% of hydroxypropyl cellulose, 17 wt% of mannitol, 5 wt% of povidone K30, 1 wt% of magnesium stearate and 1 wt% of aspartame, wherein the weight percentage is the weight percentage of each component of the drug-containing layer; the osmotic push layer is composed of 60 wt% of sodium carboxymethyl cellulose 7H4XF, 10 wt% of povidone K30, 26 wt% of sorbitol, 2 wt% of iron oxide red and 2 wt% of magnesium stearate, wherein the weight percentage is the weight percentage of each component of the osmotic push layer; the coating membrane is composed of 70 wt% of cellulose acetate membrane and 30 wt% of copovidone VA64, wherein the weight percentage is the weight percentage of each component of the coating membrane; and the weight of the coating membrane is 4.8% of the weight of the tablet core, the overcoat is composed of 93 wt% of CD, 2 wt% of hydroxypropyl cellulose and 5 wt% of aspartame immediate release composition, wherein the weight percentage is the weight percentage of each component of the overcoat, and the weight increase of the overcoat relative to the tablet core is 13.2 wt% of. The immediate release overcoat of the dosage form is rapidly released first, followed by sustained releasing for approximately 8 hours. The dosage form can be maintained in the oral cavity for 4-5 hours and then swallow before eating or be maintained in the oral cavity for the entire release period.

In another embodiment, the pharmaceutical composition is composed of a drug-containing layer, an osmotic push layer, a coating membrane and an overcoat, the drug-containing layer is composed of 45 wt% of levodopa, 31 wt% of hydroxypropyl cellulose, 12 wt% of mannitol, 5 wt% of povidone K30, 5 wt% of Mint Flavor, 1 wt% of aspartame and 1 wt% of magnesium stearate, wherein the weight percentage is the weight percentage of each component of the drug-containing layer; the osmotic push layer is composed of 40 wt% of sodium carboxymethyl cellulose 7H4XF, 20 wt% of povidone K30, 36 wt% of sorbitol, 3.5 wt% of iron oxide red and 0.5 wt% of magnesium stearate, wherein the weight percentage is the weight percentage of each component of the osmotic push layer; the coating membrane is composed of 70 wt% of cellulose acetate membrane and 30 wt% of copovidone VA64, wherein the weight percentage is the weight percentage of each component of the coating membrane; and the weight of the coating membrane is 4.8% of the weight of the tablet core, the overcoat is composed of 75 wt% of levodopa, 20 wt% of hydroxypropyl cellulose and 5 wt% of Mint Flavor immediate release composition, wherein the weight percentage is the weight percentage of each component of the overcoat, and the weight gain of the overcoat relative to the tablet core is 13.2 wt%. The immediate release overcoat of the dosage form is rapidly released first, followed by sustained releasing for approximately 8 hours. The dosage form can be maintained in the oral cavity for 4-5 hours and then swallow before eating or be maintained in the oral cavity for the entire period of releasing.

In another embodiment, the pharmaceutical composition is composed of a drug-containing layer, an osmotic push layer, a coating membrane and an overcoat, the drug-containing layer is composed of 62.5 wt% of levodopa, 31 wt% of hydroxypropyl cellulose, 4.5 wt% of mannitol, 0.1 wt% of Mint Flavor, 0.9 wt% of aspartame and 1 wt% of magnesium stearate, wherein the weight percentage is the weight percentage of each component of the drug-containing layer; the osmotic push layer is composed of 55 wt% of sodium carboxymethyl cellulose 7H4XF, 10 wt% of hydroxypropyl cellulose, 34 wt% of sorbitol, 0.5 wt% of iron oxide red and 0.5 wt% of magnesium stearate, wherein the weight percentage is the weight percentage of each component of the osmotic push layer; the coating membrane is composed of 70 wt% of cellulose acetate membrane and 30 wt% of copovidone VA64, wherein the weight percentage is the weight percentage of each component of the coating membrane; and the weight of the coating membrane is 6.5% of the weight of the tablet core, the overcoat is composed of 62.15 wt% of levodopa, 26.85 wt% of carbidopa, 10 wt% of hydroxypropyl cellulose, 0.9 wt% of aspartame and 0.1 wt% of Mint Flavor immediate release composition, wherein the weight percentage is the weight percentage of each component of the overcoat, and the weight gain of the overcoat relative to the tablet core is 21.0 wt%.

In another embodiment, the pharmaceutical composition is composed of a drug-containing layer, an osmotic push layer, a coating membrane and an overcoat, the drug-containing layer is composed of 46.9 wt% of levodopa, 31 wt% of hydroxypropyl cellulose, 20.1 wt% of mannitol, 0.1 wt% of Mint Flavor, 0.9 wt% of aspartame and 1 wt% of magnesium stearate, wherein the weight percentage is the weight percentage of each component of the drug-containing layer; the osmotic push layer is composed of 55 wt% of sodium carboxymethyl cellulose 7H4XF, 10 wt% of hydroxypropyl cellulose, 34 wt% of sorbitol, 0.5 wt% of iron oxide red and 0.5 wt% of magnesium stearate, wherein the weight percentage is the weight percentage of each component of the osmotic push layer; the coating membrane is composed of 70 wt% of cellulose acetate membrane and 30 wt% of copovidone VA64, wherein the weight percentage is the weight percentage of each component of the coating membrane; and the weight of the coating membrane is 6.5% of the weight of the tablet core, the overcoat is composed of 62.15 wt% of levodopa, 26.85 wt% of carbidopa, 10 wt% of hydroxypropyl cellulose, 0.9 wt% of aspartame and 0.1 wt% of Mint Flavor immediate release composition, wherein the weight percentage is the weight percentage of each component of the overcoat, and the weight increase of the overcoat relative to the tablet core is 15.7 wt%.

In a specific preferred embodiment, the pharmaceutical composition is composed of a drug-containing layer, an osmotic push layer, a coating membrane and an overcoat; wherein the drug-containing layer is composed of 45 wt% of levodopa, 31 wt% of hydroxypropyl cellulose, 16 wt% of mannitol, 5 wt% of povidone K30, 1 wt% of magnesium stearate, 1 wt% of Mint Flavor and 1 wt% of aspartame, wherein the weight percentage is the weight percentage of each component of the drug-containing layer; the osmotic push layer is composed of 55 wt% of sodium carboxymethyl cellulose 7H4XF, 10 wt% of hydroxypropyl cellulose, 34 wt% of sorbitol, 0.5 wt% of iron oxide red and 0.5 wt% of magnesium stearate, wherein the weight percentage is the weight percentage of each component of the osmotic push layer; the coating membrane is composed of 70 wt% of cellulose acetate membrane and 30 wt% of copovidone VA64, wherein the weight percentage is the weight percentage of each component of the coating membrane; wherein, the cellulose acetate is a cellulose acetate membrane containing 39.8 wt% of acetyl, and the weight of the coating membrane is 5.9 % of the weight of the tablet core; and, the overcoat is composed of 64.22 wt% of levodopa, 23.78 wt% of carbidopa, 10 wt% of hydroxypropyl cellulose, 1 wt% of aspartame and 1 wt% of Mint Flavor, wherein the weight percentage is the weight percentage of each component of the overcoat, the weight of the overcoat is 13.1% of the total weight of the tablet core and the coating membrane. When the solvent for the coating suspension of the overcoat is anhydrous ethanol, the content of carbidopa-related genotoxic impurity hydrazine in the obtained dosage form is 1.7 ppm, the content of carbidopa-related impurity dihydroxyphenyl- acetone (DHPA) is 0.21%. When the solvent for the coating suspension of the overcoat is purified water, the concentration of the solid content of the overcoat is 10.0 wt%, including 24.0% wt% of levodopa, 65.0 wt% of carbidopa monohydrate, 10.0 wt% of hydroxylpropyl cellulose and 1.0 wt% of aspartame in weight percentage; the content of carbidopa-related genotoxic impurity hydrazine in the obtained dosage form is 3.8 ppm, and the content of carbidopa-related impurity DHPA is 0.28%. When the coating solution solvent of the overcoat is anhydrous ethanol, the carbidopa-related genotoxic impurity hydrazine and impurity DHPA of the obtained dosage form are significantly lower than the coating solution solvent of the overcoat is purified water. The immediate release overcoat of the dosage form is rapidly released first, followed by a sustained release with a duration of approximately 8 hours. The osmotic delivery system can be maintained in the oral cavity for 3-5 hours and then swallow before eating or be maintained in the oral cavity for the entire release period.

In a specific preferred embodiment, the pharmaceutical composition is composed of a drug-containing layer, an osmotic push layer, a coating membrane and an overcoat; the drug-containing layer is composed of 45 wt% of levodopa, 31 wt% of hydroxypropyl cellulose, 22 wt% of mannitol, 0.9 wt% of aspartame, 0.5 wt% of magnesium stearate and 0.1 wt% of Mint Flavor, wherein the weight percentage is the weight percentage of each component of the drug-containing layer; the osmotic push layer is composed of 55 wt% of sodium carboxymethyl cellulose 7H4XF, 10 wt% of hydroxypropyl cellulose, 34 wt% of sorbitol, 0.5 wt% of iron oxide red and 0.5 wt% of magnesium stearate, wherein the weight percentage is the weight percentage of each component of the osmotic push layer; the coating membrane is composed of 70 wt% of cellulose acetate membrane and 30 wt% of copovidone VA64, wherein the weight percentage is the weight percentage of each component of the coating membrane; wherein, the cellulose acetate is a cellulose acetate membrane containing 39.8 wt% of acetyl, and the weight of the coating membrane is 6.5 % of the weight of the tablet core; and, the overcoat is composed of 54 wt% of levodopa, 35 wt% of carbidopa, 10 wt% of hydroxypropyl cellulose, 0.9 wt% of aspartame and 0.1 wt% of Mint Flavor, wherein the weight percentage is the weight percentage of each component of the overcoat, the weight of the overcoat is 13.1% of the total weight of the tablet core and the coating membrane. The final dosage form is composed of an immediate release overcoat of 62.5 mg levodopa and 37.5 mg carbidopa, and 187.5 mg levodopa was contained in an extended release drug-containing layer. The immediate release overcoat of the dosage form is rapidly released first, followed by a sustained release with a duration of approximately 8 hours. The osmotic delivery system can be maintained in the oral cavity for 4-5 hours and then swallow before eating or be maintained in the oral cavity for the entire release period.

In a specific preferred embodiment, the pharmaceutical composition is composed of a drug-containing layer, an osmotic push layer, a coating membrane and an overcoat; the drug-containing layer is composed of 45 wt% of levodopa, 31 wt% of hydroxypropyl cellulose, 22 wt% of mannitol, 0.9 wt% of aspartame, 1 wt% of magnesium stearate and 0.1 wt% of Mint Flavor, wherein the weight percentage is the weight percentage of each component of the drug-containing layer; the osmotic push layer is composed of 55 wt% of sodium carboxymethyl cellulose 7H4XF, 10 wt% of hydroxypropyl cellulose, 34 wt% of sorbitol, 0.5 wt% of iron oxide red and 0.5 wt% of magnesium stearate, wherein the weight percentage is the weight percentage of each component of the osmotic push layer; the coating membrane is composed of 70 wt% of cellulose acetate membrane and 30 wt% of copovidone VA64, wherein the weight percentage is the weight percentage of each component of the coating membrane; wherein, the cellulose acetate is a cellulose acetate membrane containing 39.8 wt% of acetyl, and the weight of the coating membrane is 7.0 % of the weight of the tablet core; and, the overcoat is composed of 42.8 wt% of levodopa, 46.2 wt% of carbidopa, 10 wt% of hydroxypropyl cellulose, 0.9 wt% of aspartame and 0.1 wt% of Mint Flavor, wherein the weight percentage is the weight percentage of each component of the overcoat, the weight of the overcoat is 13.1% of the total weight of the tablet core and the coating membrane. The final dosage form is composed of an immediate release overcoat of 37.5 mg levodopa and 37.5 mg carbidopa, and 112.5 mg levodopa was contained in an extended release drug-containing layer. The immediate release overcoat of the dosage form is rapidly released first, followed by a sustained release with a duration of approximately 8 hours. The osmotic delivery system can be maintained in the oral cavity for 4-5 hours and then swallow before eating or be maintained in the oral cavity for the entire release period.

In a specific preferred embodiment, the pharmaceutical composition is composed of a drug-containing layer, an osmotic push layer, a coating membrane and an overcoat; the drug-containing layer is composed of 45 wt% of levodopa, 31 wt% of hydroxypropyl cellulose, 22 wt% of mannitol, 0.9 wt% of aspartame, 1 wt% of magnesium stearate and 0.1 wt% of Mint Flavor, wherein the weight percentage is the weight percentage of each component of the drug-containing layer; the osmotic push layer is composed of 55 wt% of sodium carboxymethyl cellulose 7H4XF, 10 wt% of hydroxypropyl cellulose, 34 wt% of sorbitol, 0.5 wt% of iron oxide red and 0.5 wt% of magnesium stearate, wherein the weight percentage is the weight percentage of each component of the osmotic push layer; the coating membrane is composed of 70 wt% of cellulose acetate membrane and 30 wt% of copovidone VA64, wherein the weight percentage is the weight percentage of each component of the coating membrane; wherein, the cellulose acetate is a cellulose acetate membrane containing 39.8 wt% of acetyl, and the weight of the coating membrane is 9.0 % of the weight of the tablet core; and, the overcoat is composed of 28.2 wt% of levodopa, 60.8 wt% of carbidopa, 10 wt% of hydroxypropyl cellulose, 0.9 wt% of aspartame and 0.1 wt% of Mint Flavor, wherein the weight percentage is the weight percentage of each component of the overcoat, the weight of the overcoat is 13.1% of the total weight of the tablet core and the coating membrane. The final dosage form is composed of an immediate release overcoat of 18.75 mg levodopa and 37.5 mg carbidopa, and 56.25 mg levodopa was contained in an extended release drug-containing layer. The immediate release overcoat of the dosage form is rapidly released first, followed by a sustained release with a duration of approximately 8 hours. The osmotic delivery system can be maintained in the oral cavity for 4-5 hours and then swallow before eating or be maintained in the oral cavity for the entire release period.

Wherein, the preparation method of the overcoat contains: dissolving each component of the above-mentioned weight percent of the overcoat in anhydrous ethanol to prepare a coating suspension. Preferably, the ratio of the components of the overcoat to the anhydrous ethanol is 1:10. In the pharmaceutical composition, carbidopa is only present in an immediate release overcoat, and the core portion of the extended release tablet does not contain carbidopa. The dosage form has the following advantages: the content of carbidopa related genotoxic impurity hydrazine and impurity dihydroxyphenylacetone (DHPA) is lower. When the solvent for the coating suspension of the overcoat is anhydrous ethanol, the carbidopa related genotoxic impurity hydrazine and impurity DHPA of the obtained dosage form are significantly lower than that of the obtained dosage form when the solvent for the coating suspension is pure water.

Unless otherwise specified, the method for preparing the aforementioned pharmaceutical composition is a conventional preparation method in the art.

Preferably, as described above, the pharmaceutical composition is an osmotic pump extended release drug delivery system. Preferably, the osmotic pump extended release drug delivery system is an extended release tablet. More preferably, the extended release tablet is a cylinder with a diameter of 5-10 mm and a height of 5-30 mm, or a caplet with a length of 10-25 mm and a width of 5-10 mm. Most preferably, each of the extended release tablets contains 62.5 mg CD and 500 mg LD, or 62.5 mg CD and 375 mg LD, or 62.5 mg carbidopa and 250 mg levodopa, or 50 mg carbidopa and 500 mg levodopa, or 37.5mg carbidopa and 375mg levodopa.

To solve the above technical problems, one of the technical solutions of the present invention is: a method for using the above extended release dosage form, that is, placing the extended release platform in the personalized retention enabling platform, and fastening the retention enabling platform on the corresponding teeth in the oral cavity; taking out the extended release dosage form after keeping for 4-24 hours, replacing the extended release platform with a new one and fastening the retention enabling platform on the corresponding teeth in the oral cavity again, so that the drug can be released continuously and stably.

Without violating the common knowledge in the art, the preferred parameters described above may be optionally combined to obtain the preferred embodiments in the present invention.

The reagents and raw materials employed in the present invention are commercially available.

The advantageous effects achieved by the present invention are as follows: The invention provides an extended release system of an active pharmaceutical ingredient (API) capable of improving absorption window and a preparation method thereof, so as to provide non-invasive extended release products having a stable plasma profiles of active pharmaceutical ingredient such as LD, or LD and CD for the treatment of patients with PD, especially those with advanced PD.

### Brief description of the drawings

Fig.1 is a schematic diagram of an extended release platform (ERP): a. Single-layer elementary osmotic pump (EOP); b. Bi-layer osmotic push-pull system.
Fig. 2 is the schematic diagram of extended release dosage form (combination of extended release platform ERP and retention enabling platform REP): 1. Personalized retention enabling modules of retention enabling platform (REP); 2. Extended release platform (ERP); 3. Drug fastened modules of retention enabling platform (REP).
Fig. 3 is a flow chart of manufacturing the extended release dosage form (ERP + REP) of the present invention: a. The extended release platform is an extended release dosage form of a single-layer elementary osmotic pump (EOP); b. The extended release platform is an extended release dosage form of a bi-layer osmotic push-pull system; c. The extended release platform is an extended release dosage form of a bi-layer push-pull system with an immediate-release drug overcoat.
Fig. 4 shows the preparation of the retention enabling platform (REP) described in embodiment 1: a. Drug fastened module; b. Drug fastened module; c. Drug fastened module; d. Drug fastened module; e. Thermoplastic tablet; f. Personalized retention enabling platform (REP).
Fig. 5 is a drug fastened module described in embodiment 1: a. Drug fastened module; b. Extended release platform fastened in the drug fastened module.

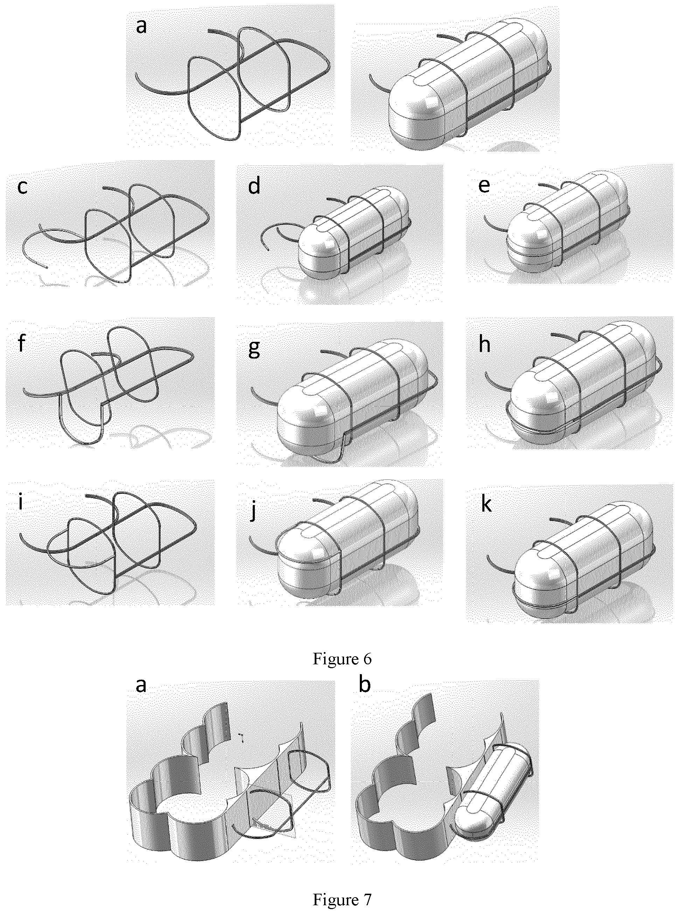

Fig. 6 shows the retention enabling platform (REP) described in embodiment 2 and embodiment 3: a. Retention enabling platform (REP); b. Extended release platform fastened in the Retention enabling platform (REP); c. Retention enabling platform (REP) with self-locking cover; d. Extended release platform fastened in the retention enabling platform (REP); e. Extended release platform with the cover closed + retention enabling platform (REP); f. Retention enabling platform with reversible cover (REP); g. Extended release platform fastened in the retention enabling platform (REP); h. Extended release platform with the cover closed + retention enabling platform (REP); i. Retention enabling platform (REP) with a cover that can slide up and down; j. Extended release platform fastened to the retention enabling platform (REP); k. Extended release platform with cover closed + Retention enabling platform (REP);
Fig. 7 is a retention enabling platform (REP) described in embodiment 4 and embodiment 5: a. Retention enabling platform (REP); b. Extended release platform fastened in the retention enabling platform (REP);
Fig. 8 is a release profile of the extended release platform (ERP) described in embodiment 6, and error bars indicate a standard deviation of n = 3.
Fig. 9 is a release profile of the extended release platform (ERP) described in embodiment 7, and error bars indicate a standard deviation of n = 3.
Fig. 10 is a release profile of the extended release platform (ERP) described in embodiment 8, and error bars indicate a standard deviation of n = 3.
Fig. 11 is a release profile of the extended release platform (ERP) described in embodiment 9, and error bars indicate a standard deviation of n = 3.
Fig. 12 is a release profile of the extended release platform (ERP) described in embodiment 10, and error bars indicate a standard deviation of n = 3.
Fig. 13 is a release profile of the extended release platform (ERP) described in embodiment 11, and error bars indicate a standard deviation of n = 3.
Fig. 14 is a release profile of the extended release platform (ERP) described in embodiment 12, and error bars indicate a standard deviation of n = 3.
Fig. 15 is a release profile of the extended release platform (ERP) described in embodiment 13, and error bars indicate a standard deviation of n = 3.
Fig. 16 is a release profile of the extended release platform (ERP) described in embodiment 14, and error bars indicate a standard deviation of n = 3.
Fig. 17 is a release profile of the extended release platform (ERP) described in embodiment 15, and error bars indicate a standard deviation of n = 3.
Fig. 18 is a release profile of the extended release platform (ERP) described in embodiment 16, and error bars indicate a standard deviation of n = 3.
Fig. 19 is a release profile of the extended release platform (ERP) described in embodiment 17, and error bars indicate a standard deviation of n = 3.
Fig. 20 is a release profile of the extended release platform (ERP) described in embodiment 18, and error bars indicate a standard deviation of n = 3.
Fig. 21 is a release profile of the extended release platform (ERP+REP) described in embodiment 19, and error bars indicate a standard deviation of n = 3.

### Detailed description of the preferred embodiment

The following embodiments further illustrate the present invention, but the present invention is not limited thereto. The experimental methods without specific conditions specified in the following embodiments were selected according to conventional methods and conditions, or according to product specifications. Active pharmaceutical ingredients (APIs) for the present invention include one or more of levodopa and carbidopa.

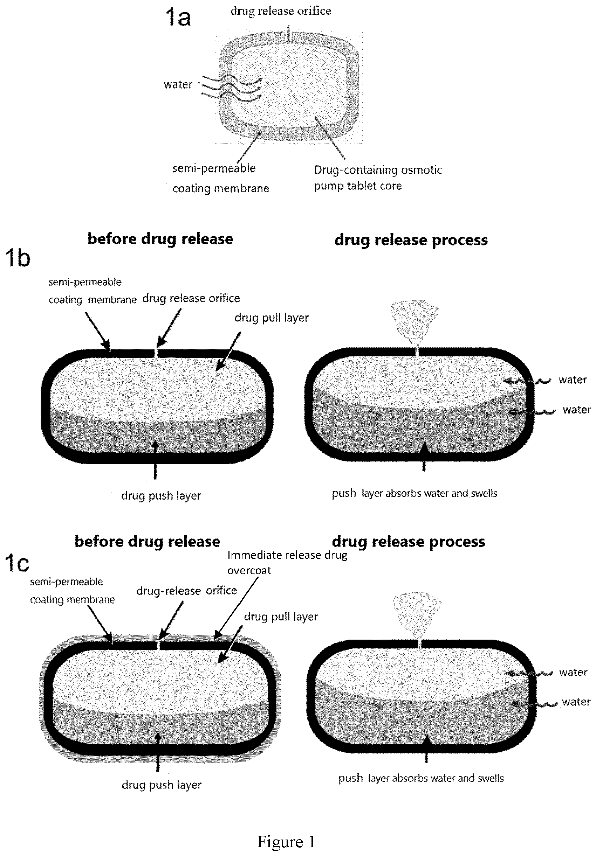

In one embodiment, the active pharmaceutical ingredient (API) is levodopa/carbidopa, which is incorporated in a single-layer elementary osmotic pump (EOP) known in the art (U.S. patents 3845770 and 3916899). As shown in Fig. 1a, EOP comprises an API tablet core and a rate control membrane encompassing the tablet core. The EOP comprises at least one orifice drilling through the membrane so that LD/CD can be released into the oral cavity through the orifice. The tablet core comprises LD/CD, osmotic agent, microcrystalline cellulose (MCC), binding agent, lubricant, flavoring agent (optional), acidifying agent (optional) and antioxidant (optional). The rate control membrane comprises a complete or at least a part of a semipermeable polymer that can penetrate water or moisture present in the oral cavity, while substantially impermeable to drugs and other optional components that can be present in the core. The representative semipermeable polymer is cellulose acetate with 32.0-39.8% acetyl content. Flow enhancers can be incorporated into rate control membranes. The flow enhancers in the invention includes but are not limited to polyethylene glycol, povidone, copovidone and other water-soluble polymers. The preferred flow enhancers are soluble in water and organic solvents such as acetone, methanol, ethanol and isopropanol copovidone (VA64). Typical solvent systems for high-throughput membrane compositions used in the prior art include acetone and water, while acetone is used to dissolve cellulose acetate and water is used to dissolve the enhancer. Their evaporation rates are significantly different, especially at low temperatures, which often lead to unknown incompatibility, i.e. potential precipitation of cellulose acetate during the coating process. Therefore, the solvent in the prior art often produces a high-throughput membrane with mechanical defects, thus weakening the mechanical strength of the membrane. A more homogenous membrane can be easily formed by using copovidone dissolved in acetone or acetone/ethanol mixed solvent, thus having a more consistent release profile compare with other membranes.

In another embodiment, LD/CD is incorporated into a bi-layer (push-pull) osmotic delivery system known in the prior art (U.S. patents 4327725 and 4612008). As shown in Fig. 1b, the push-pull system is composed of two layers of core (including drug-containing pull layer and osmotic push layer) and a rate control membrane encompassing the core. The push-pull system comprises at least one orifice through the membrane on the side containing the drug-containing layer, so that the contents of the pull layer can be released into the oral cavity through the orifice. The pull layer comprises LD/CD, a hydrophilic polymer, an osmotic agent, a binding agent, a lubricant, a flavoring agent (optional), an acidifying agent (optional), and an antioxidant (optional). The push layer comprises a high molecular weight hydrophilic polymer, an osmotic agent, a binding agent, a lubricant, and a colorant (optional). The push-pull osmotic delivery system operates by absorbing water or moisture through a rate control membrane into a bi-layer core, wherein it hydrates the two layers, thereby expanding the osmotic push layer and push the hydrated, dispensable drug-containing layer formulation through orifice from the system.

Due to the presence of the hydrophilic polymer, the push layer composition can retain a large amount of water in the layer. The hydrophilic polymer can be κ-carrageenan, sodium carboxymethyl cellulose, polyethylene oxide with a molecular weight of 75,000 to 7,500,000.

The osmotic agent used for the purpose of the present invention is selected from magnesium sulfate, magnesium chloride, sodium chloride, lithium chloride, potassium sulfate, sodium sulfate, mannitol, urea, sorbitol, inositol, sucrose, glucose, etc. Mannitol and sorbitol are the preferred osmotic agents due to the release of drug-containing layer formulation in the oral cavity and thus attracting attention to their taste and cariogenicity.

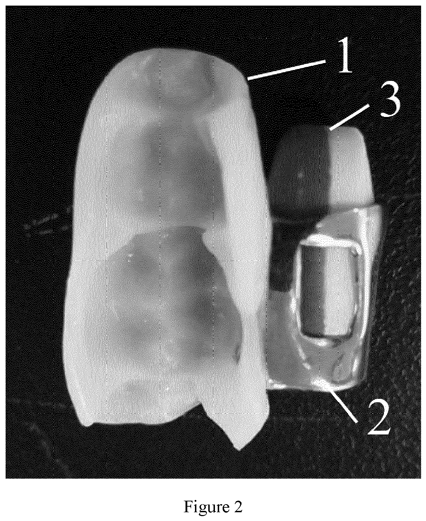

Figure 2 is a schematic diagram of an extend release dosage form (a combination of an extended release platform ERP and a retention enabling platform REP). The retention enabling platform comprises a personalized retention enabling module and a drug fastened module. The retention enabling module can be personalized to match the corresponding teeth. The drug fastened module serves as a reservoir to fasten the extended release platform ERP for maintaining the ERP in a right place. The retention enabling platform REP personalized retention enabling module can match the second molar and anterior and posterior teeth. Due to the length of the retention enabling platform REP, it (the REP that maintains the ERP) is in a non-inhalable position, thus eliminating the possibility of blocking. The preferred structure of the REP is a buckle-type or nut-type basket with various mesh sizes so that the ERP does not fall out. Other structures of the REP can be, but are not limited to, a holder with two clamping arms.

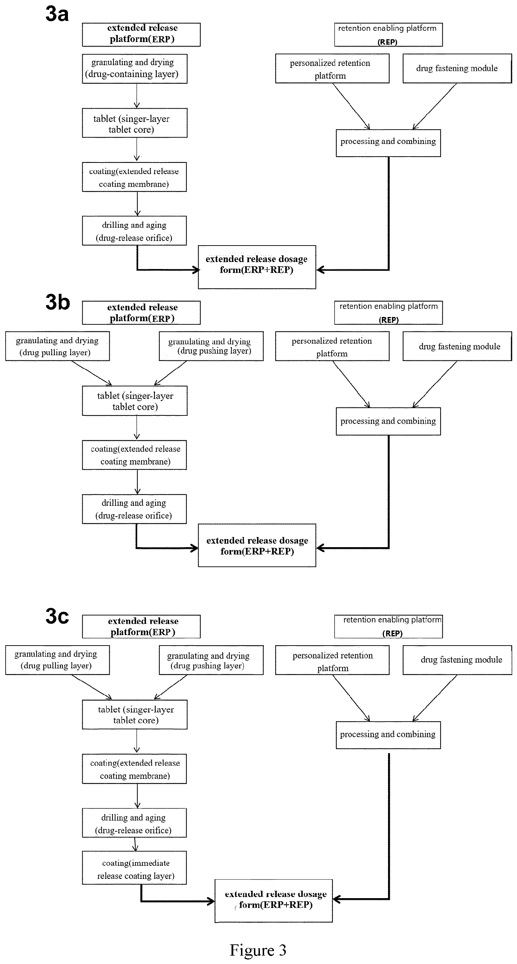

Fig. 3 is a flow chart of manufacturing the extended release dosage form (ERP + REP) of the present invention. Fig. 3a shows an extended release dosage form for manufacturing an extended release platform as a single-layer elementary osmotic pump (EOP); Fig. 3b shows an extended release dosage form for manufacturing an extended release platform as a bi-layer push-pull osmotic system; Fig. 3c shows an extended release dosage form for manufacturing an extended release platform as a bi-layer push-pull system with an immediate release drug overcoat.

The retention enabling module REP can be made of polymer materials or dental titanium using 3D printing technology, injection molding processes or impression molding. Polymer materials include, but are not limited to, polycaprolactone (PCL), ethylene-vinyl acetate copolymer (EVA), high density polyethylene (HDPE), polypropylene (PP), polyacrylate and any other tissue compatible polymers. PCL is a good material because of its low melting point and good biocompatibility. Therefore, the personalized retention enabling module of REP made by PCL is easily softened in hot water, and then matches the molar or premolar.

One of the ERP in the invention is a single-layer elementary osmotic pump, and can be manufactured by standard manufacturing techniques. First of all, in the conventional wet granulation method, a high-shear granulator or a fluidized bed granulator can be used to prepare tablet core particle. In the second step, the granules are pressed into a single-layer tablet core in the press. Next, the tablet core is coated with a semi-permeable membrane coating composition. Finally, an orifice was drilled through the coating membrane.

Another ERP in the present invention is a bi-layer push-pull osmotic pump, and can be manufactured as follows. First of all, a high-shear granulator or fluidized bed granulator can be used to prepare granules of drug-containing layer and osmotic push layer. Secondly, the granules of these two layers are pressed into a bi-layer tablet core in a press. Next, the bi-layer tablet core is coated with a semi-permeable membrane coating composition, followed by a drying process. Finally, an orifice was drilled through the membrane of drug-containing layer.

Semi-permeable membranes of osmotic dosage forms can be formed by air suspension technology. The method comprises suspending and tumbling a single-layer tablet core or a bi-layer tablet core in an air flow and coating composition until the membrane is homogenously formed around the core. The air suspension process can be realized by the fluidized bed granulator with a Wurster plug-in. Acetone or acetone ethanol mixed solubilizer can be used as coating solvent, in which 2.0-5 wt% of the membrane-forming composition is dissolved. Other membrane forming techniques, such as pan coating, can also be used. In the pan coating system, the membrane is continuously sprayed onto the tablet core of the rotary pan and deposited into a membrane composition. Generally, the membrane formed by these techniques with a thickness of 25-250 µm, preferably 100-150 µm.

Optionally, a single-layer EOP or bi-layer push-pull system with drilled orifice can be coated with an immediate release LD/CD drug-containing layer.

The personalized retention enabling platform (REP) in the present invention can be manufactured as follows. REP was prepared by 3D printing technology. First, an oral image of an individual was obtained by an oral scanner. Next, the CAD/CAM software was used to design the REP, which was the holder of the ERP. Then, according to CAD/CAM design, 3D printing technology or injection molding process was used to make REP from histocompatible polymer or dental titanium, or cobalt chromium alloy, or cobalt chromium molybdenum alloy.

The personalized retention enabling platform (REP) in the present invention can also be manufactured by injection molding technology. First, the dental impression material was used to make a mold to prepare the same plaster tooth model as the patient; secondly, REP shape of dental wax was prepared on plaster dental model by conventional process; then, the embedded powder and water mixture were used to wrap the dental wax REP, and the embedded dental wax was heated to melt after standing and curing to obtain a REP-shaped injection mold cavity; finally, at a certain temperature, the completely molten cobalt-chromium alloy was poured into the mold cavity, cooled and cured, and then polished.

The personalized retention enabling platform (REP) in the present invention can also be manufactured by impression molding technology. Firstly, a drug fastened module was manufactured, and then, an oval thermoplastic sheet was processed by a conventional process. Then, the thermoplastic sheet was heated to soften it, and the softened thermoplastic sheet was pressed to completely cover the teeth to form a personalized retention enabling module that is completely fitted to the teeth. The drug fastened module was then quickly embedded on the uncured retention enabling module and cooled and solidify to form a personalized retention enabling platform (REP). Wait for the personalized retention enabling platform to cool and restore the original opaque hard membrane state, and remove the cooled personalized retention enabling platform from the oral cavity.

### Embodiment 1 Preparation of retention enabling platform (impression molding)

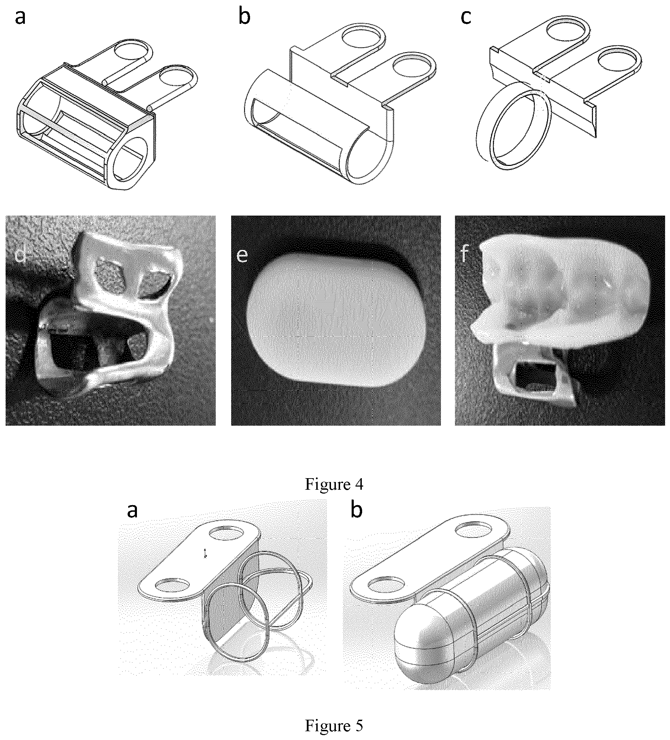

1. Drug fastened modules suitable as shown in a, b, c, d of Fig. 4, which is suitable for the extended release platform, were prepared. The drug fastened modules is made of stainless steel, dental titanium, cobalt chromium alloy, or cobalt chromium molybdenum alloy, and the shape of which can be one or more reservoirs with one or both ends open. The shape of the cross section of the reservoir can be polygon, circular closed-loop or open-loop with the opening smaller than the minimum diameter of the tablet.
2. 50g polycaprolactone was processed into an oval thermoplastic sheet with a size of 2.5cm×1.5cm by conventional process (as shown in Fig. 4e);
3. Preparation of personalized REP: a piece of thermoplastic sheet was heated in hot water at about 70 °C to soften it with good moldability. When the thermoplastic sheet becomes translucent (about 1 minute), it was taken out and placed on the teeth, and the softened thermoplastic sheet was pressed to completely wrap the teeth to form a retention enabling module that completely matches the teeth. Then, the stainless-steel drug fastened module was quickly embedded on the uncured retention enabling module, and cooled and cured to form a personalized retention enabling platform (REP). Some water can be briefly sprayed to further accelerate the cooling, then wait for the personalized retention enabling platform to cool and restore the original opaque hard sheet state, and the cooled personalized retention enabling platform was taken out the from the oral cavity (as shown in Fig. 4f). The following Fig. 5 can be used to explain its association and difference with Fig. 4.

Fig. 5 is the aforementioned drug fastened module: a. The drug fastened module; b. The extended release platform was fastened in the drug fastened module.

### Embodiment 2 Preparation of retention enabling platform (injection molding)

According to the following procedures, a retention enabling platform that fitted to the mandibular molar teeth was prepared by an injection molding process, as shown in Fig. 6.

First, a plaster dental model with the same dental properties as a patient was prepared by using a dental impression material to form a mold;

REP shape of dental wax was prepared on a plaster dental model by conventional process using dental wax;

The above dental wax REP was then wrapped with a mixture of embedding powder and water, and after standing and curing, the embedded dental wax was melted to obtain an injection mold cavity in the shape of REP;

At a certain temperature, the completely melted cobalt chromium alloy was poured into the mold cavity, cooled and cured, and then polished.

### Embodiment 3 Preparation of retention enabling platform (3D printing)

According to the following procedures, a retention enabling platform that fitted to the mandibular molar teeth was prepared by oral scanning and 3D printing processes, as shown in Fig. 6.

Firstly, an individual oral image was obtained by the oral scanner;

CAD/CAM software or dental software was used to process scanning data, generate electronic tooth model, transform the date into editable file, and design a personalized retention enabling platform device that completely fitted to the teeth;

Cobalt chromium molybdenum 3D printing material was used to print the designed personalized device by 3D printer laser sintering technology.

### Embodiment 4 Preparation of retention enabling platform (injection molding)

According to the following procedures, the retention enabling platform that fitted to the periphery of the non-occlusal surface of the mandibular molar was prepared by injection molding process, as shown in Fig. 7.

First, a plaster teeth model with the same dental properties as a patient is prepared by using a dental impression material to form a mold;

REP shape of dental wax was prepared on a plaster dental model by conventional process using dental wax;

The above dental wax REP was then wrapped with a mixture of embedding powder and water, and after standing and curing, the embedded dental wax was melted to obtain an injection mold cavity in the shape of REP;

At a certain temperature, the completely melted cobalt-chromium alloy is poured into the mold cavity, cooled and cured, and then polished.

### Embodiment 5 Preparation of retention enabling platform (3D printing)

According to the following procedures, a retention enabling platform that fitted to the periphery of the non-occlusal surface of the mandibular molar was prepared by oral scanning and 3D printing processes, as shown in Fig. 7.

Firstly, an individual oral image was obtained by the oral scanner;

CAD/CAM software or dental software was used to process scanning data, generate electronic tooth model, transform the data into editable file, and design a personalized retention enabling platform device that completely fits to the teeth;

Cobalt chromium molybdenum 3D printing material was used to print the designed personalized device by 3D printer laser sintering technology.

### Embodiment 6 Preparation of extended release platform

A dosage form for distributing the beneficial drugs levodopa and carbidopa to oral cavity was manufactured as follows: first, a tablet core was prepared, comprising, in weight percentage, 40.0Wt% of levodopa (LD), 10.8Wt% of carbidopa monohydrate (CD), 20.0Wt% of microcrystalline cellulose, 18.7Wt% of mannitol, 5.0Wt% of hydroxypropyl methylcellulose (HPMC E5) and 5.0Wt% of citric acid that were each passed through a 40-mesh stainless steel sieve, then blended and granulated with pure water until homogeneous wet mass was formed; the wet mass was passed through a 20-mesh stainless steel sieve and dried at 80°C for 2 hours; the dried granules were passed through an 18-mesh stainless steel sieve and then mixed with 0.5Wt% of magnesium stearate.

Then, 500 mg of the drug core granules were compressed into a single-layer tablet core with a 9.0 mm round punch using a tablet press.

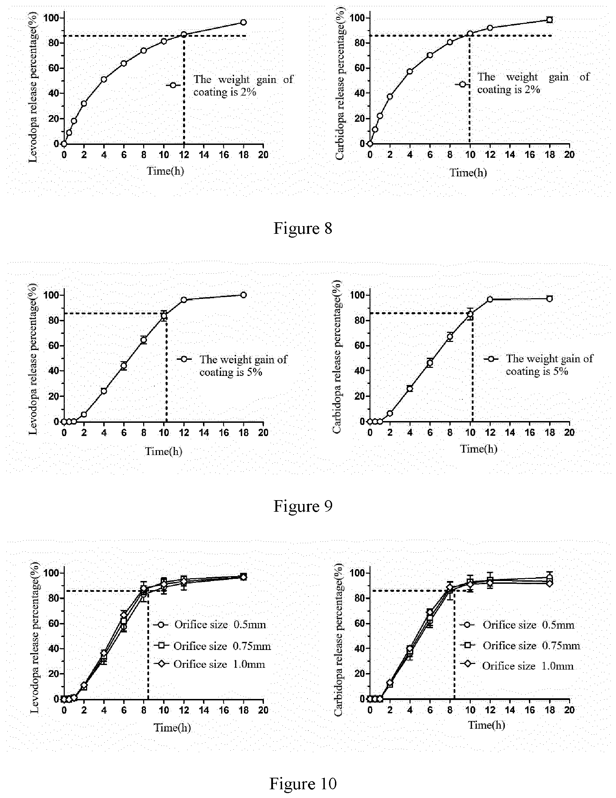

Next, the single-layer tablet core was coated with a semi-permeable membrane. The membrane-forming composition comprised, in weight percentage, 50Wt% of cellulose acetate and 50Wt% of Copovidone VA64. The membrane-forming composition was mixed with acetone to make a 4% of solid suspension. Using the process parameters listed in the table below, the membrane-forming composition was sprayed onto the tablet cores in a Glatt GC 1 pan coater to form a coating membrane. The membrane weight gain of the coated tablet was 2.0%. Finally, a 0.5 mm exit orifice was drilled mechanically on the drug layer side of the coated tablet. Residual solvents were removed by drying the dosage form at 40°C and ambient humidity for 24 hours. The release profile of the final manufactured dosage form was measured in 0.1N HCl aqueous solution using USP I pulp board. As shown in Figure 8, the final manufactured ERP dosage form delivered LD and CD at an average rate of 14.17 mg/hr and 4.59 mg/hr, with 85% of the drugs delivered in 12 and 10 hours, respectively. The ERP osmotic delivery system can be kept in oral cavity until the push-layer reaches the delivery orifice, or kept there for 8-9 hours, and then swallowed; or it can be fasten on the corresponding teeth in the oral cavity after being combined with the retention enabling platform REP and kept there for 12 hours .

| **parameters of coating process** | |
|---|---|
| **Inlet temperature (°C)** | 20-40 |
| **Exhaust temperature (°C)** | 14-25 |
| **Air flow rate (m³/h)** | 20-40 |
| **Fluid delivery rate (g/min)** | 5-25 |
| **Atomizing air pressure (bar)** | 0.6-0.8 |
| **Pattern air pressure (bar)** | 0.6-0.8 |
| **Rotating speed (rpm)** | 6-15 |
| **Batch size (g)** | 400 |

### Embodiment 7 Preparation of extended release platform

A dosage form designed, shaped and adapted for dispensing the beneficial drugs levodopa and carbidopa monohydrate to oral cavity was manufactured as follows: first, a drug layer composition was prepared, comprising, in weight percentage, 40.0Wt% of LD, 10.8Wt% of CD, 31.0Wt% of hydroxypropyl cellulose having an average molecular weight of 80,000, 12.7Wt% of mannitol and 5.0Wt% of citric acid, these excipients were each pass through a 40-mesh stainless steel sieve, then blended and granulated with 95% ethanol until homogeneous wet mass was formed; the wet mass was passed through a 20-mesh stainless steel sieve and dried at 80 °C for 2 hours; the dried granules were passed through an 18-mesh stainless steel sieve and then mixed with 0.5Wt% of magnesium stearate.

Next, a second composition, the osmotic layer, was prepared, comprising 55.0Wt% of sodium carboxymethyl cellulose 7H4XF, 39.0Wt% of sorbitol, 5.0Wt% of Povidone K30 and 0.5Wt% of iron oxide red; these components were each passed through a 40-mesh stainless steel sieve, then blended and granulated with 95% ethanol until homogeneous wet mass was formed; the wet mass was passed through a 20-mesh stainless steel sieve and dried at 80°C for 2 hours; dried granules were passed through a 18-mesh stainless steel sieve and then mixed with 0.5 wt% of magnesium stearate.

Next, the drug-containing layer and the osmotic layer granules were pressed into a bi-layer tablet core. First, 500 mg of drug layer granules were added to a 9 mm round punch of a tablet press and tamped, then 250 mg of osmotic layer granules were added to the punch, and the granules of both layers were pressed with a tablet press into a contacting bi-layer tablet core.

Next, the bi-layer tablet core was coated with a semi-permeable membrane. The membrane-forming composition comprises, in weight percentage, 70 wt% of cellulose acetate with 39.8 wt% of acetyl content and 30 wt% of copovidone VA64. The membrane-forming composition was mixed with acetone to form a 4% of solid suspension. Using the process parameters listed in embodiment 6, the membrane-forming composition was sprayed onto the bi-layer tablet cores in a Glatt GC 1 pan coater to form a coating membrane, and the membrane weight gain of the coated tablet was 5.0%. Finally, a 0.5 mm exit orifice was drilled mechanically on the drug layer side of the dosage form. Residual solvents were removed by drying the dosage form at 40°C and ambient humidity for 24 hours. The release profile of the final manufactured dosage form was measured using a USP I paddle method in an aqueous solution of 0.1 N HCl. The final manufactured dosage form delivered LD and CD at an average rate of 17.0 mg/hr and 4.6 mg/hr, respectively, with 85% of LD/CD delivered in 10.0 hours. Figure 9 depicts the consistent release profiles for both LD and CD. The osmotic delivery system can be kept in oral cavity until the osmotic layer reached the delivery orifice, or kept there for 6-7 hours, and then was swallowed, or it was fastened on the corresponding teeth in the oral cavity and maintained there for 10 hours after combination with the retention enabling platform REP.

### Embodiment 8 Preparation of extended release platform

In this embodiment, the procedures of Embodiment 7 were repeated, and the dosage form consisted of the drug layer, osmotic layer, and coating membrane was identical to those provided in Embodiment 6. In this example, the membrane weight gain was 4.0%, and the size of the delivery orifice varied from 0.5 mm, 0.75 mm, to 1.0 mm. The final manufactured dosage form delivered LD and CD at an average rate of 21.3 mg/hr and 5.7 mg/hr, respectively, with 85% of LD/CD delivered in 8.0 hours. As shown in Figure 10, the size of the delivery orifice has no significant impact on the release profile. The osmotic delivery system can be kept in oral cavity until the osmotic layer reached the delivery orifice, or kept there for 4-5 hours, and then was swallowed, or it was fastened on the corresponding teeth in the oral cavity and maintained there for 8 hours after combination with the retention enabling platform REP.

### Embodiment 9 Preparation of extended release platform

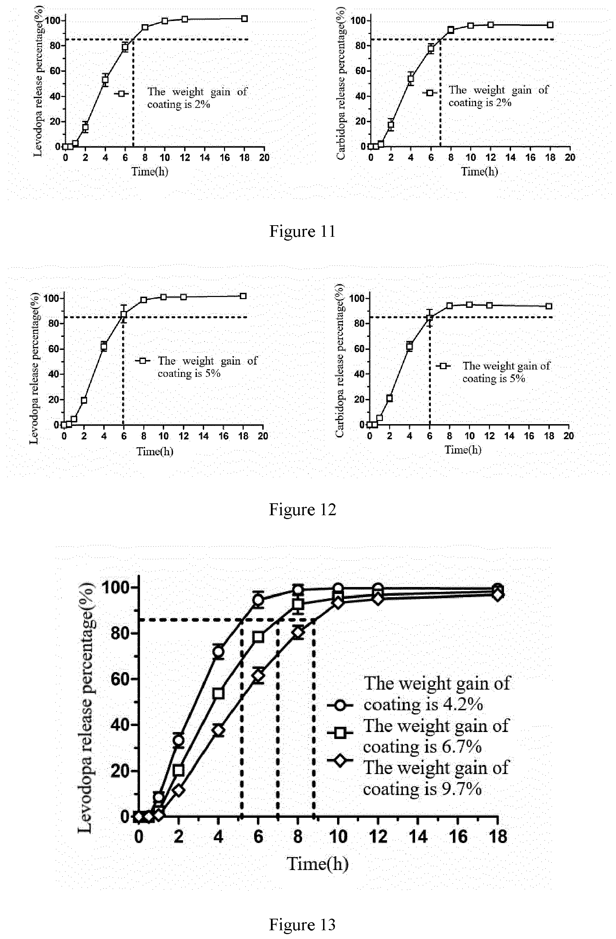

In this embodiment, the procedures of Embodiment 7 were repeated, and the dosage form consisted of the drug layer was identical to those provided in example 2, while the osmosis layer comprised sodium carboxymethyl cellulose 9H4XF instead of 7H4XF. In this Embodiment, the membrane-forming composition and the size of the delivery orifice were also identical to those in Embodiment 6. The coating membrane weight gain of the dosage form was 2.0%. As shown in Figure 11, the dosage form delivered LD and CD at an average rate of 24.3 mg/hr and 6.6 mg/hr, respectively, with 85% of LD/CD delivered in 7.0 hours. The osmotic delivery system can be kept in oral cavity until the osmotic layer reached the delivery orifice, or kept there for 3-4 hours, and then was swallowed, or it was fastened on the corresponding teeth in the oral cavity and maintained there for 7 hours after combination with the retention enabling platform REP.

### Embodiment 10 Preparation of extended release platform

In this embodiment, the procedures of Embodiment 7 were repeated, and the dosage form consisted of the drug layer and osmotic layer was identical to those provided in Embodiment 7, while the membrane-forming composition comprised 60wt% of cellulose acetate of cellulose acetate with an acetyl content of 39.8% and 40Wt% of copovidone VA64. The membrane weight gain was 5.0%. As shown in Figure 12, the dosage form delivered 85% of LD/CD in 6 hours. The osmotic delivery system can be kept in oral cavity until the osmotic layer reached the delivery orifice, or kept there for 2-3 hours, and then was swallowed, or it was fastened on the corresponding teeth in the oral cavity and maintained there for 6 hours after combination with the retention enabling platform REP.

### Embodiment 11 Preparation of extended release platform

In this embodiment, the procedures of embodiment 7 were repeated to provide a dosage form.

In this embodiment, the drug layer comprised 45.0Wt% of LD, 31.0Wt% of hydroxypropyl cellulose (Klucel EXF), 16.0Wt% of mannitol, 5.0Wt% of Povidone K30, 1.0Wt% of aspartame, 1.0Wt% of Mint flavor and 1.0Wt% of magnesium stearate. The osmosis layer comprised 55Wt% of sodium carboxymethyl cellulose 7H4XF, 34.0Wt% of sorbitol, 10.0Wt% of Povidone K30 and 0.5Wt% of iron oxide red and 0.5Wt% of magnesium stearate.

The drug layer (500mg) and osmotic layer granules (250mg) were compressed into a bi-layer tablet core using a 16×7 capsule-shape tooling.

The bi-layer tablet core was wrapped with the semi-permeable membrane, and the weight gains were 4.2Wt%, 6.7% and 9.7%, respectively. The membrane-forming composition comprised 60wt% of cellulose acetate having an acetyl content of 39.8%, 40wt% of Copovidone VA64. A 1.0 mm exit orifice was drilled mechanically on the drug layer side of the dosage form. Residual solvents were removed by drying the dosage form at 40°C and ambient humidity for 24 hours.

As shown in Figure 13, the dosage form delivered LD at an average rate of 38.3 mg/hr, 27.3 mg/hr, and 21.3 mg/hr, and the membrane weight gains were 4.2%, 6.7%, and 9.7%, respectively, with 85% of LD delivered in 5.0 hours, 7.0 hours and 9.0 hours.

### Embodiment 12 Preparation of extended release platform

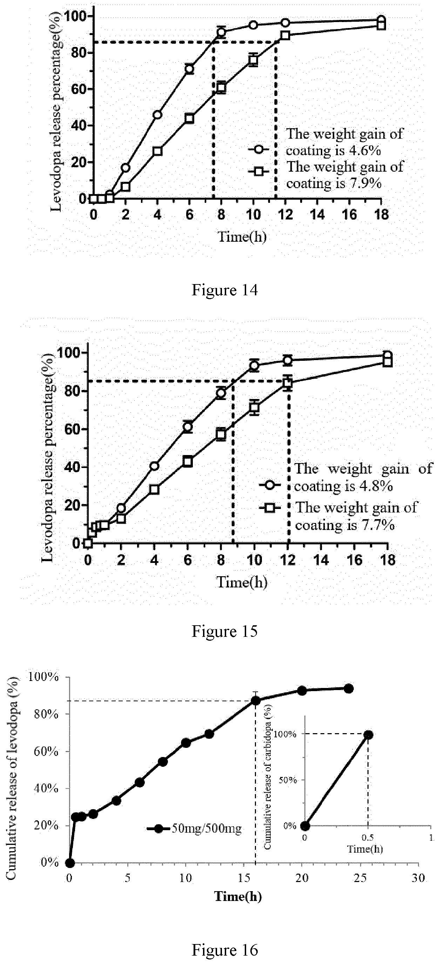

In this embodiment, the procedures of embodiment 11 were repeated to provide a dosage form other than a membrane-forming composition. In this embodiment, the bi-layer tablet core comprises a membrane-forming composition comprising 70 wt% of cellulose acetate with 39.8% acetyl content and 30 wt% of copovidone VA 64 in weight percentage. The membrane-forming composition was dissolved in a mixed solvent comprising 90% acetone, 9.0% ethanol and 1.0% deionized water to produce a 4% of solid suspension. As shown in Fig. 14, the dosage forms with membrane weight gain of 4.6% and 7.9% delivered LD at an average rate of 25.5 mg/hr and 16.9 mg/hr, respectively, and correspondingly with 85% of LD delivered at 7.5 hours and 11.5 hours.

### Embodiment 13 Preparation of extended release platform

In this embodiment, the procedures of embodiment 12 were repeated to provide a dosage form. In this embodiment, an immediate release composition comprising 23.78 wt% of LD, 64.22 wt% of CD, 10 wt% of hydroxypropyl cellulose, 1 wt% of aspartame and 1 wt% of Mint Flavor was used to overcoat the dried dosage form with 4.8% and 7.7% membrane weight gain (as shown in c of Fig. 3c). The immediate release overcoat composition was mixed with ethanol to form a 6.7% solid suspension. The final dosage form comprised an immediate-release coating comprising 62.5 mg of CD and 25 mg of LD, and a controlled-release drug layer comprising 225 mg of LD. As shown in Fig. 15, the release profile of the dosage form showed rapid release of LD/CD, followed by an extended release with a release duration of approximately 8.5 hours and 12.0 hours, respectively. The osmotic delivery system having a membrane weight gain of 4.8% can be kept in oral cavity for 4-5 hours, and then kept in oral cavity at meal time or for the whole release duration. The osmotic delivery system having a membrane weight gain of 7.7% can be kept in oral cavity for 8-9 hours before swallowed, or kept in oral cavity for the whole release duration, or fastened on the corresponding teeth in the oral cavity after being combined with the retention enabling platform REP and maintained there for 12 hours.

### Embodiment 14 Preparation of extended release platform

In this embodiment, the procedures of Embodiment 6 were repeated, and the dosage form comprised a drug layer and a membrane-forming composition were identical to those provided in Embodiment 6. The drug layer comprised, in weight percentage, 38.0Wt% of levodopa, 50.0Wt% of microcrystalline cellulose, 2.0Wt% of magnesium stearate and 10.0Wt% of hydroxypropyl methylcellulose. The coating membrane comprised 50Wt% of acetyl acetate membrane with an acetyl content of 39.8% and 50Wt% of Copovidone VA64. In this example, the membrane weight gain was 4.50%. The final manufactured dosage form delivered levodopa at an average rate of 9.4 mg/hr, with 85% of levodopa delivered in 9.0 hours.

### Embodiment 15 Preparation of extended release platform

In this embodiment, the procedures of Embodiment 6 were repeated, and the dosage form comprised a drug layer and a membrane-forming composition were identical to those provided in Embodiment 6. The drug containing layer comprised, in weight percentage, 19.5Wt% of levodopa, 20.0Wt% of carbidopa, 50.0Wt% of mannitol and 10.0Wt% of citric acid. The coating membrane comprised 50Wt% of acetyl acetate membrane with an acetyl content of 39.8% and 50Wt% of Copovidone VA64. In this example, the membrane weight gain was 4.50%. The final manufactured dosage form delivered levodopa at an average rate of 22.9 mg/hr, with 85% of levodopa delivered in 13.0 hours.

### Embodiment 16 Preparation of extended release platform

In this embodiment, the procedures of Embodiment 7 were repeated, and the dosage form comprised a drug layer, an osmosis layer and a membrane-forming composition were identical to those provided in Embodiment 7. The drug layer comprised, in weight percentage, 70.0Wt% of levodopa, 9.0Wt% of mannitol, 20.0% of Povidone K30 and 1.0Wt% of magnesium stearate. The osmosis layer comprised, in weight percentage, 85.0Wt% of sodium carboxymethyl cellulose (7H4XF), 3.0Wt% of Povidone K30, 5.0Wt% of sorbitol, 5.0Wt% of iron oxide red and 2.0Wt% of magnesium stearate. The coating membrane comprised, in weight percentage, 70Wt% of acetyl acetate membrane with an acetyl content of 39.8% and 30Wt% of Copovidone VA64. In this example, the membrane weight gain was 4.5%. The final manufactured dosage form delivered levodopa at an average rate of 35.0 mg/hr, with 85% of levodopa delivered in 8.5 hours.

### Embodiment 17 Preparation of extended release platform

In this embodiment, the procedures of Embodiment 7 were repeated, and the dosage form comprised a drug layer, an osmosis layer and a membrane-forming composition were identical to those provided in Embodiment 2. The drug layer comprised, in weight percentage, 20.0Wt% of levodopa, 20.0Wt% of carbidopa, 50.0Wt% of hydroxypropyl cellulose, 4.0Wt% of mannitol, 5.0Wt% of aspartame and 1.0Wt% of magnesium stearate. The osmosis layer comprised, in weight percentage, 25.0Wt% of sodium carboxymethyl cellulose (7H4XF), 9.5Wt% of Povidone K30, 65.0Wt% of sorbitol and 0.5Wt% of magnesium stearate. The coating membrane comprised, in weight percentage, 90Wt% of acetyl acetate membrane with an acetyl content of 39.8% and 10Wt% of Copovidone VA64. In this example, the membrane weight gain was 4.5%. The final manufactured dosage form delivered levodopa and CD at an average rate of 7.1 mg/hr, with 85% of levodopa/CD delivered in 12 hours.

### Embodiment 18 Preparation of extended release platform

In this embodiment, the procedures of Embodiment 13 were repeated, and the dosage form comprised a drug layer, an osmosis layer, a membrane-forming composition and an overcoat were identical to those provided in Embodiment 13. The drug layer comprised, in weight percentage, 45.0Wt% of levodopa, 31.0Wt% of hydroxypropyl cellulose, 17.0Wt% of mannitol, 5.0Wt% of Povidone K30, 1.0Wt% of magnesium stearate and 1.0Wt% of aspartame. The osmosis layer comprised, in weight percentage, 60.0Wt% of sodium carboxymethyl cellulose (7H4XF), 10.0Wt% of Povidone K30, 26.0Wt% of sorbitol, 2.0Wt% of iron oxide red and 2.0Wt% of magnesium stearate. The coating membrane comprised, in weight percentage, 70Wt% of acetyl acetate membrane with an acetyl content of 39.8% and 30Wt% of Copovidone VA64. The weight of the coating membrane was 4.5% of the mass of the tablet core. The immediate-release overcoat comprised, in weight percentage, 93.0Wt% of CD, 2.0Wt% of hydroxypropyl cellulose EF and 5.0Wt% of aspartame; the mass of the overcoat was 13.2% of the mass of the tablet core (table core + first layer coating membrane comprising cellulose acetate and Copovidone VA64). The immediate-release overcoat of the dosage form was first released rapidly, followed by an extended release with a release duration of approximately 8 hours. The osmotic delivery system can be kept in oral cavity for 4-5 hours and then swallowed before meal time or kept in oral cavity for the whole release duration, or fastened on the corresponding teeth in the oral cavity after being combined with the retention enabling platform REP and maintained there for 8 hours.

### Embodiment 19 Preparation of extended release platform

In this embodiment, the procedures of embodiment 13 were repeated, and the dosage form comprised a drug layer, an osmosis layer, a membrane-forming composition and an overcoat were identical to those provided in Embodiment 13. The drug layer comprised, in weight percentage, 45.0Wt% of levodopa, 31.0Wt% of hydroxypropyl cellulose, 12.0Wt% of mannitol, 5.0Wt% of Povidone K30, 5.0Wt% of Mint flavor, 1.0Wt% of magnesium stearate and 1.0Wt% of aspartame. The osmosis layer comprised, in weight percentage, 40.0Wt% of sodium carboxymethyl cellulose (7H4XF), 20.0Wt% of Povidone K30, 36.0Wt% of sorbitol, 3.5Wt% of iron oxide red and 0.5Wt% of magnesium stearate. The coating membrane comprised, in weight percentage, 70Wt% of acetyl acetate membrane with an acetyl content of 39.8% and 30Wt% of copovidone VA64. The weight of the coating membrane was 4.5% of the mass of the tablet core. The immediate-release overcoat comprised, in weight percentage, 75.0Wt% of LD, 20.0Wt% of hydroxypropyl cellulose and 5.0Wt% Mint flavor; the mass of the overcoat was 13.2% of the mass of the tablet core (table core + first layer coating membrane comprising cellulose acetate and Copovidone VA64). The immediate-release overcoat of the dosage form was first released rapidly, followed by an extended release with a release duration of approximately 8 hours. The osmotic delivery system can be kept in oral cavity for 4-5 hours and then be swallowed before meal time or kept in oral cavity for the whole release duration, or fastened on the corresponding teeth in the oral cavity after being combined with the retention enabling platform REP and maintained there for 8 hours.

### Embodiment 20 Preparation of extended release platform

In this embodiment, the procedures of embodiment 8 were repeated, and the dosage form comprised a drug layer, an osmosis layer, a membrane-forming composition and an overcoat were identical to those provided in example 8. The drug layer comprised, in weight percentage, 45.0Wt% of levodopa, 31.0Wt% of hydroxypropyl cellulose, 17.0Wt% of mannitol, 5.0Wt% of Povidone K30, 1.0Wt% magnesium stearate and 1.0Wt% of aspartame. The osmosis layer comprised, in weight percentage, 55.0Wt% of sodium carboxymethyl cellulose (7H4XF), 10.0Wt% of hydroxypropyl cellulose, 34.0Wt% sorbitol, 0.5Wt% of iron oxide red and 0.5Wt% of magnesium stearate. The coating membrane comprised, in weight percentage, 70Wt% of acetyl acetate membrane with an acetyl content of 39.8% and 30Wt% of copovidone VA64. The weight of the coating membrane was 5.9% of the mass of the tablet core. The solid content of the immediate-release overcoat suspension was 10.0Wt%, comprising, in weight percentage, 24.0Wt% of levodopa, 65.0Wt% of carbidopa monohydrate, 10.0Wt% of hydroxypropyl cellulose and 1.0Wt% of aspartame; the weight of the overcoat was 13.2% of the weight of the tablet core (table core + first layer coating membrane comprising cellulose acetate and Copovidone VA64). When the solvent for the overcoat suspension was anhydrous ethanol, the level of the carbidopa-related genotoxic impurity hydrazine in the obtained dosage form was 1.7 ppm, and the level of the carbidopa-related impurity dihydroxyphenylacetone (DHPA) was 0.21%. When the solvent for the coating suspension of the overcoat was purified water, the solid content of the overcoat was 10.0Wt%, comprising, in weight percentage, 24.0Wt% of levodopa, 65.0Wt% of carbidopa monohydrate, 10.0Wt% of hydroxypropyl cellulose and 1.0Wt% of aspartame; the level of the carbidopa-related genotoxic impurity hydrazine in the obtained dosage form was 3.8 ppm, and the level of the carbidopa-related impurity DHPA was 0.28%. The carbidopa-related genotoxic impurity hydrazine and impurity DHPA of the obtained dosage form when the solvent for the coating suspension of the overcoat was anhydrous ethanol, were significantly lower than those of the obtained dosage form when the solvent for the coating suspension was purified water. The immediate-release overcoat of the dosage form was first released rapidly, followed by a sustained release with a duration of approximately 8 hours. The dosage form can be held in the oral cavity for 3-5 hours, and then be swallowed before meal time or kept in oral cavity for the whole release duration.

### Embodiment 21 Preparation of extended release platform (Each tablet contains 62.5mgCD + 500mgLD)

Dosage forms that were designed, shaped and suitable for dispensing the beneficial drugs levodopa and carbidopa monohydrate to the oral cavity were prepared as follows: First, a drug-containing layer composition was prepared, which comprises, in weight percentage, 67.9 wt% of LD, 25.1 wt% of hydroxypropyl cellulose with an average molecular weight of 80,000, 5.0 wt% of povidone K30, and 1.0 wt% of aspartame. The excipients were sieved by a 40 mesh stainless steel sieve respectively, then mixed with pure water and granulated until a homogenous wet substance was formed; the wet substance was sieved by a 4×4mm sieve and dried at 60 °C for 1 hour; the dried granules were sieved by a Φ1.5mm sieve, and then mixed with 1.0 wt% of magnesium stearate.

Next, a second composition, an osmotic layer, which comprises 55.0 wt% of sodium carboxymethylcellulose 7H4XF, 39.0 wt% of sorbitol, 5.0 wt% of povidone K30 and 0.5 wt% of iron oxide red, was prepared; each of these components was sieved by 40 mesh stainless steel sieve, and then mixed with 95% ethanol and granulated until a homogenous wet substance was formed; the wet substance was sieved by a 4×4mm sieve and dried at 80 °C for 2 hours; the dried granules were sieved by a Φ1.2mm sieve, and then mixed with 0.5 wt% of magnesium stearate.

Next, the drug-containing layer and the osmotic layer granules were pressed into a bi-layer tablet core. First, 700 mg of the drug-containing layer granules were added to the 19×7.5mm special-shaped punch of the tablet press and compacted, then 350 mg of the osmotic layer granules were added to the punch, and the two layers of granules were pressed into a contact bi-layer tablet core by the tablet press.

Next, the bi-layer tablet core was coated with a semi-permeable membrane. The membrane-forming composition comprised, in weight percentage, 70 wt% of cellulose acetate with an acetyl content of 39.8 wt% and 30 wt% of copovidone VA64. The membrane-forming composition was mixed with acetone to form a 4% of solid suspension. Using the technological parameters listed in embodiment 6, the membrane-forming composition was sprayed onto the bi-layer tablet cores in a Glatt GC 1 pan coater to form a coating membrane. The membrane weight gain of the coated tablets was 5.0%. Finally, a 1.0 mm exit orifice was mechanically drilled on the side of drug-containing layer of the dosage form. Residual solvent was removed by drying the dosage forms at 40 °C and ambient humidity for 72 hours. Immediate release overcoat comprised, in weight percentage, 24.1 wt% of LD, 64.9 wt% of CD, 10.0 wt% of hydroxypropyl cellulose and 1.0 wt% of aspartame; the weight of the overcoat was 9.4% of the weight of the tablet core (table core + first coating membrane cellulose acetate and copovidone VA64). The immediate release overcoat of the dosage form was rapidly released first, then released for an extended release with a duration of approximately 16 hours. The osmotic delivery system can be maintained in the oral cavity for 16 hours, maintained in the oral cavity during the entire release duration, or fastened on the corresponding teeth in the oral cavity after being combined with the retention enabling platform REP and maintained there for 16 hours.

### Embodiment 22 Preparation of extended release platform (Each tablet comprises 62.5mgCD + 375mgLD)

In this embodiment, the procedures of embodiment 20 were repeated, and the dosage form comprising a drug-containing layer, an osmotic layer, a membrane-forming composition and an overcoat was identical to those provided in embodiment 20. The drug-containing layer comprised, in weight percentage, 53.5 wt% of LD, 39.5 wt% of hydroxypropyl cellulose with an average molecular weight of 80,000, 5.0 wt% of povidone K30, 1.0 wt% of aspartame and 1.0 wt% of magnesium stearate. The osmotic layer comprised, in weight percentage, 55.0 wt% of sodium carboxymethylcellulose 7H4XF, 39.0 wt% of sorbitol, 5.0 wt% of povidone K30, 0.5 wt% of iron oxide red and 0.5 wt% of magnesium stearate. The membrane-forming composition comprised, in weight percentage, 70 wt% of cellulose acetate with 39.8 wt% of acetyl content, and 30 wt% of copovidone VA64. The weight of the overcoat is 9.4% of the weight of the tablet core (table core + first coating membrane cellulose acetate and copovidone VA64). The immediate release overcoat of the dosage form was rapidly released first, then released for an extended release with a duration of approximately 16 hours. The osmotic delivery system can be maintained in the oral cavity for 16 hours, maintained in the oral cavity during the entire release duration, or fastened on the corresponding teeth in the oral cavity after being combined with the retention enabling platform REP and maintained there for 16 hours.

### Embodiment 23 Preparation of extended release platform (Each tablet contains 50mgCD + 500mgLD)

Dosage forms that were designed, shaped and suitable for dispensing the beneficial drugs levodopa and carbidopa monohydrate to the oral cavity were prepared as follows: First, a drug-containing layer composition was prepared, which comprises 62.5 wt% of LD, 31 wt% of hydroxypropyl cellulose with an average molecular weight of 80,000, 4.5 wt% of mannitol, 0.9 wt% of aspartame, 0.1% wt% of Mint Flavor and 0.5 wt% of magnesium stearate, these excipients were made by dry granulation, then sieved by a 1.2mm sieve, and then mixed with 0.5 wt% of magnesium stearate.

Next, a second composition, an osmotic layer, which comprises 55.0 wt% of sodium carboxymethylcellulose 7H4XF, 34.0 wt% of sorbitol, 10 wt% of hydroxypropyl cellulose and 0.5 wt% of iron oxide red, was prepared; these excipients were made by dry granulation, then sieved by a 1.2mm sieve, and then mixed with 0.5 wt% of magnesium stearate.

Next, the drug-containing layer and the osmotic layer granules were pressed into a bi-layer tablet core. First, 600 mg of the drug-containing layer granules were added to the 19×7.5mm special-shaped punch of the tablet press and compacted, then 300 mg of the osmotic layer granules were added to the punch, and the two layers of granules were pressed into a contact bi-layer tablet core by the tablet press.

Next, the bi-layer tablet core was coated with a semi-permeable membrane. The membrane-forming composition comprised, in weight percentage, 70 wt% of cellulose acetate with 39.8 wt% of acetyl content, and 30 wt% of copovidone VA64. The membrane-forming composition was mixed with acetone to form a 4% of solid suspension. Using the technological parameters listed in embodiment 6, the membrane-forming composition was sprayed onto the bi-layer tablet cores in a Glatt GC 1 pan coater to form a coating membrane. The membrane weight gain of the coated tablets was 6.5 %. Finally, a 1.0 mm exit orifice was mechanically drilled on the drug-containing layer side of the dosage form. The immediate release overcoat comprised, in weight percentage, 62.15 wt% of LD, 26.85 wt% of CD, 10.0 wt% of hydroxypropyl cellulose and 0.9 wt% of aspartame; the weight of the overcoat is 21.0 % of the weight of the tablet core (table core + first coating membrane cellulose acetate and copovidone VA64). As shown in Fig. 16, the release profile of the dosage form showed rapid release of LD/CD, followed by an extended release with a duration of approximately 16 hours. The osmotic delivery system can be maintained in the oral cavity for 16 hours, maintained in the oral cavity during the entire release duration, or fastened on the corresponding teeth in the oral cavity after being combined with the retention enabling platform REP and maintained there for 16 hours.

### Embodiment 24 Preparation of extended release platform (Each tablet contains 37.5mgCD + 375mgLD)

Dosage forms that were designed, shaped and suitable for dispensing the beneficial drugs levodopa and carbidopa monohydrate to the oral cavity were prepared as follows: First, a drug-containing layer composition was prepared, which comprised 46.9 wt% of LD, 31.0 wt% of hydroxypropyl cellulose with an average molecular weight of 80,000, 20.1 wt% of mannitol, 0.9 wt% of aspartame, 0.1% wt% of Mint Flavor and 0.5 wt% of magnesium stearate, these excipients were made by dry granulation, then sieved by a 1.2mm sieve, and then mixed with 0.5 wt% of magnesium stearate.

Next, a second composition, the osmotic layer, which comprised 55.0 wt% of sodium carboxymethylcellulose 7H4XF, 34.0 wt% of sorbitol, 10 wt% of hydroxypropyl cellulose and 0.5 wt% of iron oxide red, was prepared; these excipients were made by dry granulation, sieved by a 1.2mm sieve, and then mixed with 0.5 wt% of magnesium stearate.

Next, the drug-containing layer and the osmotic layer granules were pressed into a bi-layer tablet core. First, 600 mg of the drug-containing layer granules were added to the 19×7.5mm special-shaped punch of the tablet press and compacted, then 300 mg of the osmotic layer granules were added to the punch, and the two layers of granules were pressed into a contact bi-layer tablet core by the tablet press.

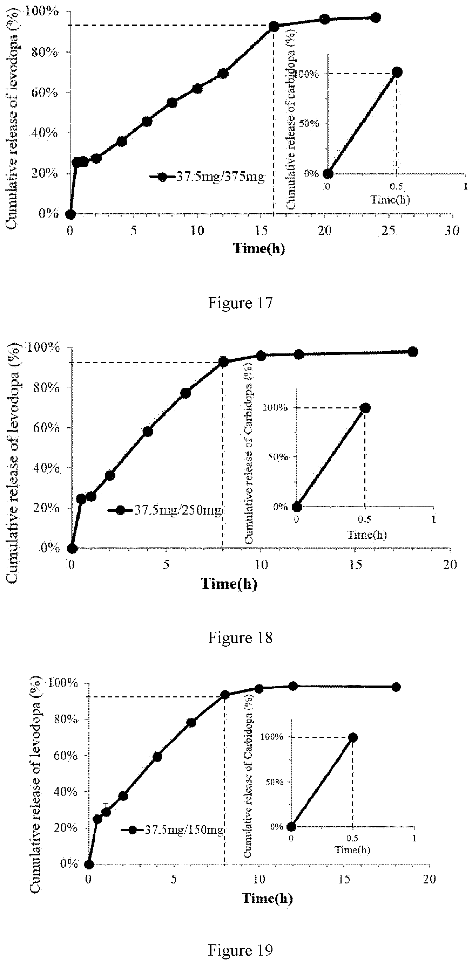

Next, the bi-layer tablet core was coated with a semi-permeable membrane. The membrane-forming composition comprised, in weight percentage, 70 wt% of cellulose acetate with 39.8 wt% of acetyl content, and 30 wt% of copovidone VA64. The membrane-forming composition was mixed with acetone to form a 4% of solid suspension. Using the technological parameters listed in embodiment 6, the membrane-forming composition was sprayed onto the bi-layer tablet cores in a Glatt GC 1 pan coater to form a coating membrane. The membrane weight gain of the coated tablets was 6.5 %. Finally, a 1.0 mm exit orifice was mechanically drilled on the drug-containing layer side of the dosage form. The immediate release overcoat comprised, in weight percentage 62.15 wt% of LD, 26.85 wt% of CD, 10.0 wt% of hydroxypropyl cellulose and 0.9 wt% of aspartame; the weight of the overcoat was 15.7 % of the weight of the tablet core (table core + first coating membrane cellulose acetate and copovidone VA64). As shown in Fig. 17, the release profile of the dosage form showed rapid release of LD/CD, followed by extended release with a duration of approximately 16 hours. The osmotic delivery system can be maintained in the oral cavity for 16 hours, maintained in the oral cavity during the entire release duration, or fastened on the corresponding teeth in the oral cavity after being combined with the retention enabling platform REP and maintained there for 16 hours.

### Embodiment 25 Preparation of extended release platform (Each tablet comprises 37.5mgCD + 250mgLD)

Firstly, a drug layer composition comprising 45.0Wt% of levodopa, 31.0Wt% of hydroxypropyl cellulose, 22.0Wt% of mannitol, 0.9Wt% of aspartame, 0.1Wt% of Mint flavor and 0.5Wt% of magnesium stearate was prepared, the components were each passed through a 40-mesh stainless steel sieve and granulated to obtain dry granules by a dry granulator, and then mixed with 0.5Wt% of magnesium stearate.

Next, a second composition, an osmosis layer, comprising 55.0Wt% of sodium carboxymethyl cellulose 7H4XF, 34.0Wt% of sorbitol, 10.0Wt% of hydroxypropyl cellulose and 0.5Wt% of iron oxide red was prepared; the components were respectively passed through a 40-mesh stainless steel sieve and then dried to obtain dry granules by a dry granulator, and then mixed with 0.5Wt% of magnesium stearate.

Next, the drug layer and osmotic layer granules were compressed into a bi-layer tablet core. First, 417 mg of drug layer granules were added to the 16×7 mm punch and tamped, and 208 mg of osmotic layer granules were added, then the two layers of granules were compressed into a contact bi-layer tablet core with a tablet press.

Next, the bi-layer tablet core was coated with a semi-permeable membrane. The membrane-forming composition comprised, in weight percentage, 70Wt% of cellulose acetate with an acetyl content of 39.8Wt%, and 30Wt% of Copovidone VA64. The membrane-forming composition was mixed with acetone to make a solution with 4% of solid suspension. Using the process parameters listed in Embodiment 6, the membrane-forming composition was sprayed onto the bi-layer tablet core in a Glatt GC 1 pan coater to form a coating membrane, and the membrane weight gain of the coated tablet was 6.5%. Finally, a 1.0 mm exit orifice was drilled mechanically on the drug layer side of the dosage form.

Next, an immediate-release composition, comprising 54.0Wt% of levodopa, 35.0Wt% of carbidopa monohydrate, 10.0Wt% of hydroxypropyl cellulose, 0.9Wt% of aspartame and 0.1Wt% of Mint flavor, was used to overcoat the dried dosage form, with the membrane weight gain of 6.5%. The immediate-release overcoat composition was mixed with anhydrous ethanol to make a 10.0Wt% of solid suspension. The final dosage form comprises an immediate-release overcoat comprising 62.5 mg of levodopa and 37.5 mg of carbidopa, and a controlled-release drug layer comprising 187.5 mg of levodopa.

As shown in Fig. 18, the release profile of the dosage form showed rapid release of LD/CD, followed by an extended release with a release duration of approximately 8 hours. The osmotic delivery system can be kept in oral cavity for 4-5 hours, and then was be swallowed before meal time or kept in oral cavity for the whole release duration.

### Embodiment 26 Preparation of extended release platform (Each tablet comprises 37.5mgCD + 150mgLD)

In this embodiment, the procedures of embodiment 25 were repeated, and the dosage form comprising a drug layer, an osmotic layer, a membrane-forming composition and an overcoat were all identical to those provided in embodiment 25.

First, a drug layer composition comprising 45.0Wt% of levodopa, 31.0Wt% of hydroxypropyl cellulose, 22.0Wt% of mannitol, 0.9Wt% of aspartame, 0.1Wt% of Mint flavor and 0.5Wt% of magnesium stearate was prepared, the components were each passed through a 40-mesh stainless steel sieve, and dried to obtain dry granules by a dry granulator, then mixed with 0.5Wt% of magnesium stearate.

Next, a second composition, i.e. an osmosis layer, comprising 55.0Wt% of sodium carboxymethyl cellulose 7H4XF, 34.0Wt% of sorbitol, 10.0Wt% of hydroxypropyl cellulose and 0.5Wt% of iron oxide red was prepared; the components were each passed through a 40-mesh stainless steel sieve, and dried to obtain dry granules by a dry granulator, then mixed with 0.5Wt% of magnesium stearate.

Next, the drug layer and osmotic layer granules were compressed into a bi-layer tablet core. Firstly, 250 mg of drug layer granules were added to a 9 mm round punch of a tablet press and tamped, then 125 mg of osmotic layer granules were added to the punch, and the two layers of granules were compressed with a tablet press into a contact bi-layer tablet core.

Next, the bi-layer tablet core was coated with a semi-permeable membrane. The membrane-forming composition comprised, in weight percentage, 70Wt% of cellulose acetate with an acetyl content of 39.8Wt%, and 30Wt% of Copovidone VA64. The membrane-forming composition was mixed with acetone to make a 4% of solid suspension. Using the technological parameters listed in embodiment 6, the membrane-forming composition was sprayed onto the bi-layer tablet core in a Glatt GC 1 pan coater to form a coating membrane, and the membrane weight gain of the coated tablet was 7.0%. Finally, a 0.75 mm exit orifice was drilled mechanically on the drug layer side of the dosage form.

Next, an immediate-release composition, comprising 42.8Wt% of levodopa, 46.2Wt% of carbidopa monohydrate, 10.0Wt% of hydroxypropyl cellulose, 0.9Wt% of aspartame and 0.1Wt% of Mint Flavor, was used to overcoat the dried dosage form, with the membrane weight gain of 7.0%. The immediate-release overcoat composition was mixed with anhydrous ethanol to make a 10.0Wt% of solid suspension. The final dosage form comprises an immediate-release overcoat comprising 37.5 mg of levodopa and 37.5 mg of carbidopa, and a controlled-release drug layer comprising 112.5 mg of levodopa.

As shown in Fig. 19, the release profile of the dosage form showed rapid release of LD/CD, followed by an extended release with a release duration of approximately 8 hours. The osmotic delivery system can be kept in oral cavity for 4-5 hours, and then be swallowed before meal time or kept in oral cavity for the whole release duration.

### Embodiment 27 Preparation of extended release platform (Each tablet contains 37.5mgCD + 75mgLD)

In this embodiment, the procedures of embodiment 25 were repeated, and the dosage form comprised a drug layer, an osmosis layer, a membrane-forming composition and an overcoat were identical to those provided in embodiment 25.

First, a drug layer composition comprising 45.0Wt% of levodopa, 31.0Wt% of hydroxypropyl cellulose, 22.0Wt% of mannitol, 0.9Wt% of aspartame, 0.1Wt% of Mint Flavor and 0.5Wt% of magnesium stearate was prepared, the components were each passed through a 40-mesh stainless steel sieve, and dried to obtain dry granules by a dry granulator, then mixed with 0.5Wt% of magnesium stearate.

Next, a second composition, i.e. an osmosis layer, comprising 55.0Wt% of sodium carboxymethyl cellulose 7H4XF, 34.0Wt% of sorbitol, 10.0Wt% of hydroxypropyl cellulose and 0.5Wt% of iron oxide red was prepared; the components were each passed through a 40-mesh stainless steel sieve, and dried to obtain dry granules by a dry granulator, then mixed with 0.5Wt% of magnesium stearate.

Next, the drug layer and osmotic layer granules were compressed into a bi-layer tablet core. Firstly, 125 mg of drug layer granules were added to a 7 mm round punch of a tablet press and tamped, then 62.5 mg of osmotic layer granules were added to the punch, and the two layers of granules were compressed into a contact bi-layer tablet core with a tablet press.

Next, the bi-layer tablet core was coated with a semi-permeable membrane. The membrane-forming composition comprised, in weight percentage, 70Wt% of cellulose acetate with an acetyl content of 39.8Wt%, and 30Wt% of Copovidone VA64. The membrane-forming composition was mixed with acetone to make a 4% of solid suspension. Using the process parameters listed in Embodiment 6, the membrane-forming composition was sprayed onto the bi-layer tablet core in a Glatt GC 1 pan coater to form a coating membrane, and the membrane weight gain of the coated tablet was 9.0%. Finally, a 0.5 mm exit orifice was drilled mechanically on the drug layer side of the dosage form.

Next, an immediate-release composition comprising 28.2Wt% of levodopa, 60.8Wt% of carbidopa monohydrate, 10.0Wt% of hydroxypropyl cellulose, 0.9Wt% of aspartame and 0.1Wt% of Mint flavor, was used to overcoat the dried dosage form, with a membrane weight gain of 9.0%. The immediate-release overcoat composition was mixed with anhydrous ethanol to make a 10.0Wt% of solid suspension. The final dosage form comprised an immediate-release overcoat comprising 18.75 mg of levodopa and 37.5 mg of carbidopa, and a controlled-release drug layer comprising 56.25 mg of levodopa.

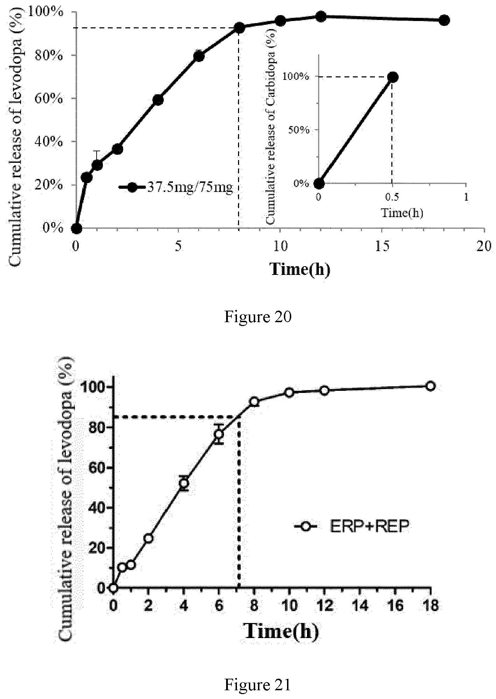

As shown in Fig. 20, the release profile of the dosage form showed rapid release of LD/CD, followed by an extended release with a release duration of approximately 8 hours. The osmotic delivery system can be kept in oral cavity for 4-5 hours, and then was be swallowed before meal time or kept in oral cavity for the whole release duration.

### Embodiment 28 Coordination of extended release platform and retention enabling platform

The extended release platform (ERP) of embodiments 6-27 was fastened to the personalized retention enabling platform (REP) of embodiments 1-5 to form the extended release dosage form (ERP + REP) of the present invention. Then, the extended release dosage form (ERP + REP) was fastened on the corresponding teeth in the oral cavity. The extended release dosage form (ERP + REP) can be maintained in the oral cavity until the push layer reaches the delivery orifice, or maintained there for 4-24 hours. The extended release dosage form (ERP + REP) was taken out, the extended release platform (ERP) was replaced, and the extended release dosage form (ERP + REP) was fastened on the corresponding teeth in the oral cavity again, thus releasing the drug continuously.

### Embodiment 29 Coordination of extended release platform and retention enabling platform (Each tablet comprises 62.5mgCD + 250mgLD)

In this embodiment, the procedures of embodiment 9 were repeated, the dried dosage form with 4.8% membrane weight gain was coated with an immediate release composition comprising 23.79 wt% of levodopa, 64.22 wt% of carbidopa, 10.0 wt% of hydroxypropyl cellulose and 1.0 wt% of aspartame (as shown in Fig. 3c). The immediate release overcoat composition was mixed with absolute ethanol to make a 6.7 wt% of solid suspension. The final extended release platform ERP dosage form comprises an immediate release coating layer of 62.5 mg carbidopa and 25 mg levodopa, and an extended release drug-containing layer of 225 mg levodopa.

The extended release platform (ERP) was fastened to the personalized retention enabling platform (REP) to form the extended release dosage form (ERP + REP) of the present invention. Then, the extended release dosage form (ERP + REP) was fastened on the corresponding teeth in the oral cavity. The extended release dosage form (ERP + REP) can be maintained in the oral cavity until the push layer reaches the delivery orifice or maintained there for 8 hours. The extended release dosage form (ERP + REP) was taken out, the extended release platform (ERP) was replaced, and the extended release dosage form (ERP + REP) was fastened on the corresponding teeth in the oral cavity again, thus releasing the drug continuously.

The USP I basket method was used to determine the release profile of an extended release dosage form (ERP + REP) in a 0.1N hydrochloric acid aqueous solution. As shown in Fig. 21, an extended release dosage form (ERP + REP) with a membrane weight gain of 4.8% delivered LD at an average rate of 27.6 mg/hr, with 85% of LD delivered in 7.7 hours and 85% of CD delivered in 1 hour.

## Claims

1. An extended release dosage form with an absorption window at the upper gastrointestinal tract comprising an extended release platform and a retention enabling platform;
wherein the extended release platform is a pharmaceutical composition comprising a tablet core and a coating membrane, the tablet core comprises a drug-containing layer, and the coating membrane comprises cellulose acetate and copovidone, wherein the weight of the cellulose acetate is 50-70% of the weight of the coating membrane, the weight of the copovidone is 30-50% of the weight of the coating membrane;
wherein the retention enabling platform is used for maintaining the extended release platform in the oral cavity, and at least one end of the retention enabling platform is connected with a cover, so that the extended release platform can be retained in the retention enabling platform;
the drug-containing layer comprises active pharmaceutical ingredients and excipients, and the active pharmaceutical ingredient is one or more of levodopa and carbidopa.

2. The extended release dosage form of claim 1, wherein the retention enabling platform comprises a personalized retention enabling module and a drug fastened module, the drug fastened module can fix the extended release platform, and the personalized retention enabling module can maintain the drug fastened module in the oral cavity;
optionally, the drug fastened module is one or more reservoirs; and/or, the retention enabling module can fit to any one or more teeth in the oral cavity; and/or, the retention enabling module can be customized to fit and wrap, clamp or insert maxillary or mandibular permanent teeth;
wherein, the reservoir has a basket structure; and/or, the shape of the cross section of the reservoir is polygon, circular closed-loop or circular open-loop, or a combination thereof; and/or,
at least one end of the reservoir is connected with a cover, so that the extended release platform can be maintained in the reservoir; the cover optionally is a strip; and/or,
the retention enabling module can fit to any one or more of the mandibular permanent teeth, the mandibular molar, the mandibular second molar and its anterior and posterior molar teeth in the oral cavity; and/or,
the maxillary or mandibular permanent teeth can be entire maxillary permanent teeth, or entire mandibular permanent teeth, or the mandibular molar, or the second mandibular molar and its adjacent parts of the first molar and the second bicuspid.

3. A method for preparing the extended release dosage form of any one of claims 1 or 2, wherein the method for preparing the retention enabling platform comprises 3D printing, injection molding or impression molding; optionally, the retention enabling platform is prepared from one or more oral stable materials, and the oral stable materials comprise oral stable metals and thermoplastic elastomers; wherein, the oral stable metal comprises dental titanium, stainless steel, cobalt chromium alloy, cobalt chromium molybdenum alloy, nickel chromium alloy or precious metal, and the thermoplastic elastomer comprises polycaprolactone, ethylene vinyl acetate copolymer, high density polyethylene, polypropylene, polyacrylate, polyurethane, silicone polymer, polyester, poly (styrene-ethylene-butene-styrene), poly (styrene-butadiene-styrene), poly (styrene-isoprene-styrene), or a copolymer of any two or more of the above, or their physical combination.

4. The extended release dosage form of claim 1, wherein in the extended release platform, in the drug-containing layer, the excipient is one or more of filler, osmotic agent, hydrophilic polymer, binding agent, lubricant, preservative, flavoring agent, acidifying agent and antioxidant; or, the excipient is one or more of filler, osmotic agent, hydrophilic polymer, binding agent, lubricant and preservative; or, the excipients are filler, osmotic agent, hydrophilic polymer, binding agent, lubricant and preservative ;
wherein, when the pharmaceutically active ingredient comprises levodopa, the weight percentage of the levodopa is 20-70%; when the active ingredient comprises carbidopa, the weight percentage of carbidopa is 0-20% but not 0%; wherein the weight percentage is the weight percentage of each component of the drug-containing layer;
or, when the pharmaceutically active ingredient comprises levodopa, the weight percentage of the levodopa is 30-50%; when the active ingredient comprises carbidopa, the weight percentage of carbidopa is 1-10%; wherein the weight percentage is the weight percentage of each component of the drug-containing layer.

5. The extended release dosage form of claim 4, wherein in the extended release platform:
when the excipient comprises a filler, the filler is one or more of microcrystalline cellulose, hydroxypropyl cellulose and mannitol, and the weight percentage of the filler is 0-50% but not 0%;
and/or, when the excipient comprises an osmotic agent, the osmotic agent is one or more of magnesium sulfate, magnesium chloride, sodium chloride, lithium chloride, potassium sulfate, sodium sulfate, mannitol, urea, sorbitol, inositol, sucrose and glucose, and the weight percentage of the osmotic agent is 0-50% but not 0%;
and/or, when the excipient comprises a hydrophilic polymer, the hydrophilic polymer is one or more of hydroxypropyl cellulose, hydroxypropyl methyl cellulose, carboxymethyl cellulose, polyvinylpyrrolidone and hydroxyethyl cellulose, and the weight percentage of the hydrophilic polymer is 0-50% but not 0%;
and/or, when the excipient comprises an acidifying agent, the acidifying agent is one or more of citric acid, sodium citrate, potassium citrate, malic acid, fumaric acid, lactic acid, phosphoric acid and tartaric acid, and the weight percentage of the acidifying agent is 0-10% but not 0%;
wherein the weight percentage is the weight percentage of each component of the drug-containing layer.

6. The extended release dosage form of claim 4 or 5, wherein in the extended release platform, the tablet core of the pharmaceutical composition further comprises an osmotic push layer, and the osmotic push layer comprising a hydrophilic polymer, an osmotic agent and a binding agent; optionally, the osmotic push layer comprises a hydrophilic polymer, an osmotic agent, a binding agent, and a lubricant; or, the osmotic push layer comprises a hydrophilic polymer, an osmotic agent, a binding agent, a lubricant and a colorant.

7. The extended release dosage form of claim 6, wherein in the extended release platform, the hydrophilic polymer in the osmotic push layer is κ-carrageenan,sodium carboxymethyl cellulose or polyethylene oxide, wherein the molecular weight of the hydrophilic polymer is 75,000-7,500,000, and the weight percentage of the hydrophilic polymer is 25-85%;
and/or, the osmotic agent in the osmotic pump push layer is one or more of magnesium sulfate, magnesium chloride, sodium chloride, lithium chloride, potassium sulfate, sodium sulfate, mannitol, urea, sorbitol, inositol, sucrose and glucose, and the weight percentage of the osmotic agent is 5-65%;
and/or, when the osmotic push layer comprises a binding agent, the binding agent is one or more of methyl cellulose, hydroxypropyl methyl cellulose, hydroxypropyl cellulose, sodium carboxymethyl cellulose, povidone and gelatin, and the weight percentage of the binding agent is 3-20%;
and/or, when the osmotic push layer comprises a lubricant, the lubricant is one or more of magnesium stearate, magnesium stearate fumarate, talc, and colloidal silica, and the weight percentage of the lubricant is 0-2% but not 0%;
and/or, when the osmotic push layer comprises a colorant, the colorant is one or more of iron oxide red, iron oxide yellow, and iron oxide black, and the weight percentage of the colorant is 0-5% but not 0%;
wherein the weight percentage is the weight percentage of each component of the osmotic push layer.

8. The extended release dosage form of claim 6, wherein in the extended release platform, the osmotic push layer comprises sodium carboxymethyl cellulose, povidone K30, sorbitol, iron oxide red and magnesium stearate; or comprises sodium carboxymethyl cellulose, hydroxypropyl cellulose, sorbitol, iron oxide red and magnesium stearate;
optionally, the sodium carboxymethyl cellulose is sodium carboxymethyl cellulose 7H4XF or 9H4XF;
and/or, the osmotic push layer comprises 25-85 wt% of sodium carboxymethyl cellulose, 5-65 wt% of sorbitol, 3-20 wt% of povidone, 0-5 wt% of iron oxide red and 0.5-2 wt% of magnesium stearate;
or, the osmotic push layer comprises 55 wt% of sodium carboxymethyl cellulose, 34.0-39.0 wt% of sorbitol, 3-20 wt% of povidone or 10 wt% of hydroxylpropyl cellulose, 0.5-5 wt% of iron oxide red and 0.5-2 wt% of magnesium stearate;
or, the osmotic push layer comprises 55 wt% of sodium carboxymethyl cellulose, 39.0 wt% of sorbitol, 5.0 wt% of povidone and 0.5 wt% of iron oxide red and 0.5 wt% of magnesium stearate; or comprises 55 wt% of sodium carboxymethyl cellulose, 34.0 wt% of sorbitol, 10.0 wt% of povidone, 0.5 wt% of iron oxide red and 0.5 wt% of magnesium stearate, or comprises 55 wt% of sodium carboxymethyl cellulose, 34.0 wt% of sorbitol, 10.0 wt% of hydroxypropyl cellulose, 0.5 wt% of iron oxide red and 0.5 wt% of magnesium stearate; wherein the weight percentage is the weight percentage of each component of the osmotic push layer.

9. The extended release dosage form of any one of claims 5-8, wherein in the extended release platform, the coating membrane of the pharmaceutical composition is further overcoated with a drug-containing immediate release overcoat;
the drug-containing immediate release overcoat optionally comprises an active pharmaceutical ingredient and an excipient, the active pharmaceutical ingredient comprises levodopa and/or carbidopa, and the excipient is one or more of hydroxypropyl cellulose, aspartame and Mint Flavor;
wherein, when the active pharmaceutical ingredient is levodopa, the weight percentage of the levodopa is 0-75% but not 0%, e.g., 23.78-75%; and/or, when the active pharmaceutical ingredient is carbidopa, the weight percentage of carbidopa is 0-93% but not 0%, e.g., 26.85-93%; and/or, when the excipient of the overcoat comprises hydroxypropyl cellulose, the weight percentage of the hydroxypropyl cellulose is 2-20%, e.g., 10%; and/or, when the excipient of the overcoat comprises aspartame, the weight percentage of the aspartame is 0-5%, e.g., 0.9-5%; and/or, when the excipient of the overcoat comprises Mint Flavor, the weight percentage of the Mint Flavor is 0-5%, e.g., 0.1%; wherein the weight percentage is the weight percentage of each component of the overcoat.

10. The extended release dosage form of any one of claims 4-9, wherein in the extended release platform, the weight of the coating membrane is not less than 2.0%, e.g. 2.0-15.0% or 4.0-8.0% of the weight of the tablet core; the coating membrane has one or more orifices, and the diameter of orifice is optionally 0.5 mm-1.0 mm, e.g., 0.5 mm, 0.75 mm and 1.0 mm;
optionally, in the extended release platform, the pharmaceutical composition is composed of a drug-containing layer and a coating membrane; the pharmaceutical composition is composed of a drug-containing layer, an osmotic push layer and a coating membrane; or the pharmaceutical composition is composed of a drug-containing layer, an osmotic push layer, a coating membrane, and an overcoat;
and/or, the drug-containing layer is composed of levodopa, carbidopa, microcrystalline cellulose, mannitol, citric acid, sodium hydroxypropyl methyl cellulose and magnesium stearate; or the drug-containing layer is composed of levodopa, microcrystalline cellulose, hydroxypropyl methyl cellulose and magnesium stearate; or the drug-containing layer is composed of levodopa, carbidopa, mannitol, citric acid and magnesium stearate; or the drug-containing layer is composed of levodopa, carbidopa, hydroxypropyl cellulose, mannitol, citric acid and magnesium stearate; or the drug-containing layer is composed of levodopa, carbidopa, hydroxypropyl cellulose, mannitol, citric acid, and povidone K30; or the drug-containing layer is composed of levodopa, hydroxypropyl cellulose, mannitol, povidone K30, magnesium stearate, Mint Flavor and aspartame; or the drug-containing layer is composed of levodopa, mannitol, povidone K30 and magnesium stearate; or the drug-containing layer is composed of levodopa, carbidopa, hydroxypropyl cellulose, mannitol, aspartame and magnesium stearate; or the drug-containing layer is composed of levodopa, hydroxypropyl cellulose, mannitol, magnesium stearate, Mint Flavor and aspartame; or the drug-containing layer is composed of levodopa, hydroxypropyl cellulose, mannitol, povidone K30, magnesium stearate and aspartame; or the drug-containing layer is composed of levodopa, hydroxypropyl cellulose, mannitol, povidone K30, magnesium stearate, and aspartame;
and/or, the osmotic push layer is composed of sodium carboxymethyl cellulose, povidone K30, sorbitol, iron oxide red and magnesium stearate, or the osmotic push layer is composed of sodium carboxymethyl cellulose, hydroxypropyl cellulose, sorbitol, iron oxide red and magnesium stearate; optionally, the sodium carboxymethyl cellulose is sodium carboxymethyl cellulose 7H4XF or 9H4XF;
and/or, the overcoat is composed of levodopa, carbidopa, hydroxypropyl cellulose, aspartame, and Mint Flavor; or the overcoat is composed of levodopa, carbidopa, hydroxypropyl cellulose and aspartame; or the overcoat is composed of carbidopa, hydroxypropyl cellulose and aspartame; or the overcoat is composed of levodopa, hydroxypropyl cellulose, and Mint Flavor.

11. The extended release dosage form of claim 10, wherein in the extended release platform, when the drug-containing layer is composed of levodopa, carbidopa, microcrystalline cellulose, mannitol, citric acid, sodium hydroxypropyl methyl cellulose and magnesium stearate, the weight percentage of levodopa is 40%, the weight percentage of carbidopa is 10.8%, the weight percentage of microcrystalline cellulose is 20%, the weight percentage of mannitol is 18.7%, the weight percentage of citric acid is 5%, the weight percentage of hydroxypropyl methyl cellulose sodium is 5%, and the weight percentage of magnesium stearate is 0.5%, wherein the weight percentage is the weight percentage of each component of the drug-containing layer;
when the drug-containing layer is composed of levodopa, microcrystalline cellulose, hydroxypropyl methyl cellulose and magnesium stearate, and the weight percentage of the levodopa is 38%, the weight percentage of microcrystalline cellulose is 50%, the weight percentage of hydroxypropyl methyl cellulose is 10%, and the weight percentage of magnesium stearate is 2%, wherein the weight percentage is the weight percentage of each component of the drug-containing layer;
when the drug-containing layer is composed of levodopa, carbidopa, mannitol, citric acid and magnesium stearate, the weight percentage of the levodopa is 19.5%, and the weight percentage of carbidopa is 20%, the weight percentage of mannitol is 50%, the weight percentage of citric acid is 10%, and the weight percentage of magnesium stearate is 0.5%, wherein the weight percentage is the weight percentage of each component of the drug-containing layer;
when the drug-containing layer is composed of levodopa, carbidopa, hydroxypropyl cellulose, mannitol, citric acid and magnesium stearate, the weight percentage of the levodopa is 40 %, the weight percentage of carbidopa is 10.8%, the weight percentage of hydroxypropyl cellulose is 31%, the weight percentage of mannitol is 12.7%, and the weight percentage of citric acid is 5%, the weight percentage of the magnesium stearate is 0.5%, wherein the weight percentage is the weight percentage of each component of the drug-containing layer;
when the drug-containing layer is composed of levodopa, carbidopa, hydroxypropyl cellulose, mannitol, citric acid and povidone K30, the weight percentage of levodopa is 40 %, the weight percentage of carbidopa is 10.8%, the weight percentage of hydroxypropyl cellulose is 31%, the weight percentage of mannitol is 12.7%, and the weight percentage of citric acid is 5%, the weight percentage of povidone K30 is 0.5%, wherein the weight percentage is the weight percentage of each component of the drug-containing layer;
when the drug-containing layer is composed of levodopa, hydroxypropyl cellulose, mannitol, povidone K30, magnesium stearate, Mint Flavor and aspartame, the weight percentage of levodopa is 45%, the weight percentage of hydroxypropyl cellulose is 31%, the weight percentage of mannitol is 16%, the weight percentage of povidone K30 is 5%, the weight percentage of the magnesium stearate is 1%, the weight percentage of the Mint Flavor is 1%, and the weight percentage of the aspartame is 1%, wherein the weight percentage is the weight percentage of each component of the drug-containing layer;
when the drug-containing layer is composed of levodopa, mannitol, povidone K30, and magnesium stearate, the weight percentage of levodopa is 70%, the weight percentage of mannitol is 9%, the weight percentage of povidone K30 is 20%, and the weight percentage of magnesium stearate is 1%, wherein the weight percentage is the weight percentage of each component of the drug-containing layer;
when the drug-containing layer is composed of levodopa, carbidopa, hydroxypropyl cellulose, mannitol, aspartame and magnesium stearate, the weight percentage of levodopa is 20%, the weight percentage of carbidopa is 20%, the weight percentage of hydroxypropyl cellulose is 50%, the weight percentage of mannitol is 4%, and the weight percentage of aspartame is 5%, the weight percentage of magnesium stearate is 1%, wherein the weight percentage is the weight percentage of each component of the drug-containing layer;
when the drug-containing layer is composed of levodopa, hydroxypropyl cellulose, mannitol, povidone K30, magnesium stearate, and aspartame, the weight percentage of levodopa is 45%, the weight percentage of the hydroxypropyl cellulose is 31%, the weight percentage of the mannitol is 16%, the weight percentage of the povidone K30 is 5%, the weight percentage of magnesium stearate is 1%, the weight percentage of the Mint Flavor is 1%, and the weight percentage of aspartame is 1%, wherein the weight percentage is the weight percentage of each component of the drug-containing layer;
when the drug-containing layer is composed of levodopa, hydroxypropyl cellulose, mannitol, povidone K30, magnesium stearate and aspartame, the weight percentage of levodopa is 45%, the weight percentage of the hydroxypropyl cellulose is 31%, the weight percentage of the mannitol is 17%, the weight percentage of the povidone K30 is 5%, the weight percentage of magnesium stearate is 1% and the weight percentage of aspartame is 1%, wherein the weight percentage is the weight percentage of each component of the drug-containing layer;
or, when the drug-containing layer is composed of levodopa, hydroxypropyl cellulose, mannitol, povidone K30, magnesium stearate, Mint Flavor and aspartame; the weight percentage of the levodopa is 45%, the weight percentage of the hydroxypropyl cellulose is 31%, the weight percentage of the mannitol is 12%, and the weight percentage of the povidone K30 is 5%, the weight percentage of the Mint Flavor is 5%, the weight percentage of aspartame is 1%, and the weight percentage of magnesium stearate is 1%, wherein the weight percentage is the weight percentage of each component of the drug-containing layer;
or, when the drug-containing layer is composed of levodopa, hydroxypropyl cellulose, mannitol, magnesium stearate, Mint Flavor and aspartame, the weight percentage of the levodopa is 62.5%, the weight percentage of the hydroxypropyl cellulose is 31%, the weight percentage of the mannitol is 4.5%, the weight percentage of the Mint Flavor is 0.1%, the weight percentage of aspartame is 0.9% and the weight percentage of magnesium stearate is 1%, wherein the weight percentage is the weight percentage of each component of the drug-containing layer;
or, when the drug-containing layer is composed of levodopa, hydroxypropyl cellulose, mannitol, magnesium stearate, Mint Flavor and aspartame, the weight percentage of the levodopa is 46.9%, the weight percentage of the hydroxypropyl cellulose is 31%, the weight percentage of the mannitol is 20.1%, the weight percentage of the Mint Flavor is 0.1%, the weight percentage of aspartame is 0.9%, the weight percentage of magnesium stearate is 1%, wherein the weight percentage is the weight percentage of each component of the drug-containing layer.

12. The extended release dosage form of claim 10, wherein in the extended release platform, the coating membrane is composed of 50 wt% of cellulose acetate membrane and 50 wt% of copovidone VA64; the coating membrane is composed of 70 wt% of cellulose acetate membrane and 30 wt% of copovidone VA64; the coating membrane is composed of 60 wt% of cellulose acetate membrane and 40 wt% of copovidone VA64; wherein the weight percentage is the weight percentage of each component of the coating membrane;
optionally, the weight of the coating membrane is 2.0%, 4.2%, 4.5%, 4.6%, 4.8%, 5.0%, 5.9%, 6.5%, 6.7%, 7.0%, 7.7%, 7.9% or 9.7% of the weight of the tablet core.

13. The extended release dosage form of any one of claims 10-12, wherein in the extended release platform,
when the osmotic push layer is composed of sodium carboxymethyl cellulose 7H4XF, povidone K30, sorbitol, iron oxide red and magnesium stearate,
the weight percentage of the carboxymethyl cellulose sodium 7H4XF is 55%, the weight percentage of the povidone K30 is 5%, the weight percentage of the sorbitol is 39%, the weight percentage of the iron oxide red is 0.5%, and the weight percentage of the magnesium stearate is 0.5%, wherein the weight percentage is the weight percentage of each component of the osmotic push layer;
the weight percentage of the carboxymethyl cellulose sodium 7H4XF is 55%, the weight percentage of the povidone K30 is 10%, the weight percentage of the sorbitol is 34%, the weight percentage of the iron oxide red is 0.5%, and the weight percentage of the magnesium stearate is 0.5%, wherein the weight percentage is the weight percentage of each component of the osmotic push layer;
the weight percentage of the sodium carboxymethyl cellulose is 85%, the weight percentage of the povidone K30 is 3%, the weight percentage of the sorbitol is 5%, the weight percentage of the iron oxide red is 5%, and the weight percentage of the magnesium stearate is 2%, wherein the weight percentage is the weight percentage of each component of the osmotic push layer;
the weight percentage of the sodium carboxymethyl cellulose is 25%, the weight percentage of the povidone K30 is 9.5%, the weight percentage of the sorbitol is 65% and the weight percentage of the magnesium stearate is 0.5%, wherein the weight percentage is the weight percentage of each component of the osmotic push layer;
the weight percentage of the carboxymethyl cellulose sodium 7H4XF is 60%, the weight percentage of the povidone K30 is 10%, the weight percentage of the sorbitol is 26%, the weight percentage of the iron oxide red is 2%, and the weight percentage of the magnesium stearate is 2%, wherein the weight percentage is the weight percentage of each component of the osmotic push layer;
the weight percentage of the carboxymethyl cellulose sodium 7H4XF is 40%, the weight percentage of the povidone K30 is 20%, the weight percentage of the sorbitol is 36%, the weight percentage of the iron oxide red is 3.5%, and the weight percentage of the magnesium stearate is 0.5%, wherein the weight percentage is the weight percentage of each component of the osmotic push layer;
or, when the osmotic push layer is composed of sodium carboxymethyl cellulose 9H4XF, povidone K30, sorbitol, iron oxide red and magnesium stearate, the weight percentage of sodium carboxymethyl cellulose 9H4XF is 55%, the weight percentage of povidone K30 is 5%, the weight percentage of sorbitol is 39%, and the weight percentage of iron oxide red is 0.5 % and the weight percentage of magnesium stearate is 0.5%, wherein the weight percentage is the weight percentage of each component of the osmotic push layer;
or, when the osmotic push layer is composed of sodium carboxymethyl cellulose 7H4XF, hydroxypropyl cellulose, sorbitol, iron oxide red and magnesium stearate, the weight percentage of sodium carboxymethyl cellulose 7H4XF is 55%, the weight percentage of hydroxypropyl cellulose is 10%, the weight percentage of sorbitol is 34 %, the weight percentage of the iron oxide red is 0.5% and the weight percentage of the magnesium stearate is 0.5%, wherein the weight percentage is the weight percentage of each component of the osmotic push layer.

14. The extended release dosage form of any one of claims 10-13, wherein in the extended release platform, when the overcoat is composed of levodopa, carbidopa, hydroxypropyl cellulose, aspartame and Mint Flavor, the weight percentage of the levodopa is 23.78%, the weight percentage of the carbidopa is 64.22%, the weight percentage of the hydroxypropyl cellulose is 10%, and the weight percentage of the aspartame is 1%, the weight percentage of the Mint Flavor is 1%; or the weight percentage of the levodopa is 54%, the weight percentage of the carbidopa is 35%, the weight percentage of the hydroxypropyl cellulose is 10%, and the weight percentage of the aspartame is 0.9%, the weight percentage of the Mint Flavor is 0.1%; the weight percentage of the levodopa is 42.8%, the weight percentage of the carbidopa is 46.2%, the weight percentage of the hydroxypropyl cellulose is 10%, and the weight percentage of the aspartame is 0.9%, and the weight percentage of the Mint Flavor is 0.1%, wherein the weight percentage is the weight percentage of each component of the overcoat;
when the overcoat is composed of levodopa, carbidopa, hydroxypropyl cellulose, aspartame and Mint Flavor, the weight percentage of the levodopa is 62.15%, the weight percentage of the carbidopa is 26.85%, the weight percentage of the hydroxypropyl cellulose is 10%, the weight percentage of the aspartame is 0.9%, and the weight percentage of the Mint Flavor is 0.1%;
or, the weight percentage of the levodopa is 42.8%, the weight percentage of the carbidopa is 46.2%, the weight percentage of the hydroxypropyl cellulose is 10%, the weight percentage of the aspartame is 0.9%, the weight percentage of the Mint Flavor is 0.1%, or the weight percentage of the levodopa is 28.2%, and the weight percentage of the carbidopa is 60.8%, the weight percentage of the hydroxypropyl cellulose is 10%, the weight percentage of the aspartame is 0.9%, and the weight percentage of the Mint Flavor is 0.1%, wherein the weight percentage is the weight percentage of each component of the overcoat;
or, when the overcoat is composed of carbidopa, hydroxypropyl cellulose and aspartame, the weight percentage of carbidopa is 93%, the weight percentage of hydroxypropyl cellulose is 2% and the weight percentage of aspartame is 5%, wherein the weight percentage is the weight percentage of each component of the overcoat;
or, when the overcoat is composed of levodopa, hydroxypropyl cellulose and Mint Flavor, the weight percentage of the levodopa is 75%, the weight percentage of the hydroxypropyl cellulose is 20%, and the weight percentage of the Mint Flavor is 5%, wherein the weight percentage is the weight percentage of each component of the overcoat;
or, when the overcoat is composed of levodopa, carbidopa, hydroxypropyl cellulose and aspartame, the weight percentage of levodopa is 24%, the weight percentage of carbidopa is 65%, the weight percentage of hydroxypropyl cellulose is 10% and the weight percentage of aspartame is 1 %, wherein the weight percentage is the weight percentage of each component of the overcoat;
optionally, the weight gain of the overcoat relative to the tablet core is 12.9% or 13.2% or 15.7% or 21.0% by weight.

15. The extended release dosage form of any one of claims 10-14, wherein in the extended release platform, the pharmaceutical composition is composed of a drug-containing layer and a coating membrane, the drug containing layer is composed of 40 wt% of levodopa, 10.8 wt% of carbidopa, 20 wt% of microcrystalline cellulose, 18.7 wt% of mannitol, 5 wt% of citric acid, 5 wt% of hydroxypropyl methyl cellulose sodium and 0.5 wt% of magnesium stearate, wherein the weight percentage is the weight percentage of each component of the drug-containing layer; the coating membrane is composed of 50% wt% of cellulose acetate membrane and 50 wt% of copovidone VA64, wherein the weight percentage is the weight percentage of each component of the coating membrane; the weight of the coating membrane is 2.0% of the weight of the tablet core;
or, the pharmaceutical composition is composed of a drug-containing layer and a coating membrane, the drug-containing layer is composed of 38 wt% of levodopa, 50 wt% of microcrystalline cellulose, 10 wt% of hydroxypropyl methyl cellulose and 2 wt% of magnesium stearate, wherein the weight percentage is the weight percentage of each component of the drug-containing layer; the coating membrane is composed of 50 wt% of cellulose acetate membrane and 50 wt% of copovidone VA64, wherein the weight percentage is the weight percentage of each component of the coating membrane, and the weight of the coating membrane is 4.5% of the weight of tablet core;
or, the pharmaceutical composition is composed of a drug-containing layer and a coating membrane, the drug-containing layer comprises 19.5 wt% of levodopa, 20 wt% of carbidopa, 50 wt% of mannitol, 10 wt% of citric acid and 0.5 wt% of magnesium stearate, wherein the weight percentage is the weight percentage of each component of the drug-containing layer; the coating membrane is composed of 50 wt% of cellulose acetate membrane and 50 wt% of copovidone VA64, wherein the weight percentage is the weight percentage of each component of the coating membrane, and the weight of the coating membrane is 4.5% of the weight of tablet core;
or, the pharmaceutical composition is composed of a drug-containing layer, an osmotic push layer, and a coating membrane, and the drug-containing layer is composed of 40 wt% of levodopa, 10.8 wt% of carbidopa, 31 wt% of hydroxypropyl cellulose, 12.7 wt% of mannitol, 5 wt% of citric acid and 0.5 wt% of magnesium stearate, wherein the weight percentage is the weight percentage of each component of the drug-containing layer; the osmotic push layer is composed of 55 wt% of sodium carboxymethyl cellulose 7H4XF or 9H4XF, 5 wt% of povidone K30, 39 wt% of sorbitol, 0.5 wt% of iron oxide red and 0.5 wt% of magnesium stearate, wherein the weight percentage is the weight percentage of each component of the osmotic push layer; the coating membrane is composed of 70 wt% of cellulose acetate membrane and 30 wt% of copovidone VA64, wherein the weight percentage is the weight percentage of each component of the coating membrane; the weight of the coating membrane is 2.0%, 4.0% or 5.0% of the weight of the tablet core;
or, the pharmaceutical composition is composed of a drug-containing layer, an osmotic push layer, and a coating membrane, the drug-containing layer is composed of 40 wt% of levodopa, 10.8 wt% of carbidopa, 31 wt% of hydroxypropyl cellulose, 12.7 wt% of mannitol, 5 wt% of citric acid and 0.5 wt% of povidone K30, wherein the weight percentage is the weight percentage of each component of the drug-containing layer; the osmotic push layer is composed of 55 wt% of sodium carboxymethyl cellulose 7H4XF, 5 wt% of povidone K30, 39 wt% of sorbitol, 0.5 wt% of iron oxide red, and 0.5 wt% of magnesium stearate, wherein the weight percentage is the weight percentage of each component of the osmotic push layer; the coating membrane is composed of 60 wt% of cellulose acetate membrane and 40% copovidone VA64, wherein the weight percentage is the weight percentage of each component of the coating membrane; and the weight of the coating membrane is 5.0% of the weight of the tablet core;
or, the pharmaceutical composition is composed of a drug-containing layer, an osmotic push layer, and a coating membrane, the drug-containing layer is composed of 45 wt% of levodopa, 31 wt% of hydroxypropyl cellulose, 16 wt% of mannitol, 5 wt% of povidone K30, and 1 wt% of magnesium stearate, 1 wt% of Mint Flavor and 1 wt% of aspartame, wherein the weight percentage is the weight percentage of each component of the drug-containing layer; the osmotic push layer is composed of 55 wt% of sodium carboxymethyl cellulose 7H4XF, 10 wt% of povidone K30, 34 wt% of sorbitol, 0.5 wt% of iron oxide red and 0.5 wt% of magnesium stearate, wherein the weight percentage is the weight percentage of each component of the osmotic push layer; the coating membrane is composed of 60 wt% of cellulose acetate membrane and 40 wt% of copovidone VA64, wherein the weight percentage is the weight percentage of each component of the coating membrane; and the weight of the coating membrane is 4.2%, 6.7% or 9.7% of the weight of the tablet core;
or, the pharmaceutical composition is composed of a drug-containing layer, an osmotic push layer, and a coating membrane, the drug-containing layer is composed of 45 wt% of levodopa, 31 wt% of hydroxypropyl cellulose, 16 wt% of mannitol, 5 wt% of povidone K30, 1 wt% of magnesium stearate, 1 wt% of Mint Flavor and 1 wt% of aspartame, wherein the weight percentage is the weight percentage of each component of the drug-containing layer; the osmotic push layer is composed of 55 wt% of sodium carboxymethyl cellulose 7H4XF, 10 wt% of povidone K30, 34 wt% of sorbitol, 0.5 wt% of iron oxide red and 0.5 wt% of magnesium stearate, wherein the weight percentage is the weight percentage of each component of the osmotic push layer; the coating membrane is composed of 70 wt% of cellulose acetate membrane and 30 wt% of copovidone VA64, wherein the weight percentage is the weight percentage of each component of the coating membrane; and the weight of the coating membrane is 4.6% or 7.9% of the weight of the tablet core;
or, the pharmaceutical composition is composed of a drug-containing layer, an osmotic push layer, and a coating membrane, the drug-containing layer is composed of 70 wt% of levodopa, 9 wt% of mannitol, 20 wt% of povidone K30, 1 wt% of magnesium stearate, wherein the weight percentage is the weight percentage of each component of the drug-containing layer; the osmotic push layer is composed of 85 wt% of sodium carboxymethyl cellulose, 3 wt% of povidone K30, 5 wt% of sorbitol, 5 wt% of iron oxide red and 2 wt% of magnesium stearate, wherein the weight percentage is the weight percentage of each component of the osmotic push layer; the coating membrane is composed of 70 wt% of cellulose acetate membrane and 30 wt% of copovidone VA64, wherein the weight percentage is the weight percentage of each component of the coating membrane; and the weight of the coating membrane is 4.5% of the weight of the tablet core;
or, the pharmaceutical composition is composed of a drug-containing layer, an osmotic push layer, a coating membrane and an overcoat, the drug-containing layer is composed of 45 wt% of levodopa, 31 wt% of hydroxypropyl cellulose, 16 wt% of mannitol, 5 wt% of povidone K30, 1 wt% of magnesium stearate, 1 wt% of Mint Flavor and 1 wt% of aspartame, wherein the weight percentage is the weight percentage of each component of the drug-containing layer; the osmotic push layer is composed of 55 wt% of sodium carboxymethyl cellulose 7H4XF, 10 wt% of povidone K30, 34 wt% of sorbitol, 0.5 wt% of iron oxide red and 0.5 wt% of magnesium stearate, wherein the weight percentage is the weight percentage of each component of the osmotic push layer; the coating membrane is composed of 70 wt% of cellulose acetate membrane and 30 wt% of copovidone VA64, wherein the weight percentage is the weight percentage of each component of the coating membrane; and the weight of the coating membrane is 4.8% or 7.7% of the weight of the tablet core, the overcoat is composed of 23.78 wt% of levodopa, 64.22 wt% of carbidopa, 10 wt% of hydroxypropyl cellulose, 1 wt% of aspartame and 1 wt% of Mint Flavor immediate release composition, wherein the weight percentage is the weight percentage of each component of the overcoat, and the weight gain of the overcoat relative to the tablet core is 13.2 % and 12.9 % by weight, respectively;
or, the pharmaceutical composition is composed of a drug-containing layer, an osmotic push layer, a coating membrane and an overcoat, the drug-containing layer is composed of 45 wt% of levodopa, 31 wt% of hydroxypropyl cellulose, 17 wt% of mannitol, 5 wt% of povidone K30, 1 wt% of magnesium stearate and 1 wt% of aspartame, wherein the weight percentage is the weight percentage of each component of the drug-containing layer; the osmotic push layer is composed of 60 wt% of sodium carboxymethyl cellulose 7H4XF, 10 wt% of povidone K30, 26 wt% of sorbitol, 2 wt% of iron oxide red and 2 wt% of magnesium stearate, wherein the weight percentage is the weight percentage of each component of the osmotic push layer; the coating membrane is composed of 70 wt% of cellulose acetate membrane and 30 wt% of copovidone VA64, wherein the weight percentage is the weight percentage of each component of the coating membrane; and the weight of the coating membrane is 4.8% of the weight of the tablet core; the overcoat is composed of 93 wt% of CD, 2 wt% of hydroxypropyl cellulose and 5 wt% of aspartame immediate release composition, wherein the weight percentage is the weight percentage of each component of the overcoat, and the weight gain of the overcoat relative to the tablet core is 13.2 % by weight;
or, the pharmaceutical composition is composed of a drug-containing layer, an osmotic push layer, a coating membrane and an overcoat, the drug-containing layer is composed of 45 wt% of levodopa, 31 wt% of hydroxypropyl cellulose, 12 wt% of mannitol, 5 wt% of povidone K30, 5 wt% of Mint Flavor, 1 wt% of aspartame and 1 wt% of magnesium stearate, wherein the weight percentage is the weight percentage of each component of the drug-containing layer; the osmotic push layer is composed of 40 wt% of sodium carboxymethyl cellulose 7H4XF, 20 wt% of povidone K30, 36 wt% of sorbitol, 3.5 wt% of iron oxide red and 0.5 wt% of magnesium stearate, wherein the weight percentage is the weight percentage of each component of the osmotic push layer; the coating membrane is composed of 70 wt% of cellulose acetate membrane and 30 wt% of copovidone VA64, wherein the weight percentage is the weight percentage of each component of the coating membrane; and the weight of the coating membrane is 4.8% of the weight of the tablet core; the overcoat is composed of 75 wt% of levodopa, 20 wt% of hydroxypropyl cellulose and 5 wt% of Mint Flavor immediate release composition, wherein the weight percentage is the weight percentage of each component of the overcoat, and the weight gain of the overcoat relative to the tablet core is 13.2 % by weight;
or, the pharmaceutical composition is composed of a drug-containing layer, an osmotic push layer, a coating membrane and an overcoat, the drug-containing layer is composed of 62.5 wt% of levodopa, 31 wt% of hydroxypropyl cellulose, 4.5 wt% of mannitol, 0.1 wt% of Mint Flavor, 0.9 wt% of aspartame and 1 wt% of magnesium stearate, wherein the weight percentage is the weight percentage of each component of the drug-containing layer; the osmotic push layer is composed of 55 wt% of sodium carboxymethyl cellulose 7H4XF, 10 wt% of hydroxypropyl cellulose, 34 wt% of sorbitol, 0.5 wt% of iron oxide red and 0.5 wt% of magnesium stearate, wherein the weight percentage is the weight percentage of each component of the osmotic push layer; the coating membrane is composed of 70 wt% of cellulose acetate membrane and 30 wt% of copovidone VA64, wherein the weight percentage is the weight percentage of each component of the coating membrane; and the weight of the coating membrane is 6.5% of the weight of the tablet core, the overcoat is composed of 62.15 wt% of levodopa, 26.85 wt% of carbidopa, 10 wt% of hydroxypropyl cellulose, 0.9 wt% of aspartame and 0.1 wt% of Mint Flavor immediate release composition, wherein the weight percentage is the weight percentage of each component of the overcoat, and the weight gain of the overcoat relative to the tablet core is 21.0 % by weight;
or, the pharmaceutical composition is composed of a drug-containing layer, an osmotic push layer, a coating membrane and an overcoat, the drug-containing layer is composed of 46.9 wt% of levodopa, 31 wt% of hydroxypropyl cellulose, 20.1 wt% of mannitol, 0.1 wt% of Mint Flavor, 0.9 wt% of aspartame and 1 wt% of magnesium stearate, wherein the weight percentage is the weight percentage of each component of the drug-containing layer; the osmotic push layer is composed of 55 wt% of sodium carboxymethyl cellulose 7H4XF, 10 wt% of hydroxypropyl cellulose, 34 wt% of sorbitol, 0.5 wt% of iron oxide red and 0.5 wt% of magnesium stearate, wherein the weight percentage is the weight percentage of each component of the osmotic push layer; the coating membrane is composed of 70 wt% of cellulose acetate membrane and 30 wt% of copovidone VA64, wherein the weight percentage is the weight percentage of each component of the coating membrane; and the weight of the coating membrane is 6.5% of the weight of the tablet core, the overcoat is composed of 62.15 wt% of levodopa, 26.85 wt% of carbidopa, 10 wt% of hydroxypropyl cellulose, 0.9 wt% of aspartame and 0.1 wt% of Mint Flavor immediate release composition, wherein the weight percentage is the weight percentage of each component of the overcoat, and the weight gain of the overcoat relative to the tablet core is 15.7 % by weight;
or, the pharmaceutical composition is composed of a drug-containing layer, an osmotic push layer, a coating membrane and an overcoat; wherein the drug-containing layer is composed of 45 wt% of levodopa, 31 wt% of hydroxypropyl cellulose, 17 wt% of mannitol, 5 wt% of povidone K30, 1 wt% of magnesium stearate and 1 wt% of aspartame, wherein the weight percentage is the weight percentage of each component of the drug-containing layer; the osmotic push layer is composed of 55 wt% of sodium carboxymethyl cellulose 7H4XF, 10 wt% of hydroxypropyl cellulose, 34 wt% of sorbitol, 0.5 wt% of iron oxide red and 0.5 wt% of magnesium stearate, wherein the weight percentage is the weight percentage of each component of the osmotic push layer; the coating membrane is composed of 70 wt% of cellulose acetate membrane and 30 wt% of copovidone VA64, wherein the weight percentage is the weight percentage of each component of the coating membrane; wherein, the cellulose acetate is a cellulose acetate membrane containing 39.8 wt% of acetyl, and the weight of the coating membrane is 5.9 % of the weight of the tablet core; and, the overcoat is composed of 24 wt% of levodopa, 65 wt% of carbidopa, 10 wt% of hydroxypropyl cellulose and 1 wt% of aspartame, wherein the weight percentage is the weight percentage of each component of the overcoat, the weight of the overcoat is 13.1% of the total weight of the tablet core and the coating membrane;
or, the pharmaceutical composition is composed of a drug-containing layer, an osmotic push layer, a coating membrane and an overcoat; the drug-containing layer is composed of 45 wt% of levodopa, 31 wt% of hydroxypropyl cellulose, 22 wt% of mannitol, 0.9 wt% of aspartame, 1 wt% of magnesium stearate and 0.1 wt% of Mint Flavor, wherein the weight percentage is the weight percentage of each component of the drug-containing layer; the osmotic push layer is composed of 55 wt% of sodium carboxymethyl cellulose 7H4XF, 10 wt% of hydroxypropyl cellulose, 34 wt% of sorbitol, 0.5 wt% of iron oxide red and 0.5 wt% of magnesium stearate, wherein the weight percentage is the weight percentage of each component of the osmotic push layer; the coating membrane is composed of 70 wt% of cellulose acetate membrane and 30 wt% of copovidone VA64, wherein the weight percentage is the weight percentage of each component of the coating membrane; wherein, the cellulose acetate is a cellulose acetate membrane containing 39.8 wt% of acetyl, and the weight of the coating membrane is 6.5 % of the weight of the tablet core; and, the overcoat is composed of 54 wt% of levodopa, 35 wt% of carbidopa, 10 wt% of hydroxypropyl cellulose, 0.9 wt% of aspartame and 0.1 wt% of Mint Flavor, wherein the weight percentage is the weight percentage of each component of the overcoat, the weight of the overcoat is 13.1% of the total weight of the tablet core and the coating membrane;
or, the pharmaceutical composition is composed of a drug-containing layer, an osmotic push layer, a coating membrane and an overcoat; the drug-containing layer is composed of 45 wt% of levodopa, 31 wt% of hydroxypropyl cellulose, 22 wt% of mannitol, 0.9 wt% of aspartame, 1 wt% of magnesium stearate and 0.1 wt% of Mint Flavor, wherein the weight percentage is the weight percentage of each component of the drug-containing layer; the osmotic push layer is composed of 55 wt% of sodium carboxymethyl cellulose 7H4XF, 10 wt% of hydroxypropyl cellulose, 34 wt% of sorbitol, 0.5 wt% of iron oxide red and 0.5 wt% of magnesium stearate, wherein the weight percentage is the weight percentage of each component of the osmotic push layer; the coating membrane is composed of 70 wt% of cellulose acetate membrane and 30 wt% of copovidone VA64, wherein the weight percentage is the weight percentage of each component of the coating membrane; wherein, the cellulose acetate is a cellulose acetate membrane containing 39.8 wt% of acetyl, and the weight of the coating membrane is 7.0 % of the weight of the tablet core; and, the overcoat is composed of 42.8 wt% of levodopa, 46.2 wt% of carbidopa, 10 wt% of hydroxypropyl cellulose, 0.9 wt% of aspartame and 0.1 wt% of Mint Flavor, wherein the weight percentage is the weight percentage of each component of the overcoat, the weight of the overcoat is 13.1% of the total weight of the tablet core and the coating membrane;
or, the pharmaceutical composition is composed of a drug-containing layer, an osmotic push layer, a coating membrane and an overcoat; the drug-containing layer is composed of 45 wt% of levodopa, 31 wt% of hydroxypropyl cellulose, 22 wt% of mannitol, 0.9 wt% of aspartame, 1 wt% of magnesium stearate and 0.1 wt% of Mint Flavor, wherein the weight percentage is the weight percentage of each component of the drug-containing layer; the osmotic push layer is composed of 55 wt% of sodium carboxymethyl cellulose 7H4XF, 10 wt% of hydroxypropyl cellulose, 34 wt% of sorbitol, 0.5 wt% of iron oxide red and 0.5 wt% of magnesium stearate, wherein the weight percentage is the weight percentage of each component of the osmotic push layer; the coating membrane is composed of 70 wt% of cellulose acetate membrane and 30 wt% of copovidone VA64, wherein the weight percentage is the weight percentage of each component of the coating membrane; wherein, the cellulose acetate is a cellulose acetate membrane containing 39.8 wt% of acetyl, and the weight of the coating membrane is 9.0 % of the weight of the tablet core; and, the overcoat is composed of 28.2 wt% of levodopa, 60.8 wt% of carbidopa, 10 wt% of hydroxypropyl cellulose, 0.9 wt% of aspartame and 0.1 wt% of Mint Flavor, wherein the weight percentage is the weight percentage of each component of the overcoat, the weight of the overcoat is 13.1% of the total weight of the tablet core and the coating membrane.

16. The extended release dosage form of any one of claims 1-15, wherein the pharmaceutical composition is an osmotic pump extended release drug delivery system, optionally, the osmotic pump extended release drug delivery system is an extended release tablet; wherein, the extended release tablet is a cylinder with a diameter of 5-10 mm and a height of 5-30 mm, or a caplet with a length of 10-25 mm and a width of 5-10 mm; and/or, each of the extended release tablets contains 62.5 mg CD and 500 mg LD, or 62.5 mg CD and 375 mg LD, or 62.5 mg carbidopa and 250 mg levodopa, or 50 mg carbidopa and 500 mg levodopa, or 37.5mg carbidopa and 375mg levodopa.

17. The extended release dosage form of any one of claims 1, 2 or 4 -16 for use in an operation procedure, which comprises the following steps: placing the extended release platform in the personalized retention enabling platform, and fastening the retention enabling platform on the corresponding teeth in the oral cavity; taking out the extended release dosage form after keeping for 4-24 hours, replacing the extended release platform with a new one and fastening the retention enabling platform on the corresponding teeth in the oral cavity again, so that the drug can be released continuously and stably.

## Patentansprüche

1. Dosierungsform mit verlängerter Freisetzung mit einem Absorptionsfenster am oberen Magen-Darm-Trakt, umfassend eine verlängerte Freisetzungsplattform ("extended release platform") und eine Plattform zur Ermöglichung der Retention ("retention enabling platform"),
wobei die verlängerte Freisetzungsplattform eine pharmazeutische Zusammensetzung ist, umfassend einen Tablettenkern und eine Beschichtungsmembran, der Tablettenkern eine arzneimittelhaltige Schicht umfasst und die Beschichtungsmembran Celluloseacetat und Copovidon umfasst, wobei das Gewicht des Celluloseacetats 50-70% des Gewichts der Beschichtungsmembran beträgt, das Gewicht des Copovidons 30-50% des Gewichts der Beschichtungsmembran beträgt;
wobei die Plattform zur Ermöglichung der Retention verwendet wird, um die verlängerte Freisetzungsplattform in der Mundhöhle zu halten, und mindestens ein Ende der Plattform zur Ermöglichung der Retention mit einer Abdeckung verbunden ist, so dass die verlängerte Freisetzungsplattform in der Plattform zur Ermöglichung der Retention behalten wird;
die arzneimittelhaltige Schicht umfasst aktive pharmazeutische Inhaltsstoffe und Hilfsstoffe, und der aktive pharmazeutische Inhaltsstoff einer oder mehrere von Levodopa und Carbidopa ist.

2. Dosierungsform mit verlängerter Freisetzung gemäß Anspruch 1, wobei die Plattform zur Ermöglichung der Retention ein personalisiertes Modul zur Ermöglichung der Retention ("personalized retention enabling module") und ein Modul zur Befestigung von Arzneimitteln ("drug fastened module") umfasst, das Modul zur Befestigung von Arzneimitteln die verlängerte Freisetzungsplattform fixieren kann, und das personalisierte Modul zur Ermöglichung der Retention das Modul zur Befestigung von Arzneimitteln in der Mundhöhle halten kann;
das Modul zur Befestigung von Arzneimitteln ist optional ein oder mehrere Behälter; und/oder, kann das Modul zur Ermöglichung der Retention zu einem oder mehreren Zähnen in der Mundhöhle passen; und/oder, kann das Modul zur Ermöglichung der Retention individuell angepasst werden, um zu bleibenden Zähnen im Ober- oder Unterkiefer zu passen und zu umwickeln, einzuklemmen oder einzusetzen;
wobei der Behälter eine Korb-Struktur aufweist; und/oder, die Form des Querschnitts des Behälters Polygon, kreisförmiger geschlossener Kreislauf ("circular closed-loop") oder kreisförmiger offener Kreislauf ("circular open-loop") oder eine Kombination davon ist; und/oder,
mindestens ein Ende des Behälters mit einer Abdeckung verbunden ist, so dass die verlängerte Freisetzungsplattform in dem Behälter behalten wird; die Abdeckung ist optional ein Streifen; und/oder,
die Plattform zur Ermöglichung der Retention kann zu irgendeinem oder mehreren von den bleibenden Zähnen im Unterkiefer, Unterkiefermolar, zweitem Unterkiefermolar und dessen vorderen und hinteren Backenzähnen in der Mundhöhle passen; und/oder,
die Oberkiefer oder Unterkiefer bleibenden Zähne können ganze bleibende Zähne im Oberkiefer oder ganze bleibende Zähne im Unterkiefer sein, oder der Unterkiefermolar, oder der zweite Unterkiefermolar und dessen angrenzende Teile des ersten Molaren und des zweiten Prämolaren.

3. Herstellungsverfahren für die Dosierungsform mit verlängerter Freisetzung gemäß irgendeinem der Ansprüche 1 oder 2, wobei das Herstellungsverfahren für die Plattform zur Ermöglichung der Retention 3D-Drucken, Spritzgießen oder Abdruckformen ("impression molding") umfasst; die Plattform zur Ermöglichung der Retention wird optional aus einem oder mehreren oral stabilen Materialien hergestellt, und die oral stabilen Materialien umfassen oral stabile Metalle und thermoplastische Elastomere; wobei das oral stabile Metall Dental Titan, Edelstahl, Kobalt-Chrom-Legierung, Kobalt-Chrom-Molybdän-Legierung, Nickel-Chrom-Legierung oder Edelmetall umfasst, und das thermoplastische Elastomer Polycaprolacton, Ethylen-Vinylacetat-Copolymer, Polyethylen hoher Dichte, Polypropylen, Polyacrylat, Polyurethan, Silikonpolymer, Polyester, Poly (Styrol-Ethylen-Buten-Styrol), Poly (StyrolButadien-Styrol), Poly (Styrol-Isopren-Styrol) oder ein Copolymer aus zwei oder mehr der oben genannten Stoffe oder deren physikalische Kombination umfasst.

4. Dosierungsform mit verlängerter Freisetzung gemäß Anspruch 1, wobei in der verlängerten Freisetzungsplattform, in der arzneimittelhaltigen Schicht, der Hilfsstoff einer oder mehrere von Füllstoff, osmotischem Mittel, hydrophilem Polymer, Bindemittel, Schmiermittel, Konservierungsmittel, Aromastoff, Säuerungsmittel und Antioxidationsmittel ist; oder der Hilfsstoff einer oder mehrere von Füllstoff, osmotischem Mittel, hydrophilem Polymer, Bindemittel, Schmiermittel und Konservierungsmittel ist; oder die Hilfsstoffe Füllstoff, osmotisches Mittel, hydrophiles Polymer, Bindemittel, Schmiermittel und Konservierungsmittel sind;
wobei, wenn der pharmazeutisch aktive Inhaltsstoff Levodopa umfasst, der Gewichtsprozentsatz des Levodopa 20-70% beträgt; wenn der aktive Inhaltsstoff Carbidopa umfasst, der Gewichtsprozentsatz von Carbidopa 0-20%, aber nicht 0% beträgt; wobei der Gewichtsprozentsatz der Gewichtsprozentsatz von jeder Komponente der arzneimittelhaltigen Schicht ist;
oder, wenn der pharmazeutisch aktive Inhaltsstoff Levodopa umfasst, der Gewichtsprozentsatz des Levodopa 30-50% beträgt; wenn der aktive Inhaltsstoff Carbidopa umfasst, der Gewichtsprozentsatz von Carbidopa 1-10% beträgt; wobei der Gewichtsprozentsatz der Gewichtsprozentsatz von jeder Komponente der arzneimittelhaltigen Schicht ist.

5. Dosierungsform mit verlängerter Freisetzung gemäß Anspruch 4, wobei in der verlängerten Freisetzungsplattform:
wenn der Hilfsstoff einen Füllstoff umfasst, der Füllstoff einer oder mehrere von mikrokristalliner Cellulose, Hydroxypropylcellulose und Mannitol ist, und der Gewichtsprozentsatz des Füllstoffs 0-50%, aber nicht 0% beträgt;
und/oder, wenn der Hilfsstoff ein osmotisches Mittel umfasst, das osmotische Mittel eines oder mehrere von Magnesiumsulfat, Magnesiumchlorid, Natriumchlorid, Lithiumchlorid, Kaliumsulfat, Natriumsulfat, Mannitol, Harnstoff, Sorbitol, Inositol, Saccharose und Glucose ist, und der Gewichtsprozentsatz des osmotischen Mittels 0-50%, aber nicht 0% beträgt;
und/oder, wenn der Hilfsstoff ein hydrophiles Polymer umfasst, das hydrophile Polymer eines oder mehrere von Hydroxypropylcellulose, Hydroxypropylmethylcellulose, Carboxymethylcellulose, Polyvinylpyrrolidon und Hydroxyethylcellulose ist, und der Gewichtsprozentsatz des hydrophilen Polymers 0-50%, aber nicht 0% beträgt;
und/oder, wenn der Hilfsstoff ein Säuerungsmittel umfasst, das Säuerungsmittel eines oder mehrere von Zitronensäure, Natriumcitrat, Kaliumcitrat, Apfelsäure, Fumarsäure, Milchsäure, Phosphorsäure und Weinsäure ist, und der Gewichtsprozentsatz des Säuerungsmittels 0-10%, aber nicht 0% beträgt;
wobei der Gewichtsprozentsatz der Gewichtsprozentsatz von jeder Komponente der arzneimittelhaltigen Schicht ist.

6. Dosierungsform mit verlängerter Freisetzung gemäß Anspruch 4 oder 5, wobei, in der verlängerten Freisetzungsplattform, der Tablettenkern der pharmazeutischen Zusammensetzung weiter eine osmotische Ausstoßschicht ("osmotic push layer") umfasst, und die osmotische Ausstoßschicht ein hydrophiles Polymer, ein osmotisches Mittel und ein Bindemittel umfasst; die osmotische Ausstoßschicht ist optional ein hydrophiles Polymer, ein osmotisches Mittel ein Bindemittel und ein Schmiermittel umfasst; oder die osmotische Ausstoßschicht ein hydrophiles Polymer, ein osmotisches Mittel, ein Bindemittel, ein Schmiermittel und einen Farbstoff umfasst.

7. Dosierungsform mit verlängerter Freisetzung gemäß Anspruch 6, wobei, in der verlängerten Freisetzungsplattform, das hydrophile Polymer in der osmotischen Ausstoßschicht κ-Carrageenan, Natriumcarboxymethylcellulose oder Polyethylenoxid ist, wobei das Molekulargewicht des hydrophilen Polymers 75.000-7.500.000 beträgt und der Gewichtsprozentsatz des hydrophilen Polymers 25-85% beträgt;
und/oder das osmotische Mittel in der osmotischen Pump-Ausstoßschicht eines oder mehrere von Magnesiumsulfat, Magnesiumchlorid, Natriumchlorid, Lithiumchlorid, Kaliumsulfat, Natriumsulfat, Mannitol, Harnstoff, Sorbitol, Inositol, Saccharose und Glucose ist, und der Gewichtsprozentsatz des osmotischen Mittels 5-65% beträgt;
und/oder, wenn die osmotische Ausstoßschicht ein Bindemittel umfasst, das Bindemittel eines oder mehrere von Methylcellulose, Hydroxypropylmethylcellulose, Hydroxypropylcellulose, Natriumcarboxymethylcellulose, Povidon und Gelatine ist, und der Gewichtsprozentsatz des Bindemittels 3-20% beträgt;
und/oder, wenn die osmotische Ausstoßschicht ein Schmiermittel umfasst, das Schmiermittel eines oder mehrere von Magnesiumstearat, Magnesiumstearatfumarat, Talkum und kolloidales Siliciumdioxid ist, wobei der Gewichtsprozentsatz des Schmiermittels 0-2%, aber nicht 0% beträgt;
und/oder, wenn die osmotische Ausstoßschicht einen Farbstoff umfasst, der Farbstoff einer oder mehrere von Eisenoxidrot, Eisenoxidgelb und Eisenoxidschwarz ist, und der Gewichtsprozentsatz des Farbstoffs 0-5%, aber nicht 0% beträgt;
wobei der Gewichtsprozentsatz der Gewichtsprozentsatz jeder Komponente der osmotische Ausstoßschicht ist.

8. Dosierungsform mit verlängerter Freisetzung gemäß Anspruch 6, wobei, in der verlängerten Freisetzungsplattform, die osmotische Ausstoßschicht Natriumcarboxymethylcellulose, Povidon K30, Sorbitol, Eisenoxidrot und Magnesiumstearat umfasst; oder Natriumcarboxymethylcellulose, Hydroxypropylcellulose, Sorbitol, Eisenoxidrot und Magnesiumstearat umfasst;
die Natriumcarboxymethylcellulose ist optional Natriumcarboxymethylcellulose 7H4XF oder 9H4XF;
und/oder die osmotische Ausstoßschicht 25-85 Gew.-% Natriumcarboxymethylcellulose, 5-65 Gew.-% Sorbitol, 3-20 Gew.-% Povidon, 0-5 Gew.-% Eisenoxidrot und 0,5-2 Gew.-% Magnesiumstearat umfasst;
oder die osmotische Ausstoßschicht 55 Gew.-% Natriumcarboxymethylcellulose, 34,0-39,0 Gew.-% Sorbitol, 3-20 Gew.-% Povidon oder 10 Gew.-% Hydroxylpropylcellulose, 0,5-5 Gew.-% Eisenoxidrot und 0,5-2 Gew.-% Magnesiumstearat umfasst;
oder die osmotische Ausstoßschicht 55 Gew.-% Natriumcarboxymethylcellulose, 39,0 Gew.-% Sorbitol, 5,0 Gew.-% Povidon und 0,5 Gew.-% Eisenoxidrot und 0,5 Gew.-% Magnesiumstearat umfasst; oder 55 Gew.-% Natriumcarboxymethylcellulose, 34,0 Gew.-% Sorbitol, 10,0 Gew.-% Povidon, 0,5 Gew.-% Eisenoxidrot und 0,5 Gew.-% Magnesiumstearat umfasst, oder 55 Gew.-% Natriumcarboxymethylcellulose, 34,0 Gew.-% Sorbitol, 10,0 Gew.-% Hydroxypropylcellulose, 0,5 Gew.-% Eisenoxidrot und 0,5 Gew.-% Magnesiumstearat umfasst; wobei der Gewichtsprozentsatz der Gewichtsprozentsatz jeder Komponente der osmotischen Ausstoßschicht ist.

9. Dosierungsform mit verlängerter Freisetzung gemäß irgendeinem der Ansprüche 5-8, wobei, in der verlängerten Freisetzungsplattform, die Beschichtungsmembran der pharmazeutischen Zusammensetzung weiter mit einem arzneimittelhaltigen, sofort freisetzenden Überzug überzogen ist;
der arzneimittelhaltige, sofort freisetzende Überzug optional einen aktiven pharmazeutischen Inhaltsstoff und einen Hilfsstoff umfasst, der aktive pharmazeutische Inhaltsstoff Levodopa und/oder Carbidopa umfasst, und der Hilfsstoff einer oder mehrere von Hydroxypropylcellulose, Aspartam und Minzgeschmack ist;
wobei, wenn der aktive pharmazeutische Inhaltsstoff Levodopa ist, der Gewichtsprozentsatz des Levodopa 0-75%, aber nicht 0% beträgt, z.B. 23,78-75%; und/oder, wenn der der aktive pharmazeutische Inhaltsstoff Carbidopa ist, der Gewichtsprozentsatz von Carbidopa 0-93%, aber nicht 0% beträgt, z.B. 26,85-93%; und/oder, wenn der Hilfsstoff des Überzugs Hydroxypropylcellulose umfasst, der Gewichtsprozentsatz der Hydroxypropylcellulose 2-20% beträgt, z.B. 10%; und/oder wenn der Hilfsstoff des Überzugs Aspartam umfasst, der Gewichtsprozentsatz des Aspartam 0-5% beträgt, z.B. 0,9-5%; und/oder wenn der Hilfsstoff des Überzugs Minzgeschmack umfasst, der Gewichtsprozentsatz des Minzgeschmacks 0-5% beträgt, z.B. 0,1%; wobei der Gewichtsprozentsatz der Gewichtsprozentsatz jeder Komponente des Überzugs ist.

10. Dosierungsform mit verlängerter Freisetzung gemäß irgendeinem der Ansprüche 4-9, wobei, in der verlängerten Freisetzungsplattform, das Gewicht der Beschichtungsmembran nicht weniger als 2,0%, z.B. 2,0-15,0% oder 4,0-8,0% des Gewichts des Tablettenkerns beträgt; die Beschichtungsmembran weist eine oder mehrere Öffnungen auf, und der Durchmesser der Öffnung ist optional 0,5 mm-1,0 mm, z.B. 0,5 mm, 0,75 mm und 1,0 mm;
in der verlängerten Freisetzungsplattform besteht die pharmazeutische Zusammensetzung optional aus einer arzneimittelhaltigen Schicht und einer Beschichtungsmembran; besteht die pharmazeutische Zusammensetzung aus einer arzneimittelhaltigen Schicht, einer osmotischen Ausstoßschicht und einer Beschichtungsmembran; oder besteht die pharmazeutische Zusammensetzung aus einer arzneimittelhaltigen Schicht, einer osmotischen Ausstoßschicht, einer Beschichtungsmembran und einem Überzug;
und/oder, die arzneimittelhaltige Schicht besteht aus Levodopa, Carbidopa, mikrokristalliner Cellulose, Mannitol, Zitronensäure, Natriumhydroxypropylmethylcellulose und Magnesiumstearat; oder die arzneimittelhaltige Schicht besteht aus Levodopa, mikrokristalliner Cellulose, Hydroxypropylmethylcellulose und Magnesiumstearat; oder die arzneimittelhaltige Schicht besteht aus Levodopa, Carbidopa, Mannitol, Zitronensäure und Magnesiumstearat; oder die arzneimittelhaltige Schicht besteht aus Levodopa, Carbidopa, Hydroxypropylcellulose, Mannitol, Zitronensäure und Magnesiumstearat; oder die arzneimittelhaltige Schicht besteht aus Levodopa, Carbidopa, Hydroxypropylcellulose, Mannitol, Zitronensäure und Povidon K30; oder die arzneimittelhaltige Schicht besteht aus Levodopa, Hydroxypropylcellulose, Mannitol, Povidon K30, Magnesiumstearat, Minzgeschmack und Aspartam; oder die arzneimittelhaltige Schicht besteht aus Levodopa, Mannitol, Povidon K30 und Magnesiumstearat; oder die arzneimittelhaltige Schicht besteht aus Levodopa, Carbidopa, Hydroxypropylcellulose, Mannitol, Aspartam und Magnesiumstearat; oder die arzneimittelhaltige Schicht besteht aus Levodopa, Hydroxypropylcellulose, Mannitol, Magnesiumstearat, Minzgeschmack und Aspartam; oder die arzneimittelhaltige Schicht besteht aus Levodopa, Hydroxypropylcellulose, Mannitol, Povidon K30, Magnesiumstearat und Aspartam; oder die arzneimittelhaltige Schicht besteht aus Levodopa, Hydroxypropylcellulose, Mannitol, Povidon K30, Magnesiumstearat und Aspartam;
und/oder die osmotischen Ausstoßschicht besteht aus Natriumcarboxymethylcellulose, Povidon K30, Sorbitol, Eisenoxidrot und Magnesiumstearat, oder die osmotischen Ausstoßschicht besteht aus Natriumcarboxymethylcellulose, Hydroxypropylcellulose, Sorbitol, Eisenoxidrot und Magnesiumstearat; die Natriumcarboxymethylcellulose ist optional Natriumcarboxymethylcellulose 7H4XF oder 9H4XF;
und/oder der Überzug besteht aus Levodopa, Carbidopa, Hydroxypropylcellulose, Aspartam und Minzgeschmack; oder der Überzug besteht aus Levodopa, Carbidopa, Hydroxypropylcellulose und Aspartam; oder der Überzug besteht aus Carbidopa, Hydroxypropylcellulose und Aspartam; oder der Überzug besteht aus Levodopa, Hydroxypropylcellulose und Minzgeschmack.

11. Dosierungsform mit verlängerter Freisetzung gemäß Anspruch 10, wobei, in der verlängerten Freisetzungsplattform, wenn die arzneimittelhaltige Schicht aus Levodopa, Carbidopa, mikrokristalliner Cellulose, Mannitol, Zitronensäure, Natriumhydroxypropylmethylcellulose und Magnesiumstearat besteht, der Gewichtsprozentsatz von Levodopa 40% beträgt, der Gewichtsprozentsatz von Carbidopa 10,8% beträgt, der Gewichtsprozentsatz von mikrokristalliner Cellulose 20% beträgt, der Gewichtsprozentsatz von Mannitol 18,7% beträgt, der Gewichtsprozentsatz von Zitronensäure 5% beträgt, der Gewichtsprozentsatz von Hydroxypropylmethylcellulose-Natrium 5% beträgt und der Gewichtsprozentsatz von Magnesiumstearat 0,5% beträgt, wobei der Gewichtsprozentsatz der Gewichtsprozentsatz jeder Komponente der arzneimittelhaltigen Schicht ist;
wenn die arzneimittelhaltige Schicht aus Levodopa, mikrokristalliner Cellulose, Hydroxypropylmethylcellulose und Magnesiumstearat besteht, und der Gewichtsprozentsatz des Levodopa 38% beträgt, der Gewichtsprozentsatz von mikrokristalliner Cellulose 50% beträgt, der Gewichtsprozentsatz von Hydroxypropylmethylcellulose 10% beträgt und der Gewichtsprozentsatz von Magnesiumstearat 2% beträgt, wobei der Gewichtsprozentsatz der Gewichtsprozentsatz jeder Komponente der arzneimittelhaltigen Schicht ist;
wenn die arzneimittelhaltige Schicht aus Levodopa, Carbidopa, Mannitol, Zitronensäure und Magnesiumstearat besteht, der Gewichtsprozentsatz des Levodopa 19,5% beträgt und der Gewichtsprozentsatz von Carbidopa 20% beträgt, der Gewichtsprozentsatz von Mannitol 50% beträgt, der Gewichtsprozentsatz von Zitronensäure 10% beträgt und der Gewichtsprozentsatz von Magnesiumstearat 0,5% beträgt, wobei der Gewichtsprozentsatz der Gewichtsprozentsatz jeder Komponente der arzneimittelhaltigen Schicht ist;
wenn die arzneimittelhaltige Schicht aus Levodopa, Carbidopa, Hydroxypropylcellulose, Mannitol, Zitronensäure und Magnesiumstearat besteht, der Gewichtsprozentsatz des Levodopa 40% beträgt, der Gewichtsprozentsatz von Carbidopa 10,8% beträgt, der Gewichtsprozentsatz von Hydroxypropylcellulose 31% beträgt, der Gewichtsprozentsatz von Mannitol 12,7% beträgt, und der Gewichtsprozentsatz von Zitronensäure 5% beträgt, der Gewichtsprozentsatz des Magnesiumstearat 0,5% beträgt, wobei der Gewichtsprozentsatz der Gewichtsprozentsatz jeder Komponente der arzneimittelhaltigen Schicht ist;
wenn die arzneimittelhaltige Schicht aus Levodopa, Carbidopa, Hydroxypropylcellulose, Mannitol, Zitronensäure und Povidon K30 besteht, der Gewichtsprozentsatz von Levodopa 40% beträgt, der Gewichtsprozentsatz von Carbidopa 10,8% beträgt, der Gewichtsprozentsatz von Hydroxypropylcellulose 31% beträgt, der Gewichtsprozentsatz von Mannitol 12,7% beträgt, und der Gewichtsprozentsatz von Zitronensäure 5% beträgt, der Gewichtsprozentsatz von Povidon K30 0,5% beträgt, wobei der Gewichtsprozentsatz der Gewichtsprozentsatz jeder Komponente der arzneimittelhaltigen Schicht ist;
wenn die arzneimittelhaltige Schicht aus Levodopa, Hydroxypropylcellulose, Mannitol, Povidon K30, Magnesiumstearat, Minzgeschmack und Aspartam besteht, der Gewichtsprozentsatz von Levodopa 45% beträgt, der Gewichtsprozentsatz von Hydroxypropylcellulose 31% beträgt, der Gewichtsprozentsatz von Mannitol 16% beträgt, der Gewichtsprozentsatz von Povidon K30 5% beträgt, der Gewichtsprozentsatz des Magnesiumstearats 1% beträgt, der Gewichtsprozentsatz des Minzgeschmacks 1% beträgt und der Gewichtsprozentsatz des Aspartams 1% beträgt, wobei der Gewichtsprozentsatz der Gewichtsprozentsatz jeder Komponente der arzneimittelhaltigen Schicht ist;
wenn die arzneimittelhaltige Schicht aus Levodopa, Mannitol, Povidon K30 und Magnesiumstearat besteht, der Gewichtsprozentsatz von Levodopa 70% beträgt, der Gewichtsprozentsatz von Mannitol 9% beträgt, der Gewichtsprozentsatz von Povidon K30 20% beträgt und der Gewichtsprozentsatz von Magnesiumstearat 1% beträgt, wobei der Gewichtsprozentsatz der Gewichtsprozentsatz jeder Komponente der arzneimittelhaltigen Schicht ist;
wenn die arzneimittelhaltige Schicht aus Levodopa, Carbidopa, Hydroxypropylcellulose, Mannitol, Aspartam und Magnesiumstearat besteht, der Gewichtsprozentsatz von Levodopa 20% beträgt, der Gewichtsprozentsatz von Carbidopa 20% beträgt, der Gewichtsprozentsatz von Hydroxypropylcellulose 50% beträgt, der Gewichtsprozentsatz von Mannitol 4% beträgt und der Gewichtsprozentsatz von Aspartam 5% beträgt, der Gewichtsprozentsatz von Magnesiumstearat 1% beträgt, wobei der Gewichtsprozentsatz der Gewichtsprozentsatz jeder Komponente der arzneimittelhaltigen Schicht ist;
wenn die arzneimittelhaltige Schicht aus Levodopa, Hydroxypropylcellulose, Mannitol, Povidon K30, Magnesiumstearat und Aspartam besteht, der Gewichtsprozentsatz von Levodopa 45% beträgt, der Gewichtsprozentsatz der Hydroxypropylcellulose 31% beträgt, der Gewichtsprozentsatz des Mannitol 16% beträgt, der Gewichtsprozentsatz des Povidon K30 5% beträgt, der Gewichtsprozentsatz von Magnesiumstearat 1% beträgt, der Gewichtsprozentsatz des Minzgeschmacks 1% beträgt und der Gewichtsprozentsatz von Aspartam 1% beträgt, wobei der Gewichtsprozentsatz der Gewichtsprozentsatz jeder Komponente der arzneimittelhaltigen Schicht ist;
wenn die arzneimittelhaltige Schicht aus Levodopa, Hydroxypropylcellulose, Mannitol, Povidon K30, Magnesiumstearat und Aspartam besteht, der Gewichtsprozentsatz von Levodopa 45% beträgt, der Gewichtsprozentsatz der Hydroxypropylcellulose 31% beträgt, der Gewichtsprozentsatz des Mannitol 17% beträgt, der Gewichtsprozentsatz des Povidon K30 5% beträgt, der Gewichtsprozentsatz von Magnesiumstearat 1% beträgt und der Gewichtsprozentsatz von Aspartam 1% beträgt, wobei der Gewichtsprozentsatz der Gewichtsprozentsatz jeder Komponente der arzneimittelhaltigen Schicht ist;
oder, wenn die arzneimittelhaltige Schicht aus Levodopa, Hydroxypropylcellulose, Mannitol, Povidon K30, Magnesiumstearat, Minzgeschmack und Aspartam besteht; der Gewichtsprozentsatz des Levodopa 45% beträgt, der Gewichtsprozentsatz der Hydroxypropylcellulose 31% beträgt, der Gewichtsprozentsatz des Mannitol 12% beträgt und der Gewichtsprozentsatz des Povidon K30 5% beträgt, der Gewichtsprozentsatz des Minzgeschmacks 5% beträgt, der Gewichtsprozentsatz von Aspartam 1% beträgt und der Gewichtsprozentsatz von Magnesiumstearat 1% beträgt, wobei der Gewichtsprozentsatz der Gewichtsprozentsatz jeder Komponente der arzneimittelhaltigen Schicht ist;
oder, wenn die arzneimittelhaltige Schicht aus Levodopa, Hydroxypropylcellulose, Mannitol, Magnesiumstearat, Minzgeschmack und Aspartam besteht, der Gewichtsprozentsatz des Levodopa 62,5% beträgt, der Gewichtsprozentsatz der Hydroxypropylcellulose 31% beträgt, der Gewichtsprozentsatz des Mannitol 4,5% beträgt, der Gewichtsprozentsatz des Minzgeschmacks 0,1% beträgt, der Gewichtsprozentsatz von Aspartam 0,9% beträgt und der Gewichtsprozentsatz von Magnesiumstearat 1% beträgt, wobei der Gewichtsprozentsatz der Gewichtsprozentsatz jeder Komponente der arzneimittelhaltigen Schicht ist;
oder, wenn die arzneimittelhaltige Schicht aus Levodopa, Hydroxypropylcellulose, Mannitol, Magnesiumstearat, Minzgeschmack und Aspartam besteht, der Gewichtsprozentsatz des Levodopa 46,9% beträgt, der Gewichtsprozentsatz der Hydroxypropylcellulose 31% beträgt, der Gewichtsprozentsatz des Mannitol 20,1% beträgt, der Gewichtsprozentsatz des Minzgeschmacks 0,1% beträgt, der Gewichtsprozentsatz von Aspartam 0,9% beträgt, der Gewichtsprozentsatz von Magnesiumstearat 1% beträgt, wobei der Gewichtsprozentsatz der Gewichtsprozentsatz jeder Komponente der arzneimittelhaltigen Schicht ist.

12. Dosierungsform mit verlängerter Freisetzung gemäß Anspruch 10, wobei, in der verlängerten Freisetzungsplattform, die Beschichtungsmembran aus 50 Gew.-% Celluloseacetatmembran und 50 Gew.-% Copovidon VA64 besteht; die Beschichtungsmembran aus 70 Gew.-% Celluloseacetatmembran und 30 Gew.-% Copovidon VA64 besteht; die Beschichtungsmembran aus 60 Gew.-% Celluloseacetatmembran und 40 Gew.-% Copovidon VA64 besteht; wobei der Gewichtsprozentsatz der Gewichtsprozentsatz jeder Komponente der Beschichtungsmembran ist;
das Gewicht der Beschichtungsmembran beträgt optional 2,0%, 4,2%, 4,5%, 4,6%, 4,8%, 5,0%, 5,9%, 6,5%, 6,7%, 7,0%, 7,7%, 7,9% oder 9,7% des Gewichts des Tablettenkerns.

13. Dosierungsform mit verlängerter Freisetzung gemäß irgendeinem der Ansprüche 10-12, wobei, in der verlängerten Freisetzungsplattform,
wenn die osmotischen Ausstoßschicht aus Natriumcarboxymethylcellulose 7H4XF, Povidon K30, Sorbitol, Eisenoxidrot und Magnesiumstearat besteht,
der Gewichtsprozentsatz der Carboxymethylcellulose-Natrium 7H4XF 55% beträgt, der Gewichtsprozentsatz des Povidon K30 5% beträgt, der Gewichtsprozentsatz des Sorbitol 39% beträgt, der Gewichtsprozentsatz des Eisenoxidrot 0,5% beträgt und der Gewichtsprozentsatz des Magnesiumstearats 0,5% beträgt, wobei der Gewichtsprozentsatz der Gewichtsprozentsatz jeder Komponente der osmotischen Ausstoßschicht ist;
der Gewichtsprozentsatz der Carboxymethylcellulose-Natrium 7H4XF 55% beträgt, der Gewichtsprozentsatz des Povidon K30 10% beträgt, der Gewichtsprozentsatz des Sorbitol 34% beträgt, der Gewichtsprozentsatz des Eisenoxidrot 0,5% beträgt und der Gewichtsprozentsatz des Magnesiumstearats 0,5% beträgt, wobei der Gewichtsprozentsatz der Gewichtsprozentsatz jeder Komponente der osmotischen Ausstoßschicht ist;
der Gewichtsprozentsatz der Natriumcarboxymethylcellulose 85% beträgt, der Gewichtsprozentsatz des Povidon K30 3% beträgt, der Gewichtsprozentsatz des Sorbitol 5% beträgt, der Gewichtsprozentsatz des Eisenoxidrot 5% beträgt und der Gewichtsprozentsatz des Magnesiumstearats 2% beträgt, wobei der Gewichtsprozentsatz der Gewichtsprozentsatz jeder Komponente der osmotischen Ausstoßschicht ist;
der Gewichtsprozentsatz der Natriumcarboxymethylcellulose 25% beträgt, der Gewichtsprozentsatz des Povidon K30 9,5% beträgt, der Gewichtsprozentsatz des Sorbitol 65% beträgt und der Gewichtsprozentsatz des Magnesiumstearat 0,5% beträgt, wobei der Gewichtsprozentsatz der Gewichtsprozentsatz jeder Komponente der osmotischen Ausstoßschicht ist;
der Gewichtsprozentsatz der Carboxymethylcellulose-Natrium 7H4XF 60% beträgt, der Gewichtsprozentsatz des Povidon K30 10% beträgt, der Gewichtsprozentsatz des Sorbitol 26% beträgt, der Gewichtsprozentsatz des Eisenoxidrot 2% beträgt und der Gewichtsprozentsatz des Magnesiumstearat 2% beträgt, wobei der Gewichtsprozentsatz der Gewichtsprozentsatz jeder Komponente der osmotischen Ausstoßschicht ist;
der Gewichtsprozentsatz der Carboxymethylcellulose-Natrium 7H4XF 40% beträgt, der Gewichtsprozentsatz des Povidon K30 20% beträgt, der Gewichtsprozentsatz des Sorbitol 36% beträgt, der Gewichtsprozentsatz des Eisenoxidrot 3,5% beträgt und der Gewichtsprozentsatz des Magnesiumstearat 0,5% beträgt, wobei der Gewichtsprozentsatz der Gewichtsprozentsatz jeder Komponente der osmotischen Ausstoßschicht ist;
oder, wenn die osmotische Ausstoßschicht aus Natriumcarboxymethylcellulose 9H4XF, Povidon K30, Sorbitol, Eisenoxidrot und Magnesiumstearat besteht, der Gewichtsprozentsatz von Natriumcarboxymethylcellulose 9H4XF 55% beträgt, der Gewichtsprozentsatz von Povidon K30 5% beträgt, der Gewichtsprozentsatz von Sorbitol 39% beträgt und der Gewichtsprozentsatz von Eisenoxidrot 0,5% beträgt und der Gewichtsprozentsatz von Magnesiumstearat 0,5% beträgt, wobei der Gewichtsprozentsatz der Gewichtsprozentsatz jeder Komponente der osmotischen Ausstoßschicht ist;
oder, wenn die osmotische Ausstoßschicht aus Natriumcarboxymethylcellulose 7H4XF, Hydroxypropylcellulose, Sorbitol, Eisenoxidrot und Magnesiumstearat besteht, der Gewichtsprozentsatz von Natriumcarboxymethylcellulose 7H4XF 55% beträgt, der Gewichtsprozentsatz von Hydroxypropylcellulose 10% beträgt, der Gewichtsprozentsatz von Sorbitol 34% beträgt, der Gewichtsprozentsatz des Eisenoxidrot 0,5% beträgt und der Gewichtsprozentsatz des Magnesiumstearat 0,5% beträgt, wobei der Gewichtsprozentsatz der Gewichtsprozentsatz jeder Komponente der osmotischen Ausstoßschicht ist.

14. Dosierungsform mit verlängerter Freisetzung gemäß irgendeinem der Ansprüche 10-13, wobei, in der verlängerten Freisetzungsplattform, wenn der Überzug aus Levodopa, Carbidopa, Hydroxypropylcellulose, Aspartam und Minzgeschmack besteht, der Gewichtsprozentsatz des Levodopa 23,78% beträgt, der Gewichtsprozentsatz des Carbidopa 64,22% beträgt, der Gewichtsprozentsatz der Hydroxypropylcellulose 10% beträgt und der Gewichtsprozentsatz des Aspartam 1% beträgt, der Gewichtsprozentsatz des Minzgeschmacks 1% beträgt; oder der Gewichtsprozentsatz des Levodopa 54% beträgt, der Gewichtsprozentsatz des Carbidopa 35% beträgt, der Gewichtsprozentsatz der Hydroxypropylcellulose 10% beträgt und der Gewichtsprozentsatz des Aspartam 0,9% beträgt, der Gewichtsprozentsatz des Minzgeschmacks 0,1% beträgt; der Gewichtsprozentsatz des Levodopa 42,8% beträgt, der Gewichtsprozentsatz des Carbidopa 46,2% beträgt, der Gewichtsprozentsatz der Hydroxypropylcellulose 10% beträgt und der Gewichtsprozentsatz des Aspartam 0,9% beträgt und der Gewichtsprozentsatz des Minzgeschmacks 0,1% beträgt, wobei der Gewichtsprozentsatz der Gewichtsprozentsatz jeder Komponente des Überzugs ist;
wenn der Überzug aus Levodopa, Carbidopa, Hydroxypropylcellulose, Aspartam und Minzgeschmack besteht, der Gewichtsprozentsatz des Levodopa 62,15% beträgt, der Gewichtsprozentsatz des Carbidopa 26,85% beträgt, der Gewichtsprozentsatz der Hydroxypropylcellulose 10% beträgt, der Gewichtsprozentsatz des Aspartam 0,9% beträgt und der Gewichtsprozentsatz des Minzgeschmacks 0,1% beträgt;
oder, der Gewichtsprozentsatz des Levodopa 42,8% beträgt, der Gewichtsprozentsatz des Carbidopa 46,2% beträgt, der Gewichtsprozentsatz der Hydroxypropylcellulose 10% beträgt, der Gewichtsprozentsatz des Aspartam 0,9% beträgt, der Gewichtsprozentsatz des Minzgeschmacks 0,1% beträgt, oder der Gewichtsprozentsatz des Levodopa 28,2% beträgt und der Gewichtsprozentsatz des Carbidopa 60,8% beträgt, der Gewichtsprozentsatz der Hydroxypropylcellulose 10% beträgt, der Gewichtsprozentsatz des Aspartam 0,9% beträgt und der Gewichtsprozentsatz des Minzgeschmacks 0,1% beträgt, wobei der Gewichtsprozentsatz der Gewichtsprozentsatz jeder Komponente des Überzugs ist;
oder, wenn der Überzug aus Carbidopa, Hydroxypropylcellulose und Aspartam besteht, der Gewichtsprozentsatz von Carbidopa 93% beträgt, der Gewichtsprozentsatz von Hydroxypropylcellulose 2% beträgt und der Gewichtsprozentsatz von Aspartam 5% beträgt, wobei der Gewichtsprozentsatz der Gewichtsprozentsatz jeder Komponente des Überzugs ist;
oder, wenn der Überzug aus Levodopa, Hydroxypropylcellulose und Minzgeschmack besteht, der Gewichtsprozentsatz des Levodopa 75% beträgt, der Gewichtsprozentsatz der Hydroxypropylcellulose 20% beträgt und der Gewichtsprozentsatz des Minzgeschmacks 5% beträgt, wobei der Gewichtsprozentsatz der Gewichtsprozentsatz jeder Komponente des Überzugs ist;
oder, wenn der Überzug aus Levodopa, Carbidopa, Hydroxypropylcellulose und Aspartam besteht, der Gewichtsprozentsatz von Levodopa 24% beträgt, der Gewichtsprozentsatz von Carbidopa 65% beträgt, der Gewichtsprozentsatz von Hydroxypropylcellulose 10% beträgt und der Gewichtsprozentsatz von Aspartam 1% beträgt, wobei der Gewichtsprozentsatz der Gewichtsprozentsatz jeder Komponente des Überzugs ist;
die Gewichtszunahme des Überzugs beträgt optional im Verhältnis zum Tablettenkern 12,9% oder 13,2% oder 15,7% oder 21,0% nach Gewicht.

15. Dosierungsform mit verlängerter Freisetzung gemäß irgendeinem der Ansprüche 10-14, wobei, in der verlängerten Freisetzungsplattform, die pharmazeutische Zusammensetzung aus einer arzneimittelhaltigen Schicht und einer Beschichtungsmembran besteht, die arzneimittelhaltigen Schicht aus 40 Gew.-% Levodopa, 10,8 Gew.-% Carbidopa, 20 Gew.-% mikrokristalliner Cellulose, 18,7 Gew.-% Mannitol, 5 Gew.-% Zitronensäure, 5 Gew.-% Hydroxypropylmethylcellulose-Natrium und 0,5 Gew.-% Magnesiumstearat besteht, wobei der Gewichtsprozentsatz der Gewichtsprozentsatz jeder Komponente der arzneimittelhaltigen Schicht ist; die Beschichtungsmembran aus 50 Gew.-% Celluloseacetatmembran und 50 Gew.-% Copovidon VA64 besteht, wobei der Gewichtsprozentsatz der Gewichtsprozentsatz jeder Komponente der Beschichtungsmembran ist; das Gewicht der Beschichtungsmembran beträgt 2,0% des Gewichts des Tablettenkerns;
oder, die pharmazeutische Zusammensetzung aus einer arzneimittelhaltigen Schicht und einer Beschichtungsmembran besteht, die arzneimittelhaltige Schicht aus 38 Gew.-% Levodopa, 50 Gew.-% mikrokristalliner Cellulose, 10 Gew.-% Hydroxypropylmethylcellulose und 2 Gew.-% Magnesiumstearat besteht, wobei der Gewichtsprozentsatz der Gewichtsprozentsatz jeder Komponente der arzneimittelhaltigen Schicht ist; die Beschichtungsmembran aus 50 Gew.-% Celluloseacetatmembran und 50 Gew.-% Copovidon VA64, wobei der Gewichtsprozentsatz der Gewichtsprozentsatz jeder Komponente der Beschichtungsmembran ist, und das Gewicht der Beschichtungsmembran 4,5% des Gewichts des Tablettenkerns beträgt;
oder, die pharmazeutische Zusammensetzung aus einer arzneimittelhaltigen Schicht und einer Beschichtungsmembran besteht, die arzneimittelhaltige Schicht 19,5 Gew.-% Levodopa, 20 Gew.-% Carbidopa, 50 Gew.-% Mannitol, 10 Gew.-% Zitronensäure und 0,5 Gew.-% Magnesiumstearat umfasst, wobei der Gewichtsprozentsatz der Gewichtsprozentsatz jeder Komponente der arzneimittelhaltigen Schicht ist; die Beschichtungsmembran aus 50 Gew.-% Celluloseacetatmembran und 50 Gew.-% Copovidon VA64 besteht, wobei der Gewichtsprozentsatz der Gewichtsprozentsatz jeder Komponente der Beschichtungsmembran ist und das Gewicht der Beschichtungsmembran 4,5% des Gewichts des Tablettenkerns beträgt;
oder, die pharmazeutische Zusammensetzung aus einer arzneimittelhaltigen Schicht, einer osmotischen Ausstoßschicht und einer Beschichtungsmembran besteht, und die arzneimittelhaltige Schicht aus 40 Gew.-% Levodopa, 10,8 Gew.-% Carbidopa, 31 Gew.-% Hydroxypropylcellulose, 12,7 Gew.-% Mannitol, 5 Gew.-% Zitronensäure und 0,5 Gew.-% Magnesiumstearat besteht, wobei der Gewichtsprozentsatz der Gewichtsprozentsatz jeder Komponente der arzneimittelhaltigen Schicht ist; die osmotischen Ausstoßschicht aus 55 Gew.-% Natriumcarboxymethylcellulose 7H4XF oder 9H4XF, 5 Gew.-% Povidon K30, 39 Gew.-% Sorbitol, 0,5 Gew.-% Eisenoxidrot und 0,5 Gew.-% Magnesiumstearat besteht, wobei der Gewichtsprozentsatz der Gewichtsprozentsatz jeder Komponente der osmotischen Ausstoßschicht ist; die Beschichtungsmembran aus 70 Gew.-% Celluloseacetatmembran und 30 Gew.-% Copovidon VA64 besteht, wobei der Gewichtsprozentsatz der Gewichtsprozentsatz jeder Komponente der Beschichtungsmembran ist; das Gewicht der Beschichtungsmembran beträgt 2,0%, 4,0% oder 5,0% des Gewichts des Tablettenkerns;
oder, die pharmazeutische Zusammensetzung aus einer arzneimittelhaltigen Schicht, einer osmotischen Ausstoßschicht und einer Beschichtungsmembran besteht, die arzneimittelhaltige Schicht aus 40 Gew.-% Levodopa, 10,8 Gew.-% Carbidopa, 31 Gew.-% Hydroxypropylcellulose, 12,7 Gew.-% Mannitol, 5 Gew.-% Zitronensäure und 0,5 Gew.-% Povidon K30 besteht, wobei der Gewichtsprozentsatz der Gewichtsprozentsatz jeder Komponente der arzneimittelhaltigen Schicht ist; die osmotische Ausstoßschicht aus 55 Gew.-% Natriumcarboxymethylcellulose 7H4XF, 5 Gew.-% Povidon K30, 39 Gew.-% Sorbitol, 0,5 Gew.-% Eisenoxidrot und 0,5 Gew.-% Magnesiumstearat besteht, wobei der Gewichtsprozentsatz der Gewichtsprozentsatz jeder Komponente der osmotischen Ausstoßschicht ist; die Beschichtungsmembran aus 60 Gew.-% Celluloseacetatmembran und 40% Copovidon VA64 besteht, wobei der Gewichtsprozentsatz der Gewichtsprozentsatz jeder Komponente der Beschichtungsmembran ist; und das Gewicht der Beschichtungsmembran 5,0 % des Gewichts des Tablettenkerns beträgt;
oder, die pharmazeutische Zusammensetzung aus einer arzneimittelhaltigen Schicht, einer osmotischen Ausstoßschicht und einer Beschichtungsmembran besteht, die arzneimittelhaltige Schicht aus 45 Gew.-% Levodopa, 31 Gew.-% Hydroxypropylcellulose, 16 Gew.-% Mannitol, 5 Gew.-% Povidon K30 und 1 Gew.-% Magnesiumstearat, 1 Gew.-% Minzgeschmack und 1 Gew.-% Aspartam besteht, wobei der Gewichtsprozentsatz der Gewichtsprozentsatz jeder Komponente der arzneimittelhaltigen Schicht ist; die osmotische Ausstoßschicht aus 55 Gew.-% Natriumcarboxymethylcellulose 7H4XF, 10 Gew.-% Povidon K30, 34 Gew.-% Sorbitol, 0,5 Gew.-% Eisenoxidrot und 0,5 Gew.-% Magnesiumstearat besteht, wobei der Gewichtsprozentsatz der Gewichtsprozentsatz jeder Komponente der osmotischen Ausstoßschicht ist; die Beschichtungsmembran aus 60 Gew.-% Celluloseacetatmembran und 40 Gew.-% Copovidon VA64 besteht, wobei der Gewichtsprozentsatz der Gewichtsprozentsatz jeder Komponente der Beschichtungsmembran ist; und das Gewicht der Beschichtungsmembran 4,2%, 6,7% oder 9,7% des Gewichts des Tablettenkerns beträgt;
oder, die pharmazeutische Zusammensetzung besteht aus einer arzneimittelhaltigen Schicht, einer osmotischen Ausstoßschicht und einer Beschichtungsmembran, die arzneimittelhaltige Schicht aus 45 Gew.-% Levodopa, 31 Gew.-% Hydroxypropylcellulose, 16 Gew.-% Mannitol, 5 Gew.-% Povidon K30, 1 Gew.-% Magnesiumstearat, 1 Gew.-% Minzgeschmack und 1 Gew.-% Aspartam besteht, wobei der Gewichtsprozentsatz der Gewichtsprozentsatz jeder Komponente der arzneimittelhaltigen Schicht ist; die osmotischen Ausstoßschicht aus 55 Gew.-% Natriumcarboxymethylcellulose 7H4XF, 10 Gew.-% Povidon K30, 34 Gew.-% Sorbitol, 0,5 Gew.-% Eisenoxidrot und 0,5 Gew.-% Magnesiumstearat besteht, wobei der Gewichtsprozentsatz der Gewichtsprozentsatz jeder Komponente der osmotischen Ausstoßschicht ist; die Beschichtungsmembran aus 70 Gew.-% Celluloseacetatmembran und 30 Gew.-% Copovidon VA64 besteht, wobei der Gewichtsprozentsatz der Gewichtsprozentsatz jeder Komponente der Beschichtungsmembran ist; und das Gewicht der Beschichtungsmembran 4,6% oder 7,9% des Gewichts des Tablettenkerns beträgt;
oder, die pharmazeutische Zusammensetzung aus einer arzneimittelhaltigen Schicht, einer osmotischen Ausstoßschicht und einer Beschichtungsmembran besteht, die arzneimittelhaltige Schicht aus 70 Gew.-% Levodopa, 9 Gew.-% Mannitol, 20 Gew.-% Povidon K30, 1 Gew.-% Magnesiumstearat besteht, wobei der Gewichtsprozentsatz der Gewichtsprozentsatz jeder Komponente der arzneimittelhaltigen Schicht ist; die osmotische Ausstoßschicht besteht aus 85 Gew.-% Natriumcarboxymethylcellulose, 3 Gew.-% Povidon K30, 5 Gew.-% Sorbitol, 5 Gew.-% Eisenoxidrot und 2 Gew.-% Magnesiumstearat, wobei der Gewichtsprozentsatz der Gewichtsprozentsatz jeder Komponente der osmotischen Ausstoßschicht ist; die Beschichtungsmembran aus 70 Gew.-% Celluloseacetatmembran und 30 Gew.-% Copovidon VA64 besteht, wobei der Gewichtsprozentsatz der Gewichtsprozentsatz jeder Komponente der Beschichtungsmembran ist; und das Gewicht der Beschichtungsmembran 4,5% des Gewichts des Tablettenkerns beträgt;
oder, die pharmazeutische Zusammensetzung aus einer arzneimittelhaltigen Schicht, einer osmotischen Ausstoßschicht, einer Beschichtungsmembran und einem Überzug besteht, die arzneimittelhaltige Schicht aus 45 Gew.-% Levodopa, 31 Gew.-% Hydroxypropylcellulose, 16 Gew.-% Mannitol, 5 Gew.-% Povidon K30, 1 Gew.-% Magnesiumstearat, 1 Gew.-% Minzgeschmack und 1 Gew.-% Aspartam besteht, wobei der Gewichtsprozentsatz der Gewichtsprozentsatz jeder Komponente der arzneimittelhaltigen Schicht ist; die osmotischen Ausstoßschicht aus 55 Gew.-% Natriumcarboxymethylcellulose 7H4XF, 10 Gew.-% Povidon K30, 34 Gew.-% Sorbitol, 0,5 Gew.-% Eisenoxidrot und 0,5 Gew.-% Magnesiumstearat besteht, wobei der Gewichtsprozentsatz der Gewichtsprozentsatz jeder Komponente der osmotischen Ausstoßschicht ist; die Beschichtungsmembran aus 70 Gew.-% Celluloseacetatmembran und 30 Gew.-% Copovidon VA64 besteht, wobei der Gewichtsprozentsatz der Gewichtsprozentsatz jeder Komponente der Beschichtungsmembran ist; und das Gewicht der Beschichtungsmembran 4,8% oder 7,7% des Gewichts des Tablettenkerns beträgt, der Überzug aus 23,78 Gew.-% Levodopa, 64,22 Gew.-% Carbidopa, 10 Gew.-% Hydroxypropylcellulose, 1 Gew.-% Aspartam und 1 Gew.-% sofort freisetzende Minzgeschmackszusammensetzung besteht, wobei der Gewichtsprozentsatz der Gewichtsprozentsatz jeder Komponente des Überzugs ist und die Gewichtszunahme des Überzugs relativ zum Tablettenkern jeweils 13,2% bzw. 12,9% nach Gewicht beträgt;
oder, die pharmazeutische Zusammensetzung aus einer arzneimittelhaltigen Schicht, einer osmotischen Ausstoßschicht, einer Beschichtungsmembran und einem Überzug besteht, die arzneimittelhaltige Schicht aus 45 Gew.-% Levodopa, 31 Gew.-% Hydroxypropylcellulose, 17 Gew.-% Mannitol, 5 Gew.-% Povidon K30, 1 Gew.-% Magnesiumstearat und 1 Gew.-% Aspartam besteht, wobei der Gewichtsprozentsatz der Gewichtsprozentsatz jeder Komponente der arzneimittelhaltigen Schicht ist; die osmotische Ausstoßschicht aus 60 Gew.-% Natriumcarboxymethylcellulose 7H4XF, 10 Gew.-% Povidon K30, 26 Gew.-% Sorbitol, 2 Gew.-% Eisenoxidrot und 2 Gew.-% Magnesiumstearat besteht, wobei der Gewichtsprozentsatz der Gewichtsprozentsatz jeder Komponente der osmotischen Ausstoßschicht ist; die Beschichtungsmembran aus 70 Gew.-% Celluloseacetatmembran und 30 Gew.-% Copovidon VA64 besteht, wobei der Gewichtsprozentsatz der Gewichtsprozentsatz jeder Komponente der Beschichtungsmembran ist; und das Gewicht der Beschichtungsmembran 4,8% des Gewichts des Tablettenkerns beträgt; der Überzug aus 93 Gew.-% CD, 2 Gew.-% Hydroxypropylcellulose und 5 Gew.-% sofort freisetzende Aspartamzusammensetzung besteht, wobei der Gewichtsprozentsatz der Gewichtsprozentsatz jeder Komponente des Überzugs ist und die Gewichtszunahme des Überzugs relativ zum Tablettenkern 13,2 Gew.-% beträgt;
oder, die pharmazeutische Zusammensetzung aus einer arzneimittelhaltigen Schicht, einer osmotischen Ausstoßschicht, einer Beschichtungsmembran und einem Überzug besteht, die arzneimittelhaltige Schicht aus 45 Gew.-% Levodopa, 31 Gew.-% Hydroxypropylcellulose, 12 Gew.-% Mannitol, 5 Gew.-% Povidon K30, 5 Gew.-% Minzgeschmack, 1 Gew.-% Aspartam und 1 Gew.-% Magnesiumstearat besteht, wobei der Gewichtsprozentsatz der Gewichtsprozentsatz jeder Komponente der arzneimittelhaltigen Schicht ist; die osmotische Ausstoßschicht aus 40 Gew.-% Natriumcarboxymethylcellulose 7H4XF, 20 Gew.-% Povidon K30, 36 Gew.-% Sorbitol, 3,5 Gew.-% Eisenoxidrot und 0,5 Gew.-% Magnesiumstearat besteht, wobei der Gewichtsprozentsatz der Gewichtsprozentsatz jeder Komponente der osmotischen Ausstoßschicht ist; die Beschichtungsmembran aus 70 Gew.-% Celluloseacetatmembran und 30 Gew.-% Copovidon VA64 besteht, wobei der Gewichtsprozentsatz der Gewichtsprozentsatz jeder Komponente der Beschichtungsmembran ist; und das Gewicht der Beschichtungsmembran 4,8% des Gewichts des Tablettenkerns beträgt; der Überzug aus 75 Gew.-% Levodopa, 20 Gew.-% Hydroxypropylcellulose und 5 Gew.-% sofort freisetzende Minzgeschmackszusammensetzung besteht, wobei der Gewichtsprozentsatz der Gewichtsprozentsatz jeder Komponente des Überzugs ist und die Gewichtszunahme des Überzugs relativ zum Tablettenkern 13,2 Gew.-% beträgt;
oder, die pharmazeutische Zusammensetzung aus einer arzneimittelhaltigen Schicht, einer osmotischen Ausstoßschicht, einer Beschichtungsmembran und einem Überzug besteht, die arzneimittelhaltige Schicht aus 62,5 Gew.-% Levodopa, 31 Gew.-% Hydroxypropylcellulose, 4,5 Gew.-% Mannitol, 0,1 Gew.-% Minzgeschmack, 0,9 Gew.- % Aspartam und 1 Gew.-% Magnesiumstearat besteht, wobei der Gewichtsprozentsatz der Gewichtsprozentsatz jeder Komponente der arzneimittelhaltigen Schicht ist; die osmotischen Ausstoßschicht aus 55 Gew.-% Natriumcarboxymethylcellulose 7H4XF, 10 Gew.-% Hydroxypropylcellulose, 34 Gew.-% Sorbitol, 0,5 Gew.-% Eisenoxidrot und 0,5 Gew.-% Magnesiumstearat besteht, wobei der Gewichtsprozentsatz der Gewichtsprozentsatz jeder Komponente der osmotischen Ausstoßschicht ist; die Beschichtungsmembran aus 70 Gew.-% Celluloseacetatmembran und 30 Gew.-% Copovidon VA64 besteht, wobei der Gewichtsprozentsatz der Gewichtsprozentsatz jeder Komponente der Beschichtungsmembran ist; und das Gewicht der Beschichtungsmembran 6,5% des Gewichts des Tablettenkerns beträgt, der Überzug aus 62,15 Gew.-% Levodopa, 26,85 Gew.-% Carbidopa, 10 Gew.-% Hydroxypropylcellulose, 0,9 Gew.-% Aspartam und 0,1 Gew.-% sofort freisetzende Minzgeschmackszusammensetzung besteht, wobei der Gewichtsprozentsatz der Gewichtsprozentsatz jeder Komponente des Überzugs ist und die Gewichtszunahme des Überzugs relativ zum Tablettenkern 21,0 Gew.-% beträgt;
oder, die pharmazeutische Zusammensetzung aus einer arzneimittelhaltigen Schicht, einer osmotischen Ausstoßschicht, einer Beschichtungsmembran und einem Überzug besteht, die arzneimittelhaltige Schicht aus 46,9 Gew.-% Levodopa, 31 Gew.-% Hydroxypropylcellulose, 20,1 Gew.-% Mannitol, 0,1 Gew.-% Minzgeschmack, 0,9 Gew.-% Aspartam und 1 Gew.-% Magnesiumstearat besteht, wobei der Gewichtsprozentsatz der Gewichtsprozentsatz jeder Komponente der arzneimittelhaltigen Schicht ist; die osmotischen Ausstoßschicht aus 55 Gew.-% Natriumcarboxymethylcellulose 7H4XF, 10 Gew.-% Hydroxypropylcellulose, 34 Gew.-% Sorbitol, 0,5 Gew.-% Eisenoxidrot und 0,5 Gew.-% Magnesiumstearat besteht, wobei der Gewichtsprozentsatz der Gewichtsprozentsatz jeder Komponente der osmotischen Ausstoßschicht ist; die Beschichtungsmembran aus 70 Gew.-% Celluloseacetatmembran und 30 Gew.-% Copovidon VA64 besteht, wobei der Gewichtsprozentsatz der Gewichtsprozentsatz jeder Komponente der Beschichtungsmembran ist; und das Gewicht der Beschichtungsmembran 6,5% des Gewichts des Tablettenkerns beträgt, der Überzug aus 62,15 Gew.-% Levodopa, 26,85 Gew.-% Carbidopa, 10 Gew.-% Hydroxypropylcellulose, 0,9 Gew.-% Aspartam und 0,1 Gew.-% sofort freisetzende Minzgeschmackszusammensetzung besteht, wobei der Gewichtsprozentsatz der Gewichtsprozentsatz jeder Komponente des Überzugs ist und die Gewichtszunahme des Überzugs relativ zum Tablettenkern 15,7 Gew.-% beträgt,
oder, die pharmazeutische Zusammensetzung aus einer arzneimittelhaltigen Schicht, einer osmotischen Ausstoßschicht, einer Beschichtungsmembran und einem Überzug besteht; wobei die arzneimittelhaltige Schicht aus 45 Gew.-% Levodopa, 31 Gew.-% Hydroxypropylcellulose, 17 Gew.-% Mannitol, 5 Gew.-% Povidon K30, 1 Gew.-% Magnesiumstearat und 1 Gew.-% Aspartam besteht, wobei der Gewichtsprozentsatz der Gewichtsprozentsatz jeder Komponente der arzneimittelhaltigen Schicht ist; die osmotische Ausstoßschicht aus 55 Gew.-% Natriumcarboxymethylcellulose 7H4XF, 10 Gew.-% Hydroxypropylcellulose, 34 Gew.-% Sorbitol, 0,5 Gew.-% Eisenoxidrot und 0,5 Gew.-% Magnesiumstearat besteht, wobei der Gewichtsprozentsatz der Gewichtsprozentsatz jeder Komponente der osmotischen Ausstoßschicht ist; die Beschichtungsmembran aus 70 Gew.-% Celluloseacetatmembran und 30 Gew.-% Copovidon VA64 besteht, wobei der Gewichtsprozentsatz der Gewichtsprozentsatz jeder Komponente der Beschichtungsmembran ist; wobei das Celluloseacetat eine Celluloseacetatmembran ist, die 39,8 Gew.-% Acetyl enthält, und das Gewicht der Beschichtungsmembran 5,9 % des Gewichts des Tablettenkerns beträgt; und der Überzug aus 24 Gew.-% Levodopa, 65 Gew.-% Carbidopa, 10 Gew.-% Hydroxypropylcellulose und 1 Gew.-% Aspartam besteht, wobei der Gewichtsprozentsatz der Gewichtsprozentsatz jeder Komponente des Überzugs ist, das Gewicht des Überzugs 13,1% des Gesamtgewichts des Tablettenkerns und der Beschichtungsmembran beträgt;
oder, die pharmazeutische Zusammensetzung aus einer arzneimittelhaltigen Schicht, einer osmotischen Ausstoßschicht, einer Beschichtungsmembran und einem Überzug besteht; die arzneimittelhaltige Schicht aus 45 Gew.-% Levodopa, 31 Gew.-% Hydroxypropylcellulose, 22 Gew.-% Mannitol, 0,9 Gew.-% Aspartam, 1 Gew.-% Magnesiumstearat und 0,1 Gew.-% Minzgeschmack besteht, wobei der Gewichtsprozentsatz der Gewichtsprozentsatz jeder Komponente der arzneimittelhaltigen Schicht ist; die osmotische Ausstoßschicht aus 55 Gew.-% Natriumcarboxymethylcellulose 7H4XF, 10 Gew.-% Hydroxypropylcellulose, 34 Gew.-% Sorbitol, 0,5 Gew.-% Eisenoxidrot und 0,5 Gew.-% Magnesiumstearat besteht, wobei der Gewichtsprozentsatz der Gewichtsprozentsatz jeder Komponente der osmotischen Ausstoßschicht ist; die Beschichtungsmembran aus 70 Gew.-% Celluloseacetatmembran und 30 Gew.-% Copovidon VA64 besteht, wobei der Gewichtsprozentsatz der Gewichtsprozentsatz jeder Komponente der Beschichtungsmembran ist; wobei das Celluloseacetat eine Celluloseacetatmembran ist, die 39,8 Gew.-% Acetyl enthält, und das Gewicht der Beschichtungsmembran 6,5% des Gewichts des Tablettenkerns beträgt; und der Überzug aus 54 Gew.-% Levodopa, 35 Gew.-% Carbidopa, 10 Gew.-% Hydroxypropylcellulose, 0,9 Gew.-% Aspartam und 0,1 Gew.-% Minzgeschmack besteht, wobei der Gewichtsprozentsatz der Gewichtsprozentsatz jeder Komponente des Überzugs ist, das Gewicht des Überzugs 13,1% des Gesamtgewichts des Tablettenkerns und der Beschichtungsmembran beträgt;
oder die pharmazeutische Zusammensetzung aus einer arzneimittelhaltigen Schicht, einer osmotischen Ausstoßschicht, einer Beschichtungsmembran und einem Überzug besteht; die arzneimittelhaltige Schicht aus 45 Gew.-% Levodopa, 31 Gew.-% Hydroxypropylcellulose, 22 Gew.-% Mannitol, 0,9 Gew.-% Aspartam, 1 Gew.-% Magnesiumstearat und 0,1 Gew.-% Minzgeschmack besteht, wobei der Gewichtsprozentsatz der Gewichtsprozentsatz jeder Komponente der arzneimittelhaltigen Schicht ist; die osmotischen Ausstoßschicht aus 55 Gew.-% Natriumcarboxymethylcellulose 7H4XF, 10 Gew.-% Hydroxypropylcellulose, 34 Gew.-% Sorbitol, 0,5 Gew.-% Eisenoxidrot und 0,5 Gew.-% Magnesiumstearat besteht, wobei der Gewichtsprozentsatz der Gewichtsprozentsatz jeder Komponente der osmotischen Ausstoßschicht ist; die Beschichtungsmembran aus 70 Gew.-% Celluloseacetatmembran und 30 Gew.-% Copovidon VA64 besteht, wobei der Gewichtsprozentsatz der Gewichtsprozentsatz jeder Komponente der Beschichtungsmembran ist; wobei das Celluloseacetat eine Celluloseacetatmembran ist, die 39,8 Gew.-% Acetyl enthält, und das Gewicht der Beschichtungsmembran 7,0% des Gewichts des Tablettenkerns beträgt; und der Überzug aus 42,8 Gew.-% Levodopa, 46,2 Gew.-% Carbidopa, 10 Gew.-% Hydroxypropylcellulose, 0,9 Gew.-% Aspartam und 0,1 Gew.-% Minzgeschmack besteht, wobei der Gewichtsprozentsatz der Gewichtsprozentsatz jeder Komponente des Überzugs ist, das Gewicht des Überzugs 13,1% des Gesamtgewichts des Tablettenkerns und der Beschichtungsmembran beträgt;
oder, die pharmazeutische Zusammensetzung aus einer arzneimittelhaltigen Schicht, einer osmotischen Ausstoßschicht, einer Beschichtungsmembran und einem Überzug besteht; die arzneimittelhaltige Schicht aus 45 Gew.-% Levodopa, 31 Gew.-% Hydroxypropylcellulose, 22 Gew.-% Mannitol, 0,9 Gew.-% Aspartam, 1 Gew.-% Magnesiumstearat und 0,1 Gew.-% Minzgeschmack besteht, wobei der Gewichtsprozentsatz der Gewichtsprozentsatz jeder Komponente der arzneimittelhaltigen Schicht ist; die osmotische Ausstoßschicht aus 55 Gew.-% Natriumcarboxymethylcellulose 7H4XF, 10 Gew.-% Hydroxypropylcellulose, 34 Gew.-% Sorbitol, 0,5 Gew.-% Eisenoxidrot und 0,5 Gew.-% Magnesiumstearat besteht, wobei der Gewichtsprozentsatz der Gewichtsprozentsatz jeder Komponente der osmotischen Ausstoßschicht ist; die Beschichtungsmembran aus 70 Gew.-% Celluloseacetatmembran und 30 Gew.-% Copovidon VA64 besteht, wobei der Gewichtsprozentsatz der Gewichtsprozentsatz jeder Komponente der Beschichtungsmembran ist; wobei das Celluloseacetat eine Celluloseacetatmembran ist, die 39,8 Gew.-% Acetyl enthält, und das Gewicht der Beschichtungsmembran 9,0% des Gewichts des Tablettenkerns beträgt; und der Überzug aus 28,2 Gew.-% Levodopa, 60,8 Gew.-% Carbidopa, 10 Gew.-% Hydroxypropylcellulose, 0,9 Gew.-% Aspartam und 0,1 Gew.-% Minzgeschmack besteht, wobei der Gewichtsprozentsatz der Gewichtsprozentsatz jeder Komponente des Überzugs ist, das Gewicht des Überzugs 13,1% des Gesamtgewichts des Tablettenkerns und der Beschichtungsmembran beträgt.

16. Dosierungsform mit verlängerter Freisetzung gemäß irgendeinem der Ansprüche 1-15, wobei die pharmazeutische Zusammensetzung ein Arzneimittelabgabesystem mit verlängerter Freisetzung durch eine osmotische Pumpe ("osmotic pump extended release drug delivery system") ist, wobei das Arzneimittelabgabesystem mit verlängerter Freisetzung durch eine osmotische Pumpe optional eine Tablette mit verlängerter Freisetzung ist; wobei die Tablette mit verlängerter Freisetzung ein Zylinder mit einem Durchmesser von 5-10 mm und einer Höhe von 5-30 mm ist oder eine Kapsel mit einer Länge von 10-25 mm und einer Breite von 5-10 mm ist; und/oder jede der Tabletten mit verlängerter Freisetzung 62,5 mg CD und 500 mg LD, oder 62,5 mg CD und 375 mg LD, oder 62,5 mg Carbidopa und 250 mg Levodopa, oder 50 mg Carbidopa und 500 mg Levodopa, oder 37,5 mg Carbidopa und 375 mg Levodopa enthält.

17. Dosierungsplattform mit verlängerter Freisetzung gemäß irgendeinem der Ansprüche 1, 2 oder 4-16 zur Verwendung in einem Operationsverfahren, das die folgenden Schritte umfasst: Platzieren der Plattform mit verlängerter Freisetzung in die personalisierte Plattform zur Ermöglichung der Retention und Befestigen der Plattform zur Ermöglichung der Retention an den entsprechenden Zähnen in der Mundhöhle; Herausnehmen der Dosierungsform mit verlängerter Freisetzung nach Aufbewahrung für 4-24 Stunden, Ersetzen der Plattform mit verlängerter Freisetzung durch eine neue und erneutes Befestigen der Plattform zur Ermöglichung der Retention an den entsprechenden Zähnen in der Mundhöhle, so dass das Arzneimittel kontinuierlich und stabil freigesetzt werden kann.

## Revendications

1. Forme posologique à libération prolongée avec une fenêtre d'absorption au niveau du tractus gastro-intestinal supérieur comprenant une plate-forme à libération prolongée et une plate-forme à rétention possible ;
dans laquelle la plate-forme à libération prolongée est une composition pharmaceutique comprenant un noyau de comprimé et une membrane de revêtement, le noyau de comprimé comprend une couche contenant un médicament, et la membrane de revêtement comprend de l'acétate de cellulose et de la copovidone, dans laquelle le poids de l'acétate de cellulose est de 50 à 70 % du poids de la membrane de revêtement, le poids de la copovidone est de 30 à 50 % du poids de la membrane de revêtement ;
dans laquelle la plate-forme à rétention possible est utilisée pour maintenir la plate-forme à libération prolongée dans la cavité buccale, et au moins une extrémité de la plate-forme à rétention possible est reliée à un couvercle, de sorte que la plate-forme à libération prolongée peut être retenue dans la plate-forme à rétention possible ;
la couche contenant un médicament comprend des ingrédients pharmaceutiques actifs et des excipients, et l'ingrédient pharmaceutique actif est un ou plusieurs parmi la lévodopa et la carbidopa.

2. Forme posologique à libération prolongée selon la revendication 1, dans laquelle la plate-forme à rétention possible comprend un module à rétention possible personnalisé et un module fixé à un médicament, le module à fixation de médicament peut fixer la plate-forme à libération prolongée, et le module à rétention possible personnalisé peut maintenir le module à fixation de médicament dans la cavité buccale ;
facultativement, le module à fixation de médicament est un ou plusieurs réservoirs ; et/ou, le module à rétention possible peut s'adapter à une ou plusieurs quelconques dents dans la cavité buccale ; et/ou, le module à rétention possible peut être personnalisé pour s'adapter et envelopper, serrer ou être inséré entre des dents permanentes maxillaires ou mandibulaires ;
dans laquelle le réservoir présente une structure de panier ; et/ou, la forme de la section transversale du réservoir est polygonale, circulaire en boucle fermée ou circulaire en boucle ouverte, ou une combinaison de celles-ci ; et/ou,
au moins une extrémité du réservoir est reliée à un couvercle, de sorte que la plate-forme à libération prolongée peut être maintenue dans le réservoir ; le couvercle est facultativement un ruban ; et/ou,
le module à rétention possible peut s'adapter à une ou plusieurs des dents permanentes mandibulaires, la molaire mandibulaire, la seconde molaire mandibulaire et ses molaires antérieures et postérieures dans la cavité buccale ; et/ou,
les dents permanentes maxillaires ou mandibulaires peuvent être des dents permanentes maxillaires entières, ou des dents permanentes mandibulaires entières, ou la molaire mandibulaire, ou la seconde molaire mandibulaire et ses parties adjacentes de la première molaire et de la seconde prémolaire.

3. Procédé de préparation de la forme posologique à libération prolongée selon l'une quelconque des revendications 1 ou 2, dans lequel le procédé de préparation de la plate-forme à rétention possible comprend une impression 3D, un moulage par injection ou le moulage par prise d'empreinte ; facultativement, la plate-forme à rétention possible est préparée à partir d'un ou plusieurs matériaux stables par voie orale, et les matériaux stables par voie orale comprennent des métaux stables par voie orale et des élastomères thermoplastiques ; dans lequel le métal stable oral comprend du titane dentaire, de l'acier inoxydable, un alliage de cobalt-chrome, un alliage de cobalt-chrome-molybdène, un alliage de nickel-chrome ou un métal précieux, et l'élastomère thermoplastique comprend de la polycaprolactone, un copolymère d'éthylène-acétate de vinyle, du polyéthylène haute densité, du polypropylène, du polyacrylate, du polyuréthane, du polymère de silicone, du polyester, du poly (styrène-éthylène-butène-styrène), du poly (styrène-butadiène-styrène), du poly (styrène-isoprène-styrène), ou un copolymère de deux des éléments ci-dessus ou plus, ou leur physique combinaison.

4. Forme posologique à libération prolongée selon la revendication 1, dans laquelle dans la plate-forme à libération prolongée, dans la couche contenant un médicament, l'excipient est un ou plusieurs parmi une charge, un agent osmotique, un polymère hydrophile, un agent de liaison, un lubrifiant, un conservateur, un agent aromatisant, un agent acidifiant et un antioxydant ; ou l'excipient est un ou plusieurs parmi une charge, un agent osmotique, un polymère hydrophile, un agent de liaison, un lubrifiant et un conservateur; ou les excipients sont une charge, un agent osmotique, un polymère hydrophile, un agent de liaison, un lubrifiant et un conservateur ;
dans laquelle, lorsque l'ingrédient pharmaceutiquement actif comprend de la lévodopa, le pourcentage en poids de la lévodopa est de 20 à 70 % ; lorsque l'ingrédient actif comprend de la carbidopa, le pourcentage en poids de la carbidopa est de 0 à 20 %, mais pas de 0 % ; dans laquelle le pourcentage en poids est le pourcentage en poids de chaque composant de la couche contenant un médicament ;
ou, lorsque l'ingrédient pharmaceutiquement actif comprend de la lévodopa, le pourcentage en poids de la lévodopa est de 30 à 50 % ; lorsque l'ingrédient actif comprend de la carbidopa, le pourcentage en poids de carbidopa est de 1 à 10 % ; dans laquelle le pourcentage en poids est le pourcentage en poids de chaque composant de la couche contenant un médicament.

5. Forme posologique à libération prolongée selon la revendication 4, dans laquelle, dans la plate-forme à libération prolongée :
lorsque l'excipient comprend une charge, la charge est un ou plusieurs parmi de la cellulose microcristalline, de l'hydroxypropylcellulose et du mannitol, et le pourcentage en poids de la charge est de 0 à 50 %, mais pas de 0 % ;
et/ou, lorsque l'excipient comprend un agent osmotique, l'agent osmotique est un ou plusieurs parmi le sulfate de magnésium, le chlorure de magnésium, le chlorure de sodium, le chlorure de lithium, le sulfate de potassium, le sulfate de sodium, le mannitol, l'urée, le sorbitol, l'inositol, le saccharose et le glucose, et le pourcentage en poids de l'agent osmotique est de 0 à 50 %, mais pas de 0 % ;
et/ou, lorsque l'excipient comprend un polymère hydrophile, le polymère hydrophile est un ou plusieurs parmi l'hydroxypropyl cellulose, l'hydroxypropylméthylcellulose, la carboxyméthylcellulose, la polyvinylpyrrolidone et l'hydroxyéthylcellulose, et le pourcentage en poids du polymère hydrophile est de 0 à 50 %, mais pas de 0 % ;
et/ou, lorsque l'excipient comprend un agent acidifiant, l'agent acidifiant est un ou plusieurs parmi l'acide citrique, le citrate de sodium, le citrate de potassium, l'acide malique, l'acide fumarique, l'acide lactique, l'acide phosphorique et l'acide tartrique, et le pourcentage en poids du l'agent acidifiant est de 0 à 10 %, mais pas de 0 % ;
dans laquelle le pourcentage en poids est le pourcentage en poids de chaque composant de la couche contenant un médicament.

6. Forme posologique à libération prolongée selon la revendication 4 ou 5, dans laquelle dans la plate-forme à libération prolongée, le noyau de comprimé de la composition pharmaceutique comprend en outre une couche de poussée osmotique, et la couche de poussée osmotique comprenant un polymère hydrophile, un agent osmotique et un agent de liaison ; facultativement, la couche de poussée osmotique comprend un polymère hydrophile, un agent osmotique, un agent de liaison et un lubrifiant; ou, la couche de poussée osmotique comprend un polymère hydrophile, un agent osmotique, un agent de liaison, un lubrifiant et un colorant.

7. Forme posologique à libération prolongée selon la revendication 6, dans laquelle dans la plate-forme à libération prolongée, le polymère hydrophile dans la couche de poussée osmotique est le K-carraghénane, la carboxyméthylcellulose sodique ou l'oxyde de polyéthylène, dans laquelle le poids moléculaire du polymère hydrophile est de 75 000 à 7 500 000, et le pourcentage en poids du polymère hydrophile est de 25 à 85 % ;
et/ou, l'agent osmotique dans la couche de poussée de pompe osmotique est un ou plusieurs parmi le sulfate de magnésium, le chlorure de magnésium, le chlorure de sodium, le chlorure de lithium, le sulfate de potassium, le sulfate de sodium, le mannitol, l'urée, le sorbitol, l'inositol, le saccharose et le glucose, et le pourcentage en poids de l'agent osmotique est de 5 à 65 % ;
et/ou, lorsque la couche de poussée osmotique comprend un agent de liaison, l'agent de liaison est un ou plusieurs parmi la méthylcellulose, l'hydroxypropylméthylcellulose, l'hydroxypropylcellulose, la carboxyméthylcellulose sodique, la povidone et la gélatine, et le pourcentage en poids de l'agent de liaison est de 3 à 20% ;
et/ou, lorsque la couche de poussée osmotique comprend un lubrifiant, le lubrifiant est un ou plusieurs parmi le stéarate de magnésium, le fumarate de stéarate de magnésium, le talc et la silice colloïdale, et le pourcentage en poids du lubrifiant est de 0 à 2 %, mais pas de 0 % ;
et/ou, lorsque la couche de poussée osmotique comprend un colorant, le colorant est un ou plusieurs parmi l'oxyde de fer rouge, l'oxyde de fer jaune et l'oxyde de fer noir, et le pourcentage en poids du colorant est de 0 à 5 %, mais pas de 0 % ;
dans laquelle le pourcentage en poids est le pourcentage en poids de chaque composant de la couche de poussée osmotique.

8. Forme posologique à libération prolongée selon la revendication 6, dans laquelle dans la plate-forme à libération prolongée, la couche de poussée osmotique comprend de la carboxyméthylcellulose sodique, de la povidone K30, du sorbitol, de l'oxyde de fer rouge et du stéarate de magnésium ; ou comprend de la carboxyméthylcellulose sodique, de l'hydroxypropylcellulose, du sorbitol, de l'oxyde de fer rouge et du stéarate de magnésium ;
facultativement, la carboxyméthylcellulose sodique est la carboxyméthylcellulose sodique 7H4XF ou 9H4XF ;
et/ou, la couche de poussée osmotique comprend de 25 à 85 % en poids de carboxyméthylcellulose sodique, de 5 à 65 % en poids de sorbitol, de 3 à 20 % en poids de povidone, de 0 à 5 % en poids d'oxyde de fer rouge et de 0,5 à 2 % en poids de stéarate de magnésium ;
ou, la couche de poussée osmotique comprend 55 % en poids de carboxyméthylcellulose sodique, de 34,0 à 39,0 % en poids de sorbitol, de 3 à 20 % en poids de povidone ou 10 % en poids d'hydroxypropylcellulose, de 0,5 à 5 % en poids d'oxyde de fer rouge et de 0,5 à 2 % en poids de stéarate de magnésium ;
ou, la couche de poussée osmotique comprend 55 % en poids de carboxyméthylcellulose sodique, 39,0 % en poids de sorbitol, 5,0 % en poids de povidone et 0,5 % en poids d'oxyde de fer rouge et 0,5 % en poids de stéarate de magnésium ; ou comprend 55 % en poids de carboxyméthylcellulose sodique, 34,0 % en poids de sorbitol, 10,0 % en poids de povidone, 0,5 % en poids d'oxyde de fer rouge et 0,5 % en poids de stéarate de magnésium, ou comprend 55 % en poids de carboxyméthylcellulose sodique, 34,0 % en poids de sorbitol, 10,0 % en poids d'hydroxypropylcellulose, 0,5 % en poids d'oxyde de fer rouge et 0,5 % en poids de stéarate de magnésium ; dans laquelle le pourcentage en poids est le pourcentage en poids de chaque composant de la couche de poussée osmotique.

9. Forme posologique à libération prolongée selon l'une quelconque des revendications 5 à 8, dans laquelle dans la plate-forme à libération prolongée, la membrane de revêtement de la composition pharmaceutique est en outre recouverte d'une surcouche à libération immédiate contenant un médicament ;
la surcouche à libération immédiate contenant un médicament comprend facultativement un ingrédient pharmaceutique actif et un excipient, l'ingrédient pharmaceutique actif comprend de la lévodopa et/ou de la carbidopa, et l'excipient est un ou plusieurs parmi l'hydroxypropylcellulose, l'aspartame et l'arôme de menthe ;
dans laquelle, lorsque l'ingrédient pharmaceutique actif est la lévodopa, le pourcentage en poids de la lévodopa est de 0 à 75 % mais pas de 0 %, par exemple de 23,78 à 75 % ; et/ou, lorsque l'ingrédient pharmaceutique actif est la carbidopa, le pourcentage en poids de carbidopa est de 0 à 93 % mais pas de 0 %, par exemple de 26,85 à 93 % ; et/ou, lorsque l'excipient de la surcouche comprend de l'hydroxypropylcellulose, le pourcentage en poids de l'hydroxypropylcellulose est de 2 à 20 %, par exemple 10 % ; et/ou, lorsque l'excipient de la surcouche comprend de l'aspartame, le pourcentage en poids de l'aspartame est de 0 à 5 %, par exemple de 0,9 à 5 % ; et/ou, lorsque l'excipient de la surcouche comprend l'arôme de menthe, le pourcentage en poids de l'arôme de menthe est de 0 à 5 %, par exemple de 0,1 % ; dans laquelle le pourcentage en poids est le pourcentage en poids de chaque composant de la surcouche.

10. Forme posologique à libération prolongée selon l'une quelconque des revendications 4-9, dans laquelle dans la plate-forme à libération prolongée, le poids de la membrane de revêtement n'est pas inférieur à 2,0 %, par exemple de 2,0 à 15,0 % ou de 4,0 à 8,0 % du poids du noyau de comprimé ; la membrane de revêtement présente un ou plusieurs orifices, et le diamètre de l'orifice est facultativement de 0,5 mm à 1,0 mm, par exemple de 0,5 mm, de 0,75 mm et de 1,0 mm ;
facultativement, dans la plate-forme à libération prolongée, la composition pharmaceutique est composée d'une couche contenant un médicament et d'une membrane de revêtement ; la composition pharmaceutique est composée d'une couche contenant un médicament, d'une couche de poussée osmotique et d'une membrane de revêtement ; ou la composition pharmaceutique est composée d'une couche contenant un médicament, d'une couche de poussée osmotique, d'une membrane de revêtement et d'une surcouche ;
et/ou, la couche contenant le médicament est composée de lévodopa, de carbidopa, de cellulose microcristalline, de mannitol, d'acide citrique, d'hydroxypropylméthylcellulose sodique et de stéarate de magnésium ; ou la couche contenant un médicament est composée de lévodopa, de cellulose microcristalline, d'hydroxypropylméthylcellulose et de stéarate de magnésium ; ou la couche contenant un médicament est composée de lévodopa, de carbidopa, de mannitol, d'acide citrique et de stéarate de magnésium ; ou la couche contenant un médicament est composée de lévodopa, de carbidopa, d'hydroxypropylcellulose, de mannitol, d'acide citrique et de stéarate de magnésium ; ou la couche contenant un médicament est composée de lévodopa, de carbidopa, d'hydroxypropylcellulose, de mannitol, d'acide citrique et de povidone K30; ou la couche contenant un médicament est composée de lévodopa, d'hydroxypropylcellulose, de mannitol, de povidone K30, de stéarate de magnésium, d'arôme de menthe et d'aspartame ; ou la couche contenant un médicament est composée de lévodopa, de mannitol, de povidone K30 et de stéarate de magnésium ; ou la couche contenant un médicament est composée de lévodopa, de carbidopa, d'hydroxypropylcellulose, de mannitol, d'aspartame et de stéarate de magnésium ; ou la couche contenant un médicament est composée de lévodopa, d'hydroxypropylcellulose, de mannitol, de stéarate de magnésium, d'arôme de menthe et d'aspartame; ou la couche contenant un médicament est composée de lévodopa, d'hydroxypropylcellulose, de mannitol, de povidone K30, de stéarate de magnésium et d'aspartame ; ou la couche contenant un médicament est composée de lévodopa, d'hydroxypropylcellulose, de mannitol, de povidone K30, de stéarate de magnésium et d'aspartame ;
et/ou, la couche de poussée osmotique est composée de carboxyméthylcellulose sodique, de povidone K30, de sorbitol, d'oxyde de fer rouge et de stéarate de magnésium, ou la couche de poussée osmotique est composée de carboxyméthylcellulose sodique, d'hydroxypropylcellulose, de sorbitol, d'oxyde de fer rouge et de stéarate de magnésium ; facultativement, la carboxyméthylcellulose sodique est la carboxyméthylcellulose sodique 7H4XF ou 9H4XF ;
et/ou, la surcouche est composée de lévodopa, de carbidopa, d'hydroxypropylcellulose, d'aspartame et d'arôme de menthe ; ou la surcouche est composée de lévodopa, de carbidopa, d'hydroxypropylcellulose et d'aspartame ; ou la surcouche est composée de carbidopa, d'hydroxypropyl cellulose et d'aspartame ; ou la surcouche est composée de lévodopa, d'hydroxypropylcellulose et d'arôme de menthe.

11. Forme posologique à libération prolongée selon la revendication 10, dans laquelle dans la plate-forme à libération prolongée, lorsque la couche contenant un médicament est composée de lévodopa, de carbidopa, de cellulose microcristalline, de mannitol, d'acide citrique, d'hydroxypropylméthylcellulose sodique et de stéarate de magnésium, le pourcentage en poids de lévodopa est de 40 %, le pourcentage en poids de carbidopa est de 10,8 %, le pourcentage en poids de cellulose microcristalline est de 20 %, le pourcentage en poids de mannitol est de 18,7 %, le pourcentage en poids d'acide citrique est de 5 %, le pourcentage en poids d'hydroxypropylméthylcellulose sodique est de 5 %, et le pourcentage en poids de stéarate de magnésium est de 0,5 %, dans laquelle le pourcentage en poids est le pourcentage en poids de chaque composant de la couche contenant un médicament ;
lorsque la couche contenant un médicament est composée de lévodopa, de cellulose microcristalline, d'hydroxypropylméthylcellulose et de stéarate de magnésium, et lorsque le pourcentage en poids de lévodopa est de 38 %, le pourcentage en poids de cellulose microcristalline est de 50 %, le pourcentage en poids d'hydroxypropylméthylcellulose est de 10 %, et le pourcentage en poids de stéarate de magnésium est de 2 %, dans laquelle le pourcentage en poids est le pourcentage en poids de chaque composant de la couche contenant un médicament ;
lorsque la couche contenant un médicament est composée de lévodopa, de carbidopa, de mannitol, d'acide citrique et de stéarate de magnésium, dans laquelle le pourcentage en poids de la lévodopa est de 19,5 % et le pourcentage en poids de carbidopa est de 20 %, le pourcentage en poids de mannitol est de 50 %, le pourcentage en poids d'acide citrique est de 10 % et le pourcentage en poids de stéarate de magnésium est de 0,5 %, dans laquelle le pourcentage en poids est le pourcentage en poids de chaque composant de la couche contenant un médicament ;
lorsque la couche contenant le médicament est composée de lévodopa, de carbidopa, d'hydroxypropylcellulose, de mannitol, d'acide citrique et de stéarate de magnésium, le pourcentage en poids de la lévodopa est de 40 %, le pourcentage en poids de carbidopa est de 10,8 %, le pourcentage en poids d'hydroxypropylcellulose est 31 %, le pourcentage en poids de mannitol est de 12,7 % et le pourcentage en poids d'acide citrique est de 5 %, le pourcentage en poids du stéarate de magnésium est de 0,5 %, dans laquelle le pourcentage en poids est le pourcentage en poids de chaque composant de la couche contenant un médicament ;
lorsque la couche contenant un médicament est composée de lévodopa, de carbidopa, d'hydroxypropylcellulose, de mannitol, d'acide citrique et de povidone K30, le pourcentage en poids de lévodopa est de 40 %, le pourcentage en poids de carbidopa est de 10,8 %, le pourcentage en poids d'hydroxypropylcellulose est de 31 %, le pourcentage en poids de mannitol est de 12,7 % et le pourcentage en poids d'acide citrique est de 5 %, le pourcentage en poids de povidone K30 est de 0,5 %, dans laquelle le pourcentage en poids est le pourcentage en poids de chaque composant de la couche contenant un médicament ;
lorsque la couche contenant un médicament est composée de lévodopa, d'hydroxypropylcellulose, de mannitol, de povidone K30, de stéarate de magnésium, d'arôme de menthe et d'aspartame, le pourcentage en poids de lévodopa est de 45 %, le pourcentage en poids d'hydroxypropylcellulose est de 31 %, le pourcentage en poids de mannitol est de 16 %, le pourcentage en poids de povidone K30 est de 5 %, le pourcentage en poids du stéarate de magnésium est de 1 %, le pourcentage en poids de l'arôme de menthe est de 1 % et le pourcentage en poids de l'aspartame est de 1 %, dans laquelle le pourcentage en poids est le pourcentage en poids de chaque composant de la couche contenant un médicament ;
lorsque la couche contenant un médicament est composée de lévodopa, de mannitol, de povidone K30 et de stéarate de magnésium, le pourcentage en poids de lévodopa est de 70 %, le pourcentage en poids de mannitol est de 9 %, le pourcentage en poids de povidone K30 est de 20 %, et le pourcentage en poids de stéarate de magnésium est de 1 %, dans laquelle le pourcentage en poids est le pourcentage en poids de chaque composant de la couche contenant un médicament ;
lorsque la couche contenant un médicament est composée de lévodopa, de carbidopa, d'hydroxypropylcellulose, de mannitol, d'aspartame et de stéarate de magnésium, le pourcentage en poids de lévodopa est de 20 %, le pourcentage en poids de carbidopa est de 20 %, le pourcentage en poids d'hydroxypropylcellulose est de 50 % , le pourcentage en poids de mannitol est de 4 % et le pourcentage en poids d'aspartame est de 5 %, le pourcentage en poids de stéarate de magnésium est de 1 %, dans laquelle le pourcentage en poids est le pourcentage en poids de chaque composant de la couche contenant un médicament ;
lorsque la couche contenant le médicament est composée de lévodopa, d'hydroxypropylcellulose, de mannitol, de povidone K30, de stéarate de magnésium et d'aspartame, le pourcentage en poids de lévodopa est de 45 %, le pourcentage en poids de l'hydroxypropylcellulose est de 31 %, le pourcentage en poids du mannitol est de 16 %, le pourcentage en poids de la povidone K30 est de 5 %, le pourcentage en poids de stéarate de magnésium est de 1 %, le pourcentage en poids de l'arôme de menthe est de 1 % et le pourcentage en poids d'aspartame est de 1 %, dans laquelle le pourcentage en poids est le pourcentage en poids de chaque composant de la couche contenant un médicament ;
lorsque la couche contenant un médicament est composée de lévodopa, d'hydroxypropylcellulose, de mannitol, de povidone K30, de stéarate de magnésium et d'aspartame, le pourcentage en poids de lévodopa est de 45 %, le pourcentage en poids de l'hydroxypropylcellulose est de 31 %, le pourcentage en poids du mannitol est de 17 %, le pourcentage en poids de la povidone K30 est de 5 %, le pourcentage en poids de stéarate de magnésium est de 1 % et le pourcentage en poids d'aspartame est de 1 %, dans laquelle le pourcentage en poids est le pourcentage en poids de chaque composant de la couche contenant un médicament ;
ou, lorsque la couche contenant un médicament est composée de lévodopa, d'hydroxypropylcellulose, de mannitol, de povidone K30, de stéarate de magnésium, d'arôme de menthe et d'aspartame ; le pourcentage en poids de la lévodopa est de 45 %, le pourcentage en poids de l'hydroxypropylcellulose est de 31 %, le pourcentage en poids du mannitol est de 12 % et le pourcentage en poids de la povidone K30 est de 5 %, le pourcentage en poids de l'arôme de menthe est de 5 %, le pourcentage en poids d'aspartame est de 1 % et le pourcentage en poids de stéarate de magnésium est de 1 %, dans laquelle le pourcentage en poids est le pourcentage en poids de chaque composant de la couche contenant un médicament ;
ou, lorsque la couche contenant un médicament est composée de lévodopa, d'hydroxypropylcellulose, de mannitol, de stéarate de magnésium, d'arôme de menthe et d'aspartame, le pourcentage en poids de la lévodopa est de 62,5 %, le pourcentage en poids de l'hydroxypropylcellulose est de 31 %, le pourcentage en poids du mannitol est de 4,5 %, le pourcentage en poids de l'arôme de menthe est de 0,1 %, le pourcentage en poids d'aspartame est de 0,9 % et le pourcentage en poids de stéarate de magnésium est de 1 %, dans laquelle le pourcentage en poids est le pourcentage en poids de chaque composant de la couche contenant un médicament ;
ou, lorsque la couche contenant un médicament est composée de lévodopa, d'hydroxypropylcellulose, de mannitol, de stéarate de magnésium, d'arôme de menthe et d'aspartame, le pourcentage en poids de la lévodopa est de 46,9 %, le pourcentage en poids de l'hydroxypropylcellulose est de 31 %, le pourcentage en poids du mannitol est de 20,1 %, le pourcentage en poids de l'arôme de menthe est de 0,1 %, le pourcentage en poids d'aspartame est de 0,9 %, le pourcentage en poids de stéarate de magnésium est de 1 %, dans laquelle le pourcentage en poids est le pourcentage en poids de chaque composant de la couche contenant un médicament.

12. Forme posologique à libération prolongée selon la revendication 10, dans laquelle, dans la plate-forme à libération prolongée, la membrane de revêtement est composée de 50 % en poids de membrane d'acétate de cellulose et de 50 % en poids de copovidone VA64; la membrane de revêtement est composée de 70 % en poids de membrane d'acétate de cellulose et de 30 % en poids de copovidone VA64; la membrane de revêtement est composée de 60 % en poids de membrane d'acétate de cellulose et de 40 % en poids de copovidone VA64; dans laquelle le pourcentage en poids est le pourcentage en poids de chaque composant de la membrane de revêtement ;
facultativement, le poids de la membrane de revêtement est de 2,0 %, 4,2 %, 4,5 %, 4,6 %, 4,8 %, 5,0 %, 5,9 %, 6,5 %, 6,7 %, 7,0 %, 7,7 %, 7,9 % ou 9,7 % du poids du noyau de comprimé.

13. Forme posologique à libération prolongée selon l'une quelconque des revendications 10-12, dans laquelle dans la plate-forme à libération prolongée,
lorsque la couche de poussée osmotique est composée de carboxyméthylcellulose sodique 7H4XF, de povidone K30, de sorbitol, d'oxyde de fer rouge et de stéarate de magnésium,
le pourcentage en poids de la carboxyméthylcellulose sodique 7H4XF est de 55 %, le pourcentage en poids de la povidone K30 est de 5 %, le pourcentage en poids du sorbitol est de 39 %, le pourcentage en poids de l'oxyde de fer rouge est de 0,5 %, et le pourcentage en poids du stéarate de magnésium est de 0,5 %, dans laquelle le pourcentage en poids est le pourcentage en poids de chaque composant de la couche de poussée osmotique ;
le pourcentage en poids de la carboxyméthylcellulose sodique 7H4XF est de 55 %, le pourcentage en poids de la povidone K30 est de 10 %, le pourcentage en poids du sorbitol est de 34 %, le pourcentage en poids de l'oxyde de fer rouge est de 0,5 %, et le pourcentage en poids du stéarate de magnésium est de 0,5 %, dans laquelle le pourcentage en poids est le pourcentage en poids de chaque composant de la couche de poussée osmotique ;
le pourcentage en poids de la carboxyméthylcellulose sodique est de 85 %, le pourcentage en poids de la povidone K30 est de 3 %, le pourcentage en poids du sorbitol est de 5 %, le pourcentage en poids de l'oxyde de fer rouge est de 5 %, et le pourcentage en poids du stéarate de magnésium est de 2 %, dans laquelle le pourcentage en poids est le pourcentage en poids de chaque composant de la couche de poussée osmotique ;
le pourcentage en poids de la carboxyméthylcellulose sodique est de 25 %, le pourcentage en poids de la povidone K30 est de 9,5 %, le pourcentage en poids du sorbitol est de 65 % et le pourcentage en poids du stéarate de magnésium est de 0,5 %, dans laquelle le pourcentage en poids est le pourcentage en poids de chaque composant de la couche de poussée osmotique ;
le pourcentage en poids de la carboxyméthylcellulose sodique 7H4XF est de 60 %, le pourcentage en poids de la povidone K30 est de 10 %, le pourcentage en poids du sorbitol est de 26 %, le pourcentage en poids de l'oxyde de fer rouge est de 2 %, et le pourcentage en poids du stéarate de magnésium est de 2 %, dans laquelle le pourcentage en poids est le pourcentage en poids de chaque composant de la couche de poussée osmotique ;
le pourcentage en poids de la carboxyméthylcellulose sodique 7H4XF est de 40 %, le pourcentage en poids de la povidone K30 est de 20 %, le pourcentage en poids du sorbitol est de 36 %, le pourcentage en poids de l'oxyde de fer rouge est de 3,5 %, et le pourcentage en poids du stéarate de magnésium est de 0,5 %, dans laquelle le pourcentage en poids est le pourcentage en poids de chaque composant de la couche de poussée osmotique ;
ou, lorsque la couche de poussée osmotique est composée de carboxyméthylcellulose sodique 9H4XF, de povidone K30, de sorbitol, d'oxyde de fer rouge et de stéarate de magnésium, le pourcentage en poids de carboxyméthylcellulose sodique 9H4XF est de 55 %, le pourcentage en poids de povidone K30 est de 5 %, le pourcentage en poids de sorbitol est de 39 %, et le pourcentage en poids d'oxyde de fer rouge est de 0,5 % et le pourcentage en poids de stéarate de magnésium est de 0,5 %, dans laquelle le pourcentage en poids est le pourcentage en poids de chaque composant de la couche de poussée osmotique ;
ou, lorsque la couche de poussée osmotique est composée de carboxyméthylcellulose sodique 7H4XF, d'hydroxypropylcellulose, de sorbitol, d'oxyde de fer rouge et de stéarate de magnésium, le pourcentage en poids de carboxyméthylcellulose sodique 7H4XF est de 55 %, le pourcentage en poids d'hydroxypropylcellulose est de 10 %, le pourcentage en poids de sorbitol est de 34 %, le pourcentage en poids de l'oxyde de fer rouge est de 0,5 % et le pourcentage en poids du stéarate de magnésium est de 0,5 %, dans laquelle le pourcentage en poids est le pourcentage en poids de chaque composant de la couche de poussée osmotique.

14. Forme posologique à libération prolongée selon l'une quelconque des revendications 10-13, dans laquelle, dans la plate-forme à libération prolongée, lorsque la surcouche est composée de lévodopa, de carbidopa, d'hydroxypropylcellulose, d'aspartame et d'arôme de menthe, le pourcentage en poids de la lévodopa est de 23,78 %, le pourcentage en poids de la carbidopa est de 64,22 %, le pourcentage en poids de l'hydroxypropylcellulose est de 10 % et le pourcentage en poids de l'aspartame est de 1 %, le pourcentage en poids de l'arôme de menthe est de 1 % ; ou le pourcentage en poids de la lévodopa est de 54 %, le pourcentage en poids de la carbidopa est de 35 %, le pourcentage en poids de l'hydroxypropylcellulose est de 10 % et le pourcentage en poids de l'aspartame est de 0,9 %, le pourcentage en poids de l'arôme de menthe est de 0,1 % ; le pourcentage en poids de la lévodopa est de 42,8 %, le pourcentage en poids de la carbidopa est de 46,2 %, le pourcentage en poids de l'hydroxypropylcellulose est de 10 % et le pourcentage en poids de l'aspartame est de 0,9 %, et le pourcentage en poids de l'arôme de menthe est de 0,1 %, dans laquelle le pourcentage en poids est le pourcentage en poids de chaque composant de la surcouche ;
lorsque la surcouche est composée de lévodopa, de carbidopa, d'hydroxypropylcellulose, d'aspartame et d'arôme de menthe, le pourcentage en poids de la lévodopa est de 62,15%, le pourcentage en poids de la carbidopa est de 26,85%, le pourcentage en poids de l'hydroxypropylcellulose est de 10%, le pourcentage en poids de l'aspartame est de 0,9 % et le pourcentage en poids de l'arôme de menthe est de 0,1 % ;
ou, le pourcentage en poids de la lévodopa est de 42,8 %, le pourcentage en poids de la carbidopa est de 46,2 %, le pourcentage en poids de l'hydroxypropylcellulose est de 10 %, le pourcentage en poids de l'aspartame est de 0,9 %, le pourcentage en poids de l'arôme de menthe est de 0,1 %, ou le pourcentage en poids de la lévodopa est de 28,2 %, et le pourcentage en poids de la carbidopa est de 60,8 %, le pourcentage en poids de l'hydroxypropylcellulose est de 10 %, le pourcentage en poids de l'aspartame est de 0,9 %, et le pourcentage en poids de la le pourcentage de l'arôme de menthe est de 0,1 %, dans laquelle le pourcentage en poids est le pourcentage en poids de chaque composant de la surcouche ;
ou, lorsque la surcouche est composée de carbidopa, d'hydroxypropylcellulose et d'aspartame, le pourcentage en poids de carbidopa est de 93 %, le pourcentage en poids d'hydroxypropyl cellulose est de 2 % et le pourcentage en poids d'aspartame est de 5 %, dans laquelle le pourcentage en poids est le pourcentage en poids de chaque composant de la surcouche ;
ou, lorsque la surcouche est composée de lévodopa, d'hydroxypropylcellulose et d'arôme de menthe, dans laquelle le pourcentage en poids de la lévodopa est de 75 %, le pourcentage en poids de l'hydroxypropylcellulose est de 20 % et le pourcentage en poids de l'arôme de menthe est de 5 %, dans laquelle le pourcentage en poids est le pourcentage en poids de chaque composant de la surcouche ;
ou, lorsque la surcouche est composée de lévodopa, de carbidopa, d'hydroxypropylcellulose et d'aspartame, le pourcentage en poids de lévodopa est de 24 %, le pourcentage en poids de carbidopa est de 65 %, le pourcentage en poids d'hydroxypropyl cellulose est de 10 % et le pourcentage en poids d'aspartame est de 1 %, dans laquelle le pourcentage en poids est le pourcentage en poids de chaque composant de la surcouche ;
facultativement, le gain de poids de la surcouche par rapport au noyau de comprimé est de 12,9 % ou de 13,2 % ou de 15,7 % ou de 21,0 % en poids.

15. Forme posologique à libération prolongée selon l'une quelconque des revendications 10-14, dans laquelle dans la plate-forme à libération prolongée, la composition pharmaceutique est composée d'une couche contenant un médicament et d'une membrane de revêtement, la couche contenant un médicament est composée de 40 % en poids de lévodopa, de 10,8 % en poids de carbidopa, de 20 % en poids de cellulose microcristalline, de 18,7 % en poids de mannitol, de 5 % en poids d'acide citrique, de 5 % en poids d'hydroxypropylméthylcellulose sodique et de 0,5 % en poids de stéarate de magnésium, dans laquelle le pourcentage en poids est le pourcentage en poids de chaque composant de la couche contenant un médicament; la membrane de revêtement est composée de 50 % en poids de membrane d'acétate de cellulose et de 50 % en poids de copovidone VA64, dans laquelle le pourcentage en poids est le pourcentage en poids de chaque composant de la membrane de revêtement; le poids de la membrane de revêtement est de 2,0 % du poids du noyau de comprimé ;
ou, la composition pharmaceutique est composée d'une couche contenant un médicament et d'une membrane de revêtement, la couche contenant un médicament est composée de 38 % en poids de lévodopa, de 50 % en poids de cellulose microcristalline, de 10 % en poids d'hydroxypropylméthylcellulose et de 2 % en poids de stéarate de magnésium, dans laquelle le pourcentage en poids est le pourcentage en poids de chaque composant de la couche contenant un médicament; la membrane de revêtement est composée de 50 % en poids de membrane d'acétate de cellulose et de 50 % en poids de copovidone VA64, dans laquelle le pourcentage en poids est le pourcentage en poids de chaque composant de la membrane de revêtement, et le poids de la membrane de revêtement est de 4,5 % du poids de noyau de comprimé ;
ou, la composition pharmaceutique est composée d'une couche contenant un médicament et d'une membrane de revêtement, la couche contenant un médicament comprend 19,5 % en poids de lévodopa, 20 % en poids de carbidopa, 50 % en poids de mannitol, 10 % en poids d'acide citrique et 0,5 % en poids de stéarate de magnésium, dans laquelle le pourcentage en poids est le pourcentage en poids de chaque composant de la couche contenant un médicament ; la membrane de revêtement est composée de 50 % en poids de membrane d'acétate de cellulose et de 50 % en poids de copovidone VA64, dans laquelle le pourcentage en poids est le pourcentage en poids de chaque composant de la membrane de revêtement, et le poids de la membrane de revêtement est de 4,5 % du poids de noyau de comprimé ;
ou, la composition pharmaceutique est composée d'une couche contenant un médicament, d'une couche de poussée osmotique et d'une membrane de revêtement, et la couche contenant un médicament est composée de 40 % en poids de lévodopa, de 10,8 % en poids de carbidopa, de 31 % en poids d'hydroxypropylcellulose, de 12,7 % en poids de mannitol, de 5 % en poids d'acide citrique et de 0,5 % en poids de stéarate de magnésium, dans laquelle le pourcentage en poids est le pourcentage en poids de chaque composant de la couche contenant un médicament ; la couche de poussée osmotique est composée de 55 % en poids de carboxyméthylcellulose sodique 7H4XF ou 9H4XF, de 5 % en poids de povidone K30, de 39 % en poids de sorbitol, de 0,5 % en poids d'oxyde de fer rouge et de 0,5 % en poids de stéarate de magnésium, dans laquelle le pourcentage en poids est le pourcentage en poids de chaque composant de la couche de poussée osmotique ; la membrane de revêtement est composée de 70 % en poids de membrane d'acétate de cellulose et de 30 % en poids de copovidone VA64, dans laquelle le pourcentage en poids est le pourcentage en poids de chaque composant de la membrane de revêtement ; le poids de la membrane de revêtement est de 2,0 %, de 4,0 % ou de 5,0 % du poids du noyau de comprimé ;
ou, la composition pharmaceutique est composée d'une couche contenant un médicament, d'une couche de poussée osmotique et d'une membrane de revêtement, la couche contenant un médicament est composée de 40 % en poids de lévodopa, de 10,8 % en poids de carbidopa, de 31 % en poids d'hydroxypropylcellulose, de 12,7 % en poids de mannitol, de 5 % en poids d'acide citrique et de 0,5 % en poids de povidone K30, dans laquelle le pourcentage en poids est le pourcentage en poids de chaque composant de la couche contenant un médicament; la couche de poussée osmotique est composée de 55 % en poids de carboxyméthylcellulose sodique 7H4XF, de 5 % en poids de povidone K30, de 39 % en poids de sorbitol, de 0,5 % en poids d'oxyde de fer rouge et de 0,5 % en poids de stéarate de magnésium, dans laquelle le pourcentage en poids est le pourcentage en poids de chaque composant de la couche de poussée osmotique ; la membrane de revêtement est composée de 60 % en poids de membrane d'acétate de cellulose et de 40 % de copovidone VA64, dans laquelle le pourcentage en poids est le pourcentage en poids de chaque composant de la membrane de revêtement ; et le poids de la membrane de revêtement est de 5,0 % du poids du noyau de comprimé ;
ou, la composition pharmaceutique est composée d'une couche contenant un médicament, d'une couche de poussée osmotique et d'une membrane de revêtement, la couche contenant un médicament est composée de 45 % en poids de lévodopa, de 31 % en poids d'hydroxypropylcellulose, de 16 % en poids de mannitol , de 5 % en poids de povidone K30 et 1 % en poids de stéarate de magnésium, 1 % en poids d'arôme de menthe et de 1 % en poids d'aspartame, dans laquelle le pourcentage en poids est le pourcentage en poids de chaque composant de la couche contenant le médicament ; la couche de poussée osmotique est composée de 55 % en poids de carboxyméthylcellulose sodique 7H4XF, de 10 % en poids de povidone K30, de 34 % en poids de sorbitol, de 0,5 % en poids d'oxyde de fer rouge et de 0,5 % en poids de stéarate de magnésium, dans laquelle le pourcentage en poids est le pourcentage en poids de chaque composant de la couche de poussée osmotique ; la membrane de revêtement est composée de 60 % en poids de membrane d'acétate de cellulose et de 40 % en poids de copovidone VA64, dans laquelle le pourcentage en poids est le pourcentage en poids de chaque composant de la membrane de revêtement ; et le poids de la membrane de revêtement est de 4,2 %, de 6,7 % ou de 9,7 % du poids du noyau de comprimé ;
ou, la composition pharmaceutique est composée d'une couche contenant un médicament, d'une couche de poussée osmotique et d'une membrane de revêtement, la couche contenant un médicament est composée de 45 % en poids de lévodopa, de 31 % en poids d'hydroxypropylcellulose, de 16 % en poids de mannitol , de 5 % en poids de povidone K30, de 1 % en poids de stéarate de magnésium, de 1 % en poids d'arôme de menthe et de 1 % en poids d'aspartame, dans laquelle le pourcentage en poids est le pourcentage en poids de chaque composant de la couche contenant un médicament ; la couche de poussée osmotique est composée de 55 % en poids de carboxyméthylcellulose sodique 7H4XF, de 10 % en poids de povidone K30, de 34 % en poids de sorbitol, de 0,5 % en poids d'oxyde de fer rouge et de 0,5 % en poids de stéarate de magnésium, dans laquelle le pourcentage en poids est le pourcentage en poids de chaque composant de la couche de poussée osmotique ; la membrane de revêtement est composée de 70 % en poids de membrane d'acétate de cellulose et de 30 % en poids de copovidone VA64, dans laquelle le pourcentage en poids est le pourcentage en poids de chaque composant de la membrane de revêtement ; et le poids de la membrane de revêtement est de 4,6 % ou de 7,9 % du poids du noyau de comprimé ;
ou, la composition pharmaceutique est composée d'une couche contenant un médicament, d'une couche de poussée osmotique et d'une membrane de revêtement, la couche contenant un médicament est composée de 70 % en poids de lévodopa, de 9 % en poids de mannitol, de 20 % en poids de povidone K30, de 1 % en poids de stéarate de magnésium, dans laquelle le pourcentage en poids est le pourcentage en poids de chaque composant de la couche contenant un médicament ; la couche de poussée osmotique est composée de 85 % en poids de carboxyméthylcellulose sodique, de 3 % en poids de povidone K30, de 5 % en poids de sorbitol, de 5 % en poids d'oxyde de fer rouge et de 2 % en poids de stéarate de magnésium, dans laquelle le pourcentage en poids est le pourcentage en poids de chaque composant de la couche de poussée osmotique ; la membrane de revêtement est composée de 70 % en poids de membrane d'acétate de cellulose et de 30 % en poids de copovidone VA64, dans laquelle le pourcentage en poids est le pourcentage en poids de chaque composant de la membrane de revêtement ; et le poids de la membrane de revêtement est de 4,5 % du poids du noyau de comprimé ;
ou, la composition pharmaceutique est composée d'une couche contenant un médicament, d'une couche de poussée osmotique, d'une membrane de revêtement et d'une surcouche, la couche contenant un médicament est composée de 45 % en poids de lévodopa, de 31 % en poids d'hydroxypropylcellulose, de 16 % en poids de mannitol, de 5 % en poids de povidone K30, de 1 % en poids de stéarate de magnésium, de 1 % en poids d'arôme de menthe et de 1 % en poids d'aspartame, dans laquelle le pourcentage en poids est le pourcentage en poids de chaque composant de la couche contenant un médicament; la couche de poussée osmotique est composée de 55 % en poids de carboxyméthylcellulose sodique 7H4XF, de 10 % en poids de povidone K30, de 34 % en poids de sorbitol, de 0,5 % en poids d'oxyde de fer rouge et de 0,5 % en poids de stéarate de magnésium, dans laquelle le pourcentage en poids est le pourcentage en poids de chaque composant de la couche de poussée osmotique ; la membrane de revêtement est composée de 70 % en poids de membrane d'acétate de cellulose et de 30 % en poids de copovidone VA64, dans laquelle le pourcentage en poids est le pourcentage en poids de chaque composant de la membrane de revêtement; et le poids de la membrane de revêtement représente de 4,8 % ou de 7,7 % du poids du noyau de comprimé, la surcouche est composée de 23,78 % en poids de lévodopa, de 64,22 % en poids de carbidopa, de 10 % en poids d'hydroxypropylcellulose, de 1 % en poids d'aspartame et de 1 % en poids de composition à libération immédiate d'arôme de menthe, dans laquelle le pourcentage en poids est le pourcentage en poids de chaque composant de la surcouche, et le gain de poids de la surcouche par rapport au noyau de comprimé est de 13,2 % et de 12,9 % en poids, respectivement ;
ou, la composition pharmaceutique est composée d'une couche contenant un médicament, d'une couche de poussée osmotique, d'une membrane de revêtement et d'une surcouche, la couche contenant un médicament est composée de 45 % en poids de lévodopa, de 31 % en poids d'hydroxypropylcellulose, de 17 % en poids de mannitol, de 5 % en poids de povidone K30, de 1 % en poids de stéarate de magnésium et de 1 % en poids d'aspartame, dans laquelle le pourcentage en poids est le pourcentage en poids de chaque composant de la couche contenant un médicament; la couche de poussée osmotique est composée de 60 % en poids de carboxyméthylcellulose sodique 7H4XF, de 10 % en poids de povidone K30, de 26 % en poids de sorbitol, de 2 % en poids d'oxyde de fer rouge et de 2 % en poids de stéarate de magnésium, dans laquelle le pourcentage en poids est le pourcentage en poids de chaque composant de la couche de poussée osmotique ; la membrane de revêtement est composée de 70 % en poids de membrane d'acétate de cellulose et de 30 % en poids de copovidone VA64, dans laquelle le pourcentage en poids est le pourcentage en poids de chaque composant de la membrane de revêtement ; et le poids de la membrane de revêtement est de 4,8 % du poids du noyau de comprimé; la surcouche est composée de 93 % en poids de CD, de 2 % en poids d'hydroxypropylcellulose et de 5 % en poids de composition à libération immédiate d'aspartame, dans laquelle le pourcentage en poids est le pourcentage en poids de chaque composant de la surcouche, et le gain de poids de la surcouche par rapport au noyau de comprimé est de 13,2 % en poids ;
ou, la composition pharmaceutique est composée d'une couche contenant un médicament, d'une couche de poussée osmotique, d'une membrane de revêtement et d'un revêtement, la couche contenant un médicament est composée de 45 % en poids de lévodopa, de 31 % en poids d'hydroxypropylcellulose, de 12 % en poids de mannitol, de 5 % en poids de povidone K30, de 5 % en poids d'arôme de menthe, de 1 % en poids d'aspartame et de 1 % en poids de stéarate de magnésium, dans laquelle le pourcentage en poids est le pourcentage en poids de chaque composant de la couche contenant un médicament; la couche de poussée osmotique est composée de 40 % en poids de carboxyméthylcellulose sodique 7H4XF, de 20 % en poids de povidone K30, 36 % en poids de sorbitol, de 3,5 % en poids d'oxyde de fer rouge et de 0,5 % en poids de stéarate de magnésium, dans laquelle le pourcentage en poids est le pourcentage en poids de chaque composant de la couche de poussée osmotique ; la membrane de revêtement est composée de 70 % en poids de membrane d'acétate de cellulose et de 30 % en poids de copovidone VA64, dans laquelle le pourcentage en poids est le pourcentage en poids de chaque composant de la membrane de revêtement; et le poids de la membrane de revêtement est de 4,8 % du poids du noyau de comprimé ; la surcouche est composée de 75 % en poids de lévodopa, de 20 % en poids d'hydroxypropylcellulose et de 5 % en poids de composition à libération immédiate d'arôme de menthe, dans laquelle le pourcentage en poids est le pourcentage en poids de chaque composant de la surcouche, et le gain de poids de la surcouche par rapport au noyau de comprimé est de 13,2 % en poids ;
ou, la composition pharmaceutique est composée d'une couche contenant un médicament, d'une couche de poussée osmotique, d'une membrane de revêtement et d'une surcouche, la couche contenant un médicament est composée de 62,5 % en poids de lévodopa, de 31 % en poids d'hydroxypropylcellulose, de 4,5 % en poids de mannitol, de 0,1 % en poids d'arôme de menthe, de 0,9 % en poids d'aspartame et de 1 % en poids de stéarate de magnésium, dans laquelle le pourcentage en poids est le pourcentage en poids de chaque composant de la couche contenant un médicament; la couche de poussée osmotique est composée de 55 % en poids de carboxyméthylcellulose sodique 7H4XF, de 10 % en poids d'hydroxypropylcellulose, de 34 % en poids de sorbitol, de 0,5 % en poids d'oxyde de fer rouge et de 0,5 % en poids de stéarate de magnésium, dans laquelle le pourcentage en poids est le pourcentage en poids de chaque composant de la couche de poussée osmotique ; la membrane de revêtement est composée de 70 % en poids de membrane d'acétate de cellulose et de 30 % en poids de copovidone VA64, dans laquelle le pourcentage en poids est le pourcentage en poids de chaque composant de la membrane de revêtement ; et le poids de la membrane de revêtement est de 6,5 % du poids du noyau de comprimé, la surcouche est composée de 62,15 % en poids de lévodopa, de 26,85 % en poids de carbidopa, de 10 % en poids d'hydroxypropylcellulose, de 0,9 % en poids d'aspartame et de 0,1 % en poids % de composition à libération immédiate d'arôme de menthe, dans laquelle le pourcentage en poids est le pourcentage en poids de chaque composant de la surcouche, et le gain de poids de la surcouche par rapport au noyau de comprimé est de 21,0 % en poids ;
ou, la composition pharmaceutique est composée d'une couche contenant un médicament, d'une couche de poussée osmotique, d'une membrane de revêtement et d'une surcouche, la couche contenant un médicament est composée de 46,9 % en poids de lévodopa, de 31 % en poids d'hydroxypropylcellulose, de 20,1 % en poids de mannitol, de 0,1 % en poids d'arôme de menthe, de 0,9 % en poids d'aspartame et de 1 % en poids de stéarate de magnésium, dans laquelle le pourcentage en poids est le pourcentage en poids de chaque composant de la couche contenant un médicament; la couche de poussée osmotique est composée de 55 % en poids de carboxyméthylcellulose sodique 7H4XF, de 10 % en poids d'hydroxypropylcellulose, de 34 % en poids de sorbitol, de 0,5 % en poids d'oxyde de fer rouge et de 0,5 % en poids de stéarate de magnésium, dans laquelle le pourcentage en poids est le pourcentage en poids de chaque composant de la couche de poussée osmotique ; la membrane de revêtement est composée de 70 % en poids de membrane d'acétate de cellulose et de 30 % en poids de copovidone VA64, dans laquelle le pourcentage en poids est le pourcentage en poids de chaque composant de la membrane de revêtement ; et le poids de la membrane de revêtement est de 6,5 % du poids du noyau de comprimé, la surcouche est composée de 62,15 % en poids de lévodopa, de 26,85 % en poids de carbidopa, de 10 % en poids d'hydroxypropylcellulose, 0de ,9 % en poids d'aspartame et de 0,1 % en poids % de composition à libération immédiate d'arôme de menthe, dans laquelle le pourcentage en poids est le pourcentage en poids de chaque composant de la surcouche, et le gain de poids de la surcouche par rapport au noyau de comprimé est de 15,7 % en poids ;
ou, la composition pharmaceutique est composée d'une couche contenant un médicament, d'une couche de poussée osmotique, d'une membrane de revêtement et d'une surcouche ; dans laquelle la couche contenant un médicament est composée de 45 % en poids de lévodopa, de 31 % en poids d'hydroxypropylcellulose, de 17 % en poids de mannitol, de 5 % en poids de povidone K30, de 1 % en poids de stéarate de magnésium et de 1 % en poids d'aspartame, dans laquelle le le pourcentage en poids est le pourcentage en poids de chaque composant de la couche contenant un médicament ; la couche de poussée osmotique est composée de 55 % en poids de carboxyméthylcellulose sodique 7H4XF, de 10 % en poids d'hydroxypropylcellulose, de 34 % en poids de sorbitol, de 0,5 % en poids d'oxyde de fer rouge et de 0,5 % en poids de stéarate de magnésium, dans laquelle le pourcentage en poids est le pourcentage en poids de chaque composant de la couche de poussée osmotique ; la membrane de revêtement est composée de 70 % en poids de membrane d'acétate de cellulose et de 30 % en poids de copovidone VA64, dans laquelle le pourcentage en poids est le pourcentage en poids de chaque composant de la membrane de revêtement; dans lequel l'acétate de cellulose est une membrane d'acétate de cellulose contenant 39,8 % en poids d'acétyle, et le poids de la membrane de revêtement est de 5,9 % du poids du noyau de comprimé ; et, la surcouche est composée de 24 % en poids de lévodopa, de 65 % en poids de carbidopa, de 10 % en poids d'hydroxypropylcellulose et de 1 % en poids d'aspartame, dans laquelle le pourcentage en poids est le pourcentage en poids de chaque composant de la surcouche, le poids de la surcouche est de 13,1 % du poids total du noyau de comprimé et de la membrane de revêtement ;
ou, la composition pharmaceutique est composée d'une couche contenant un médicament, d'une couche de poussée osmotique, d'une membrane de revêtement et d'une surcouche ; la couche contenant un médicament est composée de 45 % en poids de lévodopa, de 31 % en poids d'hydroxypropylcellulose, de 22 % en poids de mannitol, de 0,9 % en poids d'aspartame, de 1 % en poids de stéarate de magnésium et de 0,1 % en poids d'arôme de menthe, dans laquelle le pourcentage en poids est le pourcentage en poids de chaque composant de la couche contenant un médicament; la couche de poussée osmotique est composée de 55 % en poids de carboxyméthylcellulose sodique 7H4XF, de 10 % en poids d'hydroxypropylcellulose, de 34 % en poids de sorbitol, de 0,5 % en poids d'oxyde de fer rouge et de 0,5 % en poids de stéarate de magnésium, dans laquelle le pourcentage en poids est le pourcentage en poids de chaque composant de la couche de poussée osmotique ; la membrane de revêtement est composée de 70 % en poids de membrane d'acétate de cellulose et de 30 % en poids de copovidone VA64, dans laquelle le pourcentage en poids est le pourcentage en poids de chaque composant de la membrane de revêtement; dans laquelle l'acétate de cellulose est une membrane d'acétate de cellulose contenant 39,8 % en poids d'acétyle, et le poids de la membrane de revêtement est de 6,5 % du poids du noyau de comprimé ; et, la surcouche est composée de 54 % en poids de lévodopa, de 35 % en poids de carbidopa, de 10 % en poids d'hydroxypropylcellulose, de 0,9 % en poids d'aspartame et de 0,1 % en poids d'arôme de menthe, dans laquelle le pourcentage en poids est le pourcentage en poids de chaque composant de la surcouche, le poids de la surcouche est de 13,1 % du poids total du noyau de comprimé et de la membrane de revêtement ;
ou, la composition pharmaceutique est composée d'une couche contenant un médicament, d'une couche de poussée osmotique, d'une membrane de revêtement et d'une surcouche ; la couche contenant un médicament est composée de 45 % en poids de lévodopa, de 31 % en poids d'hydroxypropylcellulose, de 22 % en poids de mannitol, de 0,9 % en poids d'aspartame, de 1 % en poids de stéarate de magnésium et de 0,1 % en poids d'arôme de menthe, dans laquelle le pourcentage en poids est le pourcentage en poids de chaque composant de la couche contenant un médicament; la couche de poussée osmotique est composée de 55 % en poids de carboxyméthylcellulose sodique 7H4XF, de 10 % en poids d'hydroxypropylcellulose, de 34 % en poids de sorbitol, de 0,5 % en poids d'oxyde de fer rouge et de 0,5 % en poids de stéarate de magnésium, dans laquelle le pourcentage en poids est le pourcentage en poids de chaque composant de la couche de poussée osmotique ; la membrane de revêtement est composée de 70 % en poids de membrane d'acétate de cellulose et de 30 % en poids de copovidone VA64, dans laquelle le pourcentage en poids est le pourcentage en poids de chaque composant de la membrane de revêtement; dans laquelle l'acétate de cellulose est une membrane d'acétate de cellulose contenant 39,8 % en poids d'acétyle, et le poids de la membrane de revêtement est de 7,0 % du poids du noyau de comprimé; et, la surcouche est composée de 42,8 % en poids de lévodopa, de 46,2 % en poids de carbidopa, de 10 % en poids d'hydroxypropylcellulose, de 0,9 % en poids d'aspartame et de 0,1 % en poids d'arôme de menthe, dans laquelle le pourcentage en poids est le pourcentage en poids de chaque composant de la surcouche, le poids de la surcouche est de 13,1 % du poids total du noyau de comprimé et de la membrane de revêtement ;
ou, la composition pharmaceutique est composée d'une couche contenant un médicament, d'une couche de poussée osmotique, d'une membrane de revêtement et d'une surcouche ; la couche contenant un médicament est composée de 45 % en poids de lévodopa, de 31 % en poids d'hydroxypropylcellulose, de 22 % en poids de mannitol, de 0,9 % en poids d'aspartame, de 1 % en poids de stéarate de magnésium et de 0,1 % en poids d'arôme de menthe, dans laquelle le pourcentage en poids est le pourcentage en poids de chaque composant de la couche contenant un médicament; la couche de poussée osmotique est composée de 55 % en poids de carboxyméthylcellulose sodique 7H4XF, de 10 % en poids d'hydroxypropylcellulose, de 34 % en poids de sorbitol, de 0,5 % en poids d'oxyde de fer rouge et de 0,5 % en poids de stéarate de magnésium, dans laquelle le pourcentage en poids est le pourcentage en poids de chaque composant de la couche de poussée osmotique ; la membrane de revêtement est composée de 70 % en poids de membrane d'acétate de cellulose et de 30 % en poids de copovidone VA64, dans laquelle le pourcentage en poids est le pourcentage en poids de chaque composant de la membrane de revêtement; dans laquelle l'acétate de cellulose est une membrane d'acétate de cellulose contenant 39,8 % en poids d'acétyle, et le poids de la membrane de revêtement est de 9,0 % du poids du noyau de comprimé; et, la surcouche est composée de 28,2 % en poids de lévodopa, de 60,8 % en poids de carbidopa, de 10 % en poids d'hydroxypropylcellulose, de 0,9 % en poids d'aspartame et de 0,1 % en poids d'arôme de menthe, dans laquelle le pourcentage en poids est le pourcentage en poids de chaque composant de la surcouche, le poids de la surcouche est de 13,1 % du poids total du noyau de comprimé et de la membrane de revêtement.

16. Forme posologique à libération prolongée selon l'une quelconque des revendications 1-15, dans laquelle la composition pharmaceutique est un système d'administration de médicament à libération prolongée par pompe osmotique, facultativement, le système d'administration de médicament à libération prolongée par pompe osmotique est un comprimé à libération prolongée ; dans laquelle, le comprimé à libération prolongée est un cylindre d'un diamètre de 5 à 10 mm et d'une hauteur de 5 à 30 mm, ou un comprimé d'une longueur de 10 à 25 mm et d'une largeur de 5 à 10 mm ; et/ou, chacun des comprimés à libération prolongée contient 62,5 mg de CD et 500 mg de LD, ou 62,5 mg de CD et 375 mg de LD, ou 62,5 mg de carbidopa et 250 mg de lévodopa, ou 50 mg de carbidopa et 500 mg de lévodopa, ou 37,5 mg de carbidopa et 375 mg de lévodopa.

17. Forme posologique à libération prolongée selon l'une quelconque des revendications 1, 2 ou 4-16 à utiliser dans une procédure d'opération, qui comprend les étapes suivantes : mise en place de la plate-forme à libération prolongée dans la plate-forme à rétention possible personnalisée, et fixation de la plate-forme à rétention possible sur les dents correspondantes dans la cavité buccale ; retrait de la forme posologique à libération prolongée après l'avoir conservée pendant 4 à 24 heures, remplacement de la plate-forme à libération prolongée par une nouvelle et nouvelle fixation de la plate-forme à rétention possible sur les dents correspondantes dans la cavité buccale, de sorte que le médicament puisse être libéré de manière continue et stable.
